(19) Europäisches Patentamt
European Patent Office
Office européen des brevets

(11) EP 2 423 316 B1

(12) **EUROPEAN PATENT SPECIFICATION**

(45) Date of publication and mention of the grant of the patent:
**16.11.2016 Bulletin 2016/46**

(51) Int Cl.:
***C12N 15/82*** *(2006.01)*

(21) Application number: **10173968.8**

(22) Date of filing: **25.08.2010**

(54) **Method for determining meiotic recombination frequencies in plants**

Methode zur Bestimmung der meiotischen Rekombinationsfrequenz in Pflanzen

Procédé pour déterminer la fréquence de la recombinaison meiotique dans les plantes

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**29.02.2012 Bulletin 2012/09**

(73) Proprietors:
- **Leibniz-Institut für Pflanzengenetik und Kulturpflanzenforschung (IPK)
06466 Gatersleben (DE)**
- **Nordsaat Saatzuchtgesellschaft mbH
38895 Langenstein (DE)**

(72) Inventor: **Gils, Mario
06484 Quedlinburg (DE)**

(74) Representative: **Lasar, Andrea Gisela
Maiwald Patentanwalts GmbH
Elisenhof
Elisenstraße 3
80335 München (DE)**

(56) References cited:
**WO-A1-03/102197 WO-A1-2004/067749
WO-A2-2007/030014 WO-A2-2009/147179**

- **WIJNKER E ET AL: "Managing meiotic recombination in plant breeding" TRENDS IN PLANT SCIENCE, ELSEVIER SCIENCE, OXFORD, GB LNKD- DOI:10.1016/J.TPLANTS.2008.09.004, vol. 13, no. 12, 1 December 2008 (2008-12-01), pages 640-646, XP025694256 ISSN: 1360-1385 [retrieved on 2008-11-26]**
- **KOVALCHUK I ET AL: "Pathogen-induced systemic plant signal triggers DNA rearrangements" NATURE, NATURE PUBLISHING GROUP, LONDON, GB, vol. 423, 12 June 2003 (2003-06-12), pages 760-762, XP007915011 ISSN: 0028-0836**
- **TOMOYUKI FUKUDA ET AL: "VDE-initiated intein homing in Saccharomyces cerevisiae proceeds in a meiotic recombination-like manner" GENES TO CELLS, OXFORD, GB, vol. 8, 1 January 2003 (2003-01-01), pages 587-602, XP007915015 ISSN: 1356-9597**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 423 316 B1

**Description**

**FIELD OF THE INVENTION**

The present invention relates to a method of determining meiotic recombination frequencies in plants by using intein-mediated reconstitution of reporter proteins.

**BACKGROUND OF THE INVENTION**

Meiotic crossing-over-recombination is a central event in the life cycle of all sexual eukaryotic organisms. The primary function of crossing-over during meiosis is the mechanical cohesion of the homologous chromosomes until metaphase (visualised as chiasmata) by physical links. The homologues are then correctly segregated during meiosis I, followed by the second meiotic division in which sister chromatids are segregated and finally, each gamete carries only a single copy of each chromosome (Petronczki et al. (2003) Cell 112(4): 423-440). In contrast to somatic recombination which seems to be controlled more randomly, the number and distribution of crossing-overs along chromosomes appear to be tightly controlled in most organisms. Different factors control crossing-over distribution: First, each chromosome pair possesses at least one crossing-over ("obligatory crossing-over", "crossing-over assurance"). Second, the presence of a crossing-over inhibits the formation of a new crossing-over nearby on the chromosome and this inhibition decreases with distance ("crossing-over interference"). Third, in all eukaryotes that have been studied including plants, the distribution of crossing-overs along chromosomes is not homogeneous. Some regions, called "hotspots", display much higher frequencies of crossing-over than others ("coldspots").

This factor is fundamental for breeding programmes since it implies that the distribution of crossing-overs with respect to the location of genes will affect the efficiency of the breeding process. Many important crop plants with large genomes (e. g. wheat, barley, and rye) exhibit an uneven distribution of recombination along their chromosomes with the proximal (centromeric) chromosome regions being highly suppressed (Drouaud et al. (2006) Genome Res. 16(1): 106-114; Mezard et al. (2007) Trends Gent. 23(2): 91-99). For example, market introduction of new barley varieties is increasingly hampered by the fact that the genetic diversity within the available gene-pools continuously decreases. It is assumed that the low variability is primarily due to the low crossing-over frequency in barley, leading to a tight or complete linkage of loci which limits breeding potential. For the above species with large genomes, the key for breeding improvement will be the possibility to alter the distribution of recombination so that recombination can be increased in regions exhibiting extensive suppression.

The frequency of meiotic recombination can vary due to several internal factors such as sex (T. Lenormand and J. Dutheil PLoS Biol 3(3): e63), the genetic plant background or between different cultivars or accessions (E. Sanchez-Moran et al. Genetics 162(3): 1415-1422). Crossing-over frequencies can also be changed artificially e. g. by temperature shock, mutagenesis (chemical or by physical), expression of transgenes or by virus-infection (Kathiria et al. (2010) Plant Physiol. 153(4): 1859-1870).

To determine the meiotic recombination frequency in a population of plants and the influence of defined factors on this frequency, a fast and reliable assay would be desirable.

The prior art discloses several methods for determining homologous recombination frequencies in plants. For example, cytological methods allow for a genome-wide determination of the recombination frequency by the detection of chiasmata or late recombination nodules (see, e.g., Siaud et al. (2004) EMBO J. 23: 1392-1401). However, this method is technically sophisticated and does not allow for high-throughput screening of recombination events at the population level. The same applies to the tetrad analysis which can be used for individually studying the four products of a single meiotic event in *Arabidopsis thaliana* (Copenhaver et al. (2000) Plant Physiol. 124: 7-16; Berchowitz and Copenhaver (2008) Nat. Protoc. 3(1): 41-50).

Another approach utilizes the plant recombination machinery for reconstituting the functionality of a marker gene by homologous recombination. Here, reporter-based constructs consist of tandem repeats of a disrupted β-glucuronidase (GUS) or luciferase (LUC) gene with overlapping sequences ("GU-US" or "LU-UC"), with "U" denoting homologous sequence regions. This assay was applied for scoring extrachromosomal or intrachromosomal homologous recombination (Molinier et al. (2004) Plant Cell 16: 1633-1643; Puchta and Hohn (1991) Mol. Gen. Genet. 230: 1-7; Swoboda et al. (1994) EMBO J. 13: 484-489), but is restricted to homologous recombination mechanisms in somatic cells and therefore cannot be used to determine meiotic recombination frequencies.

Finally, recombination events within defined chromosomal regions can also be measured by using phenotypical or molecular markers. However, there are only a few morphological markers available and most of them can only be distinguished in mature plants. Furthermore, determining specific crossing-over frequencies is costly and laborious and mostly confined to model species. If molecular markers are to be used, the sample sizes and number of mapping populations are restricted, as for each sample DNA has to be isolated and analyzed by laboratory procedures such as polymerase chain reaction and electrophoresis.

Hence, there is still a need for a rapid assay that reliably and cost-effectively allows the determination of the frequency of meiotic crossing-over.

## OBJECT AND SUMMARY OF THE INVENTION

It is thus on object of the present invention to provide a method for determining meiotic recombination frequencies which method is reliable and cost-effective and which allows high-throughput screening to be performed.

It is a further object of the present invention to provide a method for identifying factors which influence the meiotic recombination frequency in plants and which therefore can be used in plant breeding to increase the recombination frequency and to combine new genotypes.

These and further objects of the invention, as will become apparent from the description, are attained by the subject-matter of the independent claims.

Some of the preferred embodiments of the present invention form the subject-matter of the dependent claims.

The present inventors have found that the frequency of meiotic recombination can be determined by functional complementation of reporter gene fragments using intein-mediated protein splicing. The method of the present invention combines the advantages of morphological markers, i.e. the visual scoring of large numbers of plants, with the benefits of molecular markers, i.e. the coverage of large and defined parts of the genome. The method is characterized by easy handling and low operation costs and can be used in high-throughput screening approaches.

The present invention is based on the finding that crossing-over between the loci coding for the N- and the C-terminal part of a reporter protein leads to a controlled transmission of the reporter protein fragments to the progeny which expresses the reporter protein. Hence, plants in which the recombination has taken place can be detected and the recombination frequency can be calculated on the basis of the expression of the reporter protein.

Accordingly, in one embodiment, the present invention provides a method for determining meiotic recombination frequencies in plants, comprising the following steps:

(a) obtaining a first plant having in a first locus of a nuclear chromosome a first expression cassette comprising in 5' to 3' direction:

- a promoter functional in plant cells;
- operatively linked thereto a nucleic acid sequence coding for the N-terminal part of a reporter protein;
- a nucleic acid sequence coding for the N-terminal part of a first intein; and
- optionally, operatively linked thereto a termination sequence functional in plant cells;

(b) obtaining a second plant having in a second locus of a nuclear chromosome homologous to said nuclear chromosome of step (a) a second expression cassette comprising in 5' to 3' direction:

- a promoter functional in plant cells;
- operatively linked thereto a nucleic acid sequence coding for the C-terminal part of said first intein;
- a nucleic acid sequence coding for the C-terminal part of said reporter protein; and
- optionally, operatively linked thereto a termination sequence functional in plant cells;
  wherein the first and the second locus are different and wherein both the N-terminal and the C-terminal part of the reporter protein contain a sequence portion necessary for the function of the reporter protein;

(c) crossing said first and said second plant to produce F1 progeny; and
(d) analyzing the pollen of the F1 progeny for the expression of the reporter protein.

In a further embodiment, the method additionally comprises step (e) of crossing a plant of the F1 progeny with a wild-type plant to produce F2 progeny and analyzing the F2 progeny for the expression of the reporter protein.

In an alternative embodiment, step (d) of the above method is replaced with step (d') of crossing a plant of the F1 progeny with a wild-type plant to produce F2 progeny and analyzing the F2 progeny for the expression of the reporter protein.

In a preferred embodiment of the present invention the method further comprises the step of calculating the meiotic recombination frequency.

In a further preferred embodiment of the present invention the reporter protein is β-glucuronidase.

In still another preferred embodiment of the present invention the first intein is selected from the group consisting of DnaB and DnaE.

In a further embodiment of the present invention said first plant further comprises a third expression cassette comprising in 5' to 3' direction:

- a tapetum-specific promoter functional in plant cells;
- operatively linked thereto a nucleic acid sequence coding for the N-terminal part of a protein which provides for male sterility;
- a nucleic acid sequence coding for the N-terminal part of a second intein; and
- optionally, operatively linked thereto a termination sequence functional in plant cells;
  and said second plant comprises a fourth expression cassette comprising in 5' to 3' direction:

  - a tapetum-specific promoter functional in plant cells;
  - operatively linked thereto a nucleic acid sequence coding for the C-terminal part of said second intein;
  - a nucleic acid sequence coding for the C-terminal part of said protein which provides for male sterility; and
  - optionally, operatively linked thereto a termination sequence functional in plant cells,
    wherein the second intein is different from the first intein.

In a preferred embodiment of the invention, the protein which provides for male sterility is an Rnase, more preferably barnase.

In a preferred embodiment of the present invention said second intein is selected from the group consisting of DnaB and DnaE.

In still another embodiment of the present invention said first and said second plant is a monocotyledonous plant, preferably a barley plant.

In a further embodiment of the present invention said first and/or said second plant further comprises at least one expression cassette comprising in 5' to 3' direction

- a promoter functional in plant cells;
- operatively linked thereto a nucleic acid sequence coding for a protein the effect of which on recombination frequency is to be tested or a nucleic acid sequence inhibiting the expression of a protein the effect of which on recombination frequency is to be tested; and
- optionally, operatively linked thereto a termination sequence functional in plant cells.

In a preferred embodiment of the present invention the promoter which regulates the expression of the protein the effect of which on recombination frequency is to be tested or of the nucleic acid sequence inhibiting the expression of the protein the effect of which on recombination frequency is to be tested is a promoter which is exclusively active during gamete development.

In still a further embodiment of the present invention, the method further comprises the step of mutagenizing the seeds obtained from crossing the first and the second plants and/or exposing the seeds obtained from crossing the first and the second plants or the F1 progeny plants to biotic or abiotic stress.

In a further preferred embodiment of the present invention the method further comprises a step of calculating the meiotic recombination frequency induced by the protein the effect of which on recombination frequency is to be tested and/or by mutagenesis and/or exposure to biotic and/or abiotic stress.

BRIEF DESCRIPTION OF THE DRAWINGS

Figure 1: Schematic drawing showing the method steps of the present invention

a) Recombination assay through pollen analysis.
Without crossing-over, N- and C-events obligatorily segregate to different gametes (left). Recombination between N- and C-terminal parts (resulting in a *"cis->trans*-transition" of the gene fragments) can be detected in the pollen of the F1 progeny displaying a "recombinant" marker phenotype (after one crossing step). The percentage of pollen showing the marker phenotype is a function of the crossing-over frequency in the investigated region.
b) Analysis of crossing-over frequency in $F_2$ progeny by use of a split barnase system
The use of a split-barnase system leads to male sterility exclusively in the heterozygous $F_1$ plants while the primary transformant To plants carry only a fragment of the sterility gene, and therefore are fertile and can be propagated by selfing.
c) Influencing crossing-over frequency by external factors (upper panel) or by transgene expression (lower panel).

Figure 2: Structure of the T-DNA constructs used in transformations

a) N- and C- expression vectors producing the N- or C-terminal part of GUS, respectively. When being jointly present in a cell or a pollen grain, the precursors can be physically/covalently linked by inteins and form an active protein. Abbreviations: RB, right border; CPr, constitutive promoter such as the 35S, rice actin or maize ubiquitin promoter; IntN, IntC, gene fragments from the DnaE and DnaB genes of *Synechocystis sp* coding for N- and C-terminal intein sequences, respectively; GUS-N, GUS-C, gene fragments from the *E.coli uidA* gene coding for the N- and C-terminal fragments of GUS, respectively; T, terminator; KAN, kanamycine resistance gene (NPTII); HYG, hygromycin resistance gene (HPT); NSIEQD, RESG, sequences from the DnaB extein; GGGGS, flexible linker sequence; LB, left border; pTAP, tapetum- specific promoter; Barnase-N, Barnase-C, gene fragments from the *Bacillus amyloliquefaciens* RNase gene coding for the N- and C-terminal fragments of barnase, respectively; DVKFAEY, CFNGH, extein sequences from the DnaE extein.

b) N- and C- expression vectors producing the N- or C-terminal part of GUS and barnase, respectively. When being jointly present in a cell or a pollen grain, the precursors can be physically/covalently linked by inteins and form an active protein.
For abbreviations see legend to Figure 2a.

Figure 3: Test of T-DNA construct functionality

a) Transient delivery of split GUS vectors pHW121+161 together into the callus of barley via biolistic particle bombardment results in reconstitution of the GUS protein and GUS activity
b) Transient delivery of split GUS vectors pHW 131+171 together into the callus of barley via biolistic particle bombardment results in reconstitution of the GUS protein and GUS activity
c) Transient delivery of split GUS vectors pHW141, 181 separately into tobacco leaves via biolistic particle bombardment does not lead to GUS expression and activity
d) Transient delivery of split GUS vectors pHW 141+181 together into tobacco leaves via biolistic particle bombardment results in reconstitution of the GUS protein and GUS activity
e) *Arabidopsis thaliana* plants stably transformed with pHW141 do not show GUS expression and activity
f) *Arabidopsis thaliana* plants stably transformed with pHW301 do not show GUS expression and activity
g) F1 progeny plant of the plants of e) and f) carrying both pHW141 and pHW301 shows GUS expression and activity
h) Test of vector functionality by infiltrating leaves on *N. benthamiana* separately with pHW121, pHW161 and pHW 301 shows that the separate expression of the GUS fragments does not result in full-length GUS expression and activity j) Test of vector functionality by infiltrating leaves on *N. bentharnaiana* with combinations of pHW121+pHW161 (left), pHW131+pHW181 (middle) and pHW141+pHW161 (right) shows GUS expression and activity

## DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments but the invention is not limited thereto but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated.

The term "meiosis" or "meiotic" refers to a two-stage process of nuclear division that reduces the somatic chromosome number (2n) to half (n) and which is usually followed by gamete formation. In the first stage of meiosis the chromosome number is reduced, wherein in the second stage of meiosis there is an equational division of the chromosome resulting in four daughter nuclei, each carrying one chromatid.

The term "recombination" refers to a process by which the linkage of genes is altered. Due to the recombination process, the combination of genes in a progeny molecule, cell or organism has a different pattern than the combination of genes in the parent molecules, cells or organisms. The recombination may occur due to the exchange of DNA sequences between two chromosomes or the new association of genes in a recombinant. Within the meaning of the present invention, the recombination is due to the exchange of DNA sequences between two chromosomes by the process of crossing-over, i.e. the reciprocal exchange of segments of homologous chromosomes by symmetrical breakage and crosswise recombination. Hence, the terms "recombination" and "crossing-over" may be used interchangeably.

The term "recombination frequency" or "crossing-over frequency" is used to denote the frequency by which crossing-over and recombination occurs between two loci on a chromosome. The term "meiotic recombination frequency" refers to the frequency of recombination during meiosis and therefore does not include somatic recombination events. The recombination frequency is usually calculated as the percentage of individuals having the recombined phenotype per total number of individuals analyzed. In the process of the invention it is calculated as the number of individuals showing expression of the reporter protein per total number of individuals analyzed in a test cross population such as the pollen of the $F_1$ progeny or the plants of the $F_2$ progeny, as discussed further below. The "basal" recombination frequency is the recombination frequency in plants which are grown under standard conditions, which have not been mutagenized or treated with biotic or abiotic stimuli and which have not been transformed with expression cassettes other than the first and second and optionally third and fourth expression cassette. The "induced" recombination frequency is the recombination frequency in plants which have been mutagenized and/or treated with biotic and/or abiotic stimuli and/or which have been transformed with a nucleic acid sequence coding for a protein which influences recombination or inhibiting the expression of a protein which influences recombination.

A plant shows expression of the reporter protein if it shows an expression of the reporter protein above the background level, i.e. the level of reporter protein expression in plants which do not contain a gene coding for the reporter protein which is split in the method of the present invention. If the reporter protein is an enzyme, the expression of the reporter protein usually correlates with enzymatic activity of the reporter protein.

The method of the present invention is suitable to determine meiotic recombination frequencies both in monocotyledonous and dicotyledonous plants. Preferably, the plant is a monocotyledonous or dicotyledonous crop plant such as a food or fodder plant. Examples for monocotyledonous plants are plants belonging to the genera Avena (oat), Triticum (wheat), Secale (rye), Hordeum (barley), Oryza (rice), Panicum, Pennisetum, Setaria, Sorghum (millet), Zea (maize), Lolium and the like.

Examples of dicotyledonous plants include, *inter alia,* cotton, legumes, soy bean, rapeseed, tomato, sugar beet, potato, ornamental plants, and trees. Further useful plants can comprise fruit (in particular apples, pears, cherries, grapes, citrus, pineapple, and bananas), pumpkin, cucumber, wine, oil palms, tea shrubs, cacao trees, and coffee shrubs, tobacco, sisal, as well as, with medicinal plants, rauwolfia and digitalis.

Preferably, the plants whose meiotic recombination frequency is determined by the process of the present invention are monocotyledonous plants, more preferably plants of the genus Hordeum and most preferably plants of the species *Hordeum vulgare* (barley).

The term "locus" refers to the position of a gene on a chromosome or gene map. The relative distance between two loci can be given by referring to the Morgan unit, but a locus can also be identified by the nature of the neighboring genes. In the process of the invention, the first and the second locus are different, i.e. if the first expression cassette is integrated into locus A on a first chromosome, the second expression cassette is integrated into locus B on a second, homologous chromosome which locus has a distance of n cM from locus A such that in meiosis recombination between the loci A and B is possible and results in the reconstitution of a functional reporter protein.

A "chromosome" is one of the self-replicating, thread- or rod-shaped structures within the nuclei of eukaryotic cells that consists of extremely condensed chromatin and contains genetic information for specific functions of the cell.

"Homologous chromosomes" are chromosomes that pair during meiosis and contain the same linear sequences of genes. During meiosis meiotic recombination occurs between homologous chromosomes by crossing-over mechanisms, resulting in the exchange of DNA segments between the homologous chromosomes.

Within the scope of the present invention, the term "expression cassette" means a nucleic acid molecule which contains all elements which are necessary for the expression of a gene or a fragment thereof, i.e. the gene to be expressed under the control of a suitable promoter and optionally further regulatory sequences such as termination sequences. An expression cassette of the present invention may be part of an expression vector which is transferred into a plant cell or may be integrated into the chromosome of a transgenic plant after transformation. The terms "first expression cassette", "second expression cassette", "third expression cassette" and "fourth expression cassette" are only used to distinguish the different expression cassettes comprising different elements, but are not intended to indicate any spatial relationship or order between the expression cassettes, i.e. the third expression cassette may be located 5' of the first expression cassette within an expression vector or a chromosome and the fourth expression cassette may be located 5' of the third expression cassette within an expression vector or a chromosome.

If the expression cassette is part of an expression vector, the expression vector is preferably selected from the group consisting of plasmids, cosmids, (recombinant) viruses and other vectors known in the field of gene technology, with which nucleic acid molecules can be transferred to plants or plant cells. The term "vector" also comprises so-called minochromosomes which are linear or circular DNA fragments which contain centromer sequences of the respective plant in addition to the transgene. Minichromosomes are stable in the nucleus and are passed on to the daughter cells during cell division. They are transferred by standard methods of transformation. Most preferably, the vector is selected from the group consisting of pBR322, pUC vectors, M13mp vectors or vectors being derived from the Ti plasmid or the Ri plasmid of agrobacteria.

Suitable cloning vectors contain a replication signal for *E. coli* and a marker gene for the selection of transformed bacterial cells. The required sequence can be introduced into the vector at an appropriate restriction site. The plasmid obtained is used for the transformation of *E. coli* cells. Transformed *E. coli* cells are cultivated in an appropriate medium, and finally harvested and lysed. The plasmid is recovered. To analyze the plasmid DNA obtained, methods such as restriction analyses, gel electrophoreses and other biochemical/molecular biological methods are available. Following each manipulation the plasmid DNA can be cleaved and the DNA fragments obtained can be combined with other DNA sequences. Each plasmid DNA sequence can be cloned into the same or other plasmids. Standard cloning methods can be taken from Sambrook et al., 2001 (Molecular cloning: A laboratory manual, 3rd edition, Cold Spring Harbor Laboratory Press).

The first and the second plant comprising the first and the second expression cassette, respectively, may be obtained by transformation of plant cells with a vector as described above or other methods for introducing genetic material into plant cells. However, once suitable first and second plants have been established by transformation, it is also possible to use the progeny of these plants which progeny carries the first or second expression cassette, respectively, in the method of the present invention.

The reporter protein is expressed under the control of a promoter which is functional in plant cells, preferably cells of a monocotyledonous plant. This promoter does not have to be derived from a monocotyledonous plant as long it can regulate the expression of a nucleic acid sequence operatively linked thereto in cells of a monocotyledonous plant. Preferably, constitutive promoters such as the 35S promoter, the actin promoter, preferably the rice actin1 promoter (McElroy et al. (1990) Plant Cell 2: 163 - 171), or the ubiquitin promoter, preferably the maize ubiquitin promoter (Christensen et al. (1996) Transgenic Res. 5: 213-218) are used. However, other promoters can of course be used which are obtainable from different sources such as plants or plant viruses or fungi. The choice of promoter and other regulatory sequences determines the local and temporal expression pattern of the gene. Besides constitutive promoters, also tissue-specific promoters such as the phosphoenolpyruvate promoter or the fructose-1,6-bisphosphatase promoter or inducible promoters are conceivable.

The promoter used in the second expression cassette may be the same as the one used in the first expression cassette or it may be different from the promoter used in the first expression cassette, preferably the same promoter is used in the first and the second expression cassette to ensure that equimolar amounts of the N- and the C-terminal fragments are produced.

The term "operatively linked" is understood to denote that the sequences linking the different nucleic acids used are selected in such a way that the function of the respectively linked nucleic acid segment is maintained. In case, for example, the nucleotide sequence coding for the N- or C-terminal part of the reporter protein is to be expressed in a cell, it has to be ensured that no sequences which would lead to a termination of the transcription are located between the promoter sequence and the nucleotide sequence coding for the N- or C-terminal part of the reporter protein.

The "termination sequences" are sequences which ensure that the transcription or the translation is properly terminated. If the transferred nucleic acids are to be translated, the termination sequences are typically stop codons and corresponding regulatory sequences; if the transferred nucleic acids are only to be transcribed, they are generally poly-A sequences. Preferably, the termination sequence is selected from the octopine synthase terminator and the nopaline synthase terminator (Jones et al. (1992) Transgenic Res. 1: 285 - 297).

A "reporter protein" is a protein which is well characterized, both genetically and biochemically, and whose activity is normally not detectable in the target organism into which it is transferred. The expression and activity of the reporter protein can be easily tested by simple assays such as the enzymatic activity of the protein product or the fluorescence mediated by the expression of the protein. Suitable reporter proteins include β-galactosidase, β-glucuronidase, luciferase and autofluorescent proteins such as green fluorescent protein and various analogs thereof. Preferably, the reporter protein is β-glucuronidase (GUS).

GUS is a bacterial enzyme which is encoded by the *uidA* gene of *Escherichia coli* and which catalyzes the hydrolysis of various β-glucuronides such as p-nitrophenyl-glucuronide (PNPG), 4-methyl-umbelliferyl-glucuronide (MUG), 5-bromo-4-chloro-3-indolyl-β-D-Glucuronide (X-gluc) and others, leading to a detectable reaction product. A suitable assay for determining GUS activity is described in the Examples section below. Preferably, the amino acid sequence of β-glucuronidase is the amino acid sequence according to SEQ ID NO: 2. This amino acid sequence is preferably encoded by a nucleic acid sequence according to SEQ ID NO: 1.

In the process of the invention, the nucleotide sequence encoding a reporter protein, preferably the GUS protein, is split into two fragments, thus obtaining a 5' and a 3' part of the nucleotide sequence. Said 5' part encodes the N-terminal part of the reporter protein and said 3' part encodes the C-terminal part of the reporter protein.

Said nucleotide sequence is typically a coding sequence (or an open reading frame) of a reporter protein. However, said nucleotide sequence may also contain one or more introns.

To obtain said 5' and 3' part of the nucleotide sequence, said nucleotide sequence is preferably split such that each obtained fragment, upon expression, is incapable of generating a reporter protein in the absence of the other fragment. Each fragment contains a sequence portion necessary for the function of the reporter protein. For example, if said protein is an enzyme, each fragment preferably contains amino acids necessary for catalysis or substrate binding of the enzyme. The reporter protein may be split into said fragments in many different ways provided that expression of the reporter protein requires all said fragments and binding thereof to each other. Structural and functional information about the reporter protein may be helpful for finding a suitable splitting site of said nucleotide sequence. In any case, one can easily test experimentally whether a fragment generated by splitting a nucleotide sequence at a randomly chosen site codes for an active reporter protein by expressing the fragment in plant cells and investigating if these plant cells show reporter activity, such as GUS activity. A further assay for testing the functionality of the fragments is described in the examples, i.e. syringe infiltration of *Nicotiana benthamiana* leaves using Agrobacterium containing either an N-terminal part or a C-terminal fragment of the protein and detecting the expression of the reporter protein in the leaves. If the reporter protein is expressed upon infiltration only with the N-terminal or the C-terminal fragment, these fragments are not suitable for use in the method of the present invention.

For example, firefly luciferase may be split into an N-terminal fragment comprising 437 amino acids and a C-terminal fragment comprising 117 amino acids (Paulmurugan et al. (2002) Proc. Natl. Acad. Sci. USA 99(24): 15608-15613) and enhanced green fluorescent protein (eGFP) may be split into an N-terminal fragment comprising 158 amino acids and a C-terminal fragment comprising 81 amino acids (Demidov et al. (2006) Proc. Natl. Acad. Sci. USA 103(7): 2052-2056).

If GUS is used as a reporter protein in the method of the present invention, the GUS is split into an N-terminal and a C-terminal fragment in the amino acid region between amino acid residues 195 and 205 of the mature protein, i.e. between amino acids 195 and 196, 196 and 197, 197 and 198, 198 and 199, 199 and 200, 200 and 201, 201 and 202, 202 and 203, 203 and 204 or 204 and 205 of the amino acid sequence according to SEQ ID No. 2.

More preferably, the N-terminal part of GUS comprises 201 amino acids and the C-terminal part of GUS comprises 407 amino acids. Alternatively, the N-terminal part of GUS may comprise 200 amino acids if no methionine is added to the N-terminus.

Most preferably, the N-terminal part of the b-glucuronidase is encoded by a nucleic acid sequence according to SEQ ID No. 3 and the C-terminal part of the b-glucuronidase is encoded by a nucleic acid sequence according to SEQ ID No. 5. The N-terminal part of the b-glucuronidase preferably has the amino acid sequence according to SEQ ID No. 4 and the C- terminal part of the b-glucuronidase preferably has the amino acid sequence according to SEQ ID No. 6.

However, the invention is also intended to comprise smaller fragments of the GUS protein or "functional GUS fragments", as long as the N- and the C-terminal parts of such fragment restore a functional, enzymatically active reporter protein upon intein-mediated trans-splicing. For example, the C-terminal part may lack one or more amino acid residues at its C-terminus and/or the N-terminal part may lack one or more amino acid residues at its N-terminus. Upon intein-mediated trans-splicing a protein smaller than the wild-type protein is formed from the two fragments which protein is functional and enzymatically active. However, also deletions within the N-and/or the C-terminal part of the GUS protein as defined above are conceivable as long as they do not affect the ability of the N- and the C-terminal part to restore a functional protein.

Further, the invention is also intended to comprise GUS proteins which are extended by one or more amino acids attached to the N-terminus of the protein, as long as the N- and the C-terminal parts of the protein restore a functional, enzymatically active protein upon intein-mediated trans-splicing.

Expression of the reporter protein requires the presence of both fragments in the same plant, preferably in the same cells thereof. Expression of the reporter protein further requires transcription and translation of said first and said second fragment and binding of the translation products of said fragments with peptide bond formation to restore a functional protein.

This peptide bond formation is accomplished by intein-mediated trans-splicing. For this purpose, said first and said second expression cassette further code for inteins capable of mediating protein trans-splicing. "Inteins" are proteins that are able to catalyze trans-splicing events between separate protein fragments as they are able to excise themselves from precursor molecules and ligate the flanking protein sequences in a process termed protein-splicing (Southworth et al. (1998) EMBO J. 17: 918 - 926; Wu et al. (1998) Proc. Natl. Acad. Sci. USA 95: 9226 - 9231; Saleh and Perler (2006) Chem. Rec. 6: 183 - 193; Perler (1998) Cell 92: 1 - 4). By said trans-splicing, the proteins and polypeptides encoded by said first and said second fragments may be linked by peptide bond formation. Trans-splicing inteins may be selected from the nucleolar and organellar genomes of different organisms including eukaryotes, archaebacteria and eubacteria. Inteins that may be used for performing this invention are listed at http://www.neb.com/neb/inteins.html. The nucleotide sequence coding for an intein may be split into a 5' and a 3' part that code for the N-terminal and C-terminal part of the intein, respectively. Sequence portions not necessary for intein splicing (e.g. homing endonuclease domain) may be deleted. The intein coding sequence is split such that the 5' and the 3' parts are capable of trans-splicing. For selecting a suitable splitting site of the intein coding sequence, the considerations published by Southworth et al. (1998) EMBO J. 17: 918 - 926 may be followed. In constructing the first and the second expression cassette, the 5' intein coding sequence is linked to the 3' end of the first fragment coding for the N-terminal part of the reporter protein and the 3' intein coding sequence is linked to the 5' end of the second fragment coding for the C-terminal part of the reporter protein. Herein, peptide bond means the amide linkage between the carboxyl group of one polypeptide and the amino group of another polypeptide.

Preferably, the first intein is selected from the group consisting of DnaB and DnaE, both from the green-blue algae of Synechocystis spec. More preferably, the first intein is DnaB and most preferably, the N-terminal part of DnaB is encoded by the nucleic acid sequence according to SEQ ID No. 7 and the C-terminal part of DnaB is shown is encoded by the nucleic acid sequence according to SEQ ID No. 9. Preferably, the N-terminal part of DnaB has the amino acid sequence according to SEQ ID No. 8 and the C-terminal part of DnaB has the amino acid sequence according to SEQ ID No. 10.

The first and/or the second expression cassette may further comprise a flexible linker sequence. The "flexible linker sequence" within the scope of the present invention is a short flexible peptide which is used to bridge the N- and the C-terminal part of the reporter protein without serious steric interference. The flexible linker sequence brings the two splice junctions in close proximity and helps to precisely align all reacting groups. Hence, efficient splicing is supported (Chong and Xu (1997) J. Biol. Chem. 272: 15587 - 15590). The most widely used linker designs have sequences consisting essentially of stretches of glycine (G) and serine (S) residues, because hydrophilic amino acids allow hydrogen bonding to the solvent and glycines provide the necessary flexibility. These properties prevent the penetration of the linker peptide into the hydrophobic interface formed in the association of the domains. Furthermore, the linkers are not able to form an ordered secondary structure. The term "essentially consist of glycine and serine residues" is intended to mean that at least 60% or 65%, preferably 70% or 75%, more preferably 80%, 82%, 84%, 86% or 88%, even more preferably 90%, 92%, 93%, 94%, 95%, 96%, 97%, 98% or 99% of the amino acid residues within the flexible linker sequence are glycine and/or serine residues. Most preferably, all amino acid residues within the flexible linker sequence are glycine and/or serine residues.

The length of the linker sequences should be selected such that the linker peptides are too short to allow pairing of the domains of the same amino acid chain, but favour pairing between domains in two adjacent chains. Thus, the length of the linker sequence may be 1 to 20 amino acids, preferably 2 to 15 amino acids, more preferably 3 to 10 amino acids, even more preferably 4 to 8 amino acids and most preferably 5 amino acids.

Most preferably, the flexible linker sequence has the amino acid sequence GGGGS.

The flexible linker sequence is present in at least one copy, preferably 1 to 5 copies, more preferably 2 to 4 copies and most preferably three copies. If multiple copies of the flexible linker sequence are used every copy is preferably encoded by different codons to avoid interference.

Preferably, stretches containing exon sequences are inserted between the nucleic acid sequences coding for the C-terminal part of the intein and the C-terminal part of the reporter protein and between the N-terminal part of the reporter protein and the N-terminal part of the intein. As protein-splicing is dependent on the chemical nature of the splice-site junction amino acids, the insertion of stretches containing exon sequences is supposed to increase the efficiency of protein trans-splicing (Sun et al. (2001) Appl. Environ. Microbiol. 67: 1025 - 1029). Thus, the inserted exon sequences provide the parts of the exteins which are advantageous for the function of the corresponding intein.

Hence, preferably the first expression cassette comprises a nucleic acid sequence coding for an amino acid sequence comprising the amino acid sequence RESG or fragments of said sequence from the DnaB extein and the second expression cassette comprises a nucleic acid sequence coding for an amino acid sequence comprising an amino acid sequence selected from the group consisting of SEEQDHG and SIEQD or fragments of said sequences. Preferably, the second expression cassette comprises a nucleic acid sequence coding for an amino acid sequence comprising the amino acid sequence SIEQD or fragments of said sequence. "Fragments of the RESG extein sequence" may be for example ESG or SG. "Fragments of the SIEQD extein sequence" may be for example IEQD, EQD or QD. "An amino acid sequence comprising the amino acid sequence RESG" or " an amino acid sequence comprising an amino acid sequence selected from the group consisting of SEEQDHG and SIEQD" may be an amino acid sequence with one or more amino acid residues in addition to the RESG, SEEQDHG and SIEQD sequence, respectively. Preferably, the one or more additional amino acid residues are located in the N-terminus of the RESG, SEEQDHG and SIEQD sequence, respectively.

Further, the third expression cassette may comprise a nucleic acid sequence coding for an amino acid sequence comprising the amino acid sequence DVKFAEY or fragments of said sequence from the DnaE extein and the fourth expression cassette may comprise a nucleic acid sequence coding for an amino acid sequence comprising the amino acid sequence CFNGH or fragments of said sequences. "Fragments of the DVKFAEY extein sequence" may be for example DVKFA or DVKF. "Fragments of the CFNGH extein sequence" may be for example CFNG or CFN. "An amino acid sequence comprising the amino acid sequence DVKFAEY " or " an amino acid sequence comprising the amino acid sequence CFNGH" may be an amino acid sequence with one or more amino acid residues in addition to the DVKFAEY and CFNGH sequence, respectively. Preferably, the one or more additional amino acid residues are located in the N-terminus of the DVKFAEY and CFNGH sequence, respectively.

For the introduction of DNA into a plant host cell there are a number of well-known techniques available and the person skilled in the art can determine the appropriate method in each case without any problem. These techniques include the transformation of plant cells with T-DNA by using *Agrobacterium tumefaciens* or *Agrobacterium rhizogenes* as a transformation agent, the fusion of protoplasts, the direct gene transfer of isolated DNA into protoplasts, the electroporation of DNA, the introduction of DNA by means of the biolistic method, as well as other possibilities. Thereby, both stable and transient transformants can be generated.

As in the method of the present invention the integration of a single copy of the first and second expression cassette into the first and second locus, respectively, is desired, although not necessary for the functioning of the method of the invention, the expression cassettes are preferably introduced into the cells by a transformation method which favours single copy integration, such as *Agrobacterium*-mediated transformation.

For the injection and electroporation of DNA into plant cells there are no special requirements per se for the plasmids used. The same applies for direct gene transfer. Simple plasmids, such as pUC derivates may be used. If, however, whole plants are to be regenerated from such transformed cells, the presence of a selectable marker gene is necessary. The person skilled in the art is acquainted with the current selection markers, and will have no problem in selecting an appropriate marker. Standard selection markers are those which mediate resistance to a biocide or an antibiotic such as kanamycin, G418, bleomycin, hygromycin, methotrexate, glyphosate, streptomycin, sulfonyl urea, gentamycin or phosphinotricin and suchlike, to the transformed plant cell.

Dependent upon the method of introduction of the desired gene into the plant cell, other DNA sequences may be required. If, for example, the Ti or Ri plasmid is used for the transformation of the plant cell, at least the right flanking region, often however the right and the left flanking region of the T-DNA contained in the Ti or Ri plasmid must be linked as a flanking region with the gene to be introduced.

If agrobacteria are used for the transformations, the DNA to be introduced must be cloned in special plasmids, either in an intermediary vector which cannot replicate in agrobacteria or in a binary vector which can replicate both in *E. coli* and in agrobacteria. The agrobacterium serving as a host cell should contain a plasmid which carries a vir region. The vir region is necessary for the transfer of the T-DNA into the plant cell. T-DNA can also be present. This type of transformed agrobacterium is used for the transformation of plant cells. The use of T-DNA for the transformation of plant cells has been intensively investigated and is sufficiently described in EP 0 120 515 A2. Both monocotyledonous and dicotyledonous plants or their cells are very accessible to transformation by means of vectors based on agrobacteria (Chan et al. (1993) Plant Mol. Biol. 22: 491-506).

For the transfer of DNA into the plant cell, plant explants can be cultivated specifically for this purpose with *Agrobacterium tumefaciens* or *Agrobacterium rhizogenes.* From the infected plant material (for example, pieces of leaf, stem segments, roots, but also protoplasts or suspension-cultivated plant cells) whole plants can be regenerated in an appropriate medium which can contain antibiotics or biocides for the selection of transformed cells. The regeneration of the plants takes place according to standard regeneration methods and using the common nutrient solutions. The plants and plant cells obtained in this way can be examined for the presence of the DNA introduced.

The person skilled in the art is acquainted with other possibilities for the introduction of foreign DNA, in particular into monocotyledonous plants or their cells, using the biolistic method (Wan and Lemaux (1994) Plant Physiol. 104: 37-48; Vasil et al. (1993) Bio/Technology 11: 1553-1558; Ritala et al. (1994) Plant Mol. Bio. 24: 317-325; Spencer et al. (1990) Theor. Appl. Genet. 79: 625-631), by protoplast transformation (see L. Willmitzer (1993) Transgenic Plants in: Biotechnology, A Multi-Volume Comprehensive Treatise (publisher: H.J. Rehm et al.), volume 2, 627-659, VCH Weinheim, Germany), electroporation of partially permeabilised cells as well as the introduction of DNA by means of glass tissues.

The transformed cells grow within the plant in the normal way (see also McCormick et al. (1986) Plant Cell Reports 5: 81-84). The resulting plants can be raised in the normal way and be crossed with plants which have the same transformed genetic disposition or other genetic dispositions. The resulting hybrid individuals have the respective phenotypical properties.

Two or more generations should be raised in order to ensure that the phenotypical feature remains stable and is inherited. Seeds should be harvested as well so as to ensure that the respective phenotype or other characteristics are maintained.

Similarly, by using the standard methods, transgenic lines can be determined which are homozygous for the expression cassettes of the present invention. In the method of the present invention, the first and the second plant may be homozygous or heterozygous for the first and second expression cassette, respectively. Preferably, the first and the second plant are homozygous for the first and the expression cassette.

After introducing the expression cassettes into plant cells, transgenic plants can be identified by conventional methods such as PCR or Southern Blot analysis of plant material. Furthermore, plants having a single-copy integration of the expression cassette may be identified by suitable methods such as Southern Blot analysis or quantitative PCR. Usually, a population of first plants carrying the first expression cassette on different loci and a population of second plants carrying the second expression cassette on different loci is identified and further investigated.

The loci into which the N- and C-terminal fragments are integrated are then mapped by suitable methods such as marker-based mapping and/or in situ fluorescence hybridization using a labeled oligonucleotide probe which hybridizes to a nucleic acid sequence present in the expression cassette.

After having identified plants in which the nucleic acid sequences coding for the N- and C-terminal fragments are integrated into homologous chromosomes, these plants are crossed and the pollen of the progeny of this cross is analyzed for the expression of the reporter protein. If the reporter protein is expressed in the pollen of the progeny, the N- and C-terminal fragments of the reporter protein were located on different loci on homologous chromosomes in the parent plants and meiotic crossing-over has taken place between the homologous chromosomes such that a functional reporter protein is expressed.

The term "crossing" refers to the process of bringing the pollen of the first plant in contact with the flower of the second plant, or vice versa, such that pollination and fertilization takes place. The crossing may be performed by manual intervention in the greenhouse by tearing anthers of one parent plant with tweezers just before anthesis and placing one anther into the closed flower of the crossing partner or it may occur naturally by wind or insects.

The pollen contains the microgametocytes of plants which produce the male gametes (sperm cells). The pollen is produced in the microsporangium contained in the anther of an angiosperm flower.

Instead of or in addition to analyzing the pollen of the F1 progeny for the expression of the reporter protein, the F1 progeny may also be crossed with a wild-type plant to obtain F2 progeny which is then analyzed for the expression of the reporter protein. Preferably, the seeds of the F2 progeny are analyzed for the expression of the reporter protein. If the seeds are analyzed, it is not necessary to grow the F2 progeny to mature plants.

Within the meaning of the present invention, the wild-type plant with which the F1 progeny is crossed does not contain the N- or C-terminal segment of a reporter gene or a gene which provides for male sterility. Further, a wild-type plant does not contain a nucleic acid sequence coding for a protein which is supposed to influence recombination frequency or a nucleic acid sequence inhibiting the expression of such a protein. However, the wild-type plants may contain other transgenes such as transgenes not involved in recombination such as a herbicide resistance gene. Preferably, the wild-type plant does not contain any transgene.

To avoid self-pollination of the F1 progeny, the F1 progeny is preferably male sterile. To produce male sterile F1 progeny, the first and the second plants further comprise a third and a fourth expression cassette, respectively, which comprises a nucleic acid sequence coding for the N- and C-terminal part, respectively, of a protein which provides for male sterility under the control of a tapetum-specific promoter. The third expression cassette is integrated into the same locus as the first expression cassette and the fourth expression cassette is integrated into the same locus as the second expression cassette. Hence, if crossing-over occurs between the first and the second locus, a functional protein conferring male sterility is restored and the F1 progeny is male sterile in addition to expressing the reporter protein.

The term "tapetum-specific promoter" within the meaning of the present invention is understood to mean that a nucleic acid sequence under the control of a tapetum-specific promoter region is expressed in the tapetum of plants. Particularly, a promoter is also tapetum-specific within the meaning of the present invention if the promoter region preferentially leads to the expression of the nucleic acid sequence in the tapetum in comparison to other cell types and leads to a significantly increased expression such as at least two-fold, preferably at least five-fold, particularly preferably at least ten-fold and most preferably at least fifty-fold increased expression in tapetum in comparison to other cell types. The expression of a nucleic acid sequence in different tissues and organs can be determined with *in situ* detection techniques known to the person skilled in the art. For example, a reporter gene such as β-glucuronidase may be expressed under the control of the promoter to be investigated and the activity of the reporter gene in different organs may be determined.

The term "tapetum" is known to the expert and is intended to mean the highly specialized, transient tissue surrounding the (micro-)spores and/or pollen grains during their development. Supplementary information can be derived from any plant anatomy or plant physiology book such as Strassburger, Lehrbuch der Botanik, 35. Auflage 2002, Spektrum Akademischer Verlag.

Suitable tapetum-specific promoters are known to the person skilled in the art and include the promoter of the rice osg6b gene (Tsuchiya et al. (1995) Plant Cell Physiol. 36: 487 - 494), the pca55 promoter from corn (WO 92/13956) and the pE1 and pT72 promoters from rice (WO 92/13957). Preferably, the tapetum-specific promoter is the promoter from the rice osg6B gene. However, the tapetum-specific promoter of the present invention does not have to be derived from a monocotyledonous plant, but can also be isolated from a dicotyledonous plant. Nevertheless, in a preferred embodiment the promoter should be capable of directing tapetum-specific expression of nucleic acid sequences operatively linked thereto in monocotyledonous plants.

The "protein which provides for male sterility" may be any protein the expression of which in the tapetum leads to male sterile plants by interfering with the function and development of pollen. Examples of such genes include RNases, ribosomal inhibitor proteins (Cho et al. (2001) Mol. Cells 11: 326 - 333), sucrose isomerase (WO 01/59135), protease and glucanase (Tsuchiya et al. (1995) Plant Cell Physiol. 36: 487 - 494). Preferably, the protein which provides for male sterility is an RNase. Examples for RNases are barnase (Mariani et al. (1990) Nature 347: 737 - 741; Mariani et al. (1992) Nature 357: 384 - 387), RNase T1 from *Aspergillus oryzae* (Denis et al. (1993) Plant Physiol. 101: 1295-1304) and rat pancreatic ribonuclease (Bernd-Souza et al. (2000) Genet. Mol. Biol. 23(2) 435-443).

Even more preferably, the protein which provides for male sterility is barnase, i.e. ribonuclease from *Bacillus amyloliquefaciens.* The amino acid sequence of barnase from *Bacillus amyloliquefaciens* is shown in SEQ ID No. 12 and the nucleic acid sequence coding for barnase is depicted in SEQ ID No. 11.

To obtain said 5' and 3' part of the nucleotide sequence coding for barnase, said nucleotide sequence is preferably split such that each obtained fragment, upon expression, is incapable of generating a functional barnase protein in the absence of the other fragment. Each barnase fragment contains a sequence portion necessary for the barnase function. The barnase may be split into said fragments in many different ways provided that expression of the male sterility requires all said fragments and binding thereof to each other. One can easily test experimentally whether a fragment generated by splitting the barnase nucleotide sequence at a randomly chosen site is capable of providing male sterility by expressing the fragment in the tapetum of plants and investigating if these plants are able to develop viable pollen. Preferably, the barnase is split into said fragments in the amino acid region between amino acid residues 30 and 40 of the mature protein, i.e. between amino acids 30 and 31, 31 and 32, 32 and 33, 33 and 34, 34 and 35, 35 and 36, 36 and 37, 37 and 38, 38 and 39 or 39 and 40 of the amino acid sequence according to SEQ ID No. 12.

More preferably, the N-terminal part of the barnase comprises 36 amino acids, and the C-terminal part of the barnase comprises 75 amino acids. Alternatively, the N-terminal part of barnase may comprise 35 amino acids if no methionine is added to the N-terminus.

Most preferably, the N-terminal part of the barnase is encoded by a nucleic acid sequence according to SEQ ID No. 13 and the C-terminal part of the barnase is encoded by a nucleic acid sequence according to SEQ ID No. 15. The N-terminal part of the barnase preferably has the amino acid sequence according to SEQ ID No. 14 and the C-terminal part of the barnase preferably has the amino acid sequence according to SEQ ID No. 16.

However, the invention is also intended to comprise smaller fragments of the barnase protein or "functional barnase fragments", as long as the N- and the C-terminal parts of such fragment restore a functional, enzymatically active protein upon intein-mediated trans-splicing which protein leads to male sterility when expressed in the tapetum of plants. For example, the C-terminal part may lack one or more amino acid residues at its C-terminus and/or the N-terminal part may lack one or more amino acid residues at its N-terminus. Upon intein-mediated trans-splicing a protein smaller than the wild-type protein is formed from the two fragments which protein is functional and enzymatically active. However, also deletions within the N- and/or the C-terminal part of barnase are conceivable as long as they do not affect the ability of the N- and the C-terminal part to restore a functional protein.

The tapetum-specific promoter used in the fourth expression cassette may be the same as the one used in the third expression cassette or it may be different from the promoter used in the third expression cassette, preferably the same tapetum-specific promoter is used in the third and the fourth expression cassette to achieve equimolar expression of the N- and the C-terminal fragments of the protein which provides for male sterility.

The second intein may be selected from the group consisting of DnaB and DnaE, both from the green-blue algae of Synechocystis spec and has to be different from the first intein. Hence, if the first intein is DnaB the second intein is DnaE and if the first intein is DnaE, the second intein is DnaB. Preferably, the secondt intein is DnaE and most preferably, the N-terminal part of DnaE is encoded by the nucleic acid sequence according to SEQ ID No. 17 and the C-terminal part of DnaE is encoded by the nucleic acid sequence according to SEQ ID No. 19. Preferably, the N-terminal part of DnaE has the amino acid sequence according to SEQ ID No. 18 and the C-terminal part of DnaE has the amino acid sequence according to SEQ ID No. 20.

The present invention may also be used to identify genetic factors such as proteins which influence the meiotic recombination frequency in plants. In this case, either the first and/or the second plant further comprises at least one expression cassette which comprises a nucleic acid sequence coding for a protein whose effect on recombination frequency is to be tested or a nucleic acid sequence inhibiting the expression of a protein whose effect on recombination frequency is to be tested.

The "protein whose effect on recombination frequency is to be tested" may be any protein which is supposed to influence the recombination frequency based on its known or proposed activity. Several classes of candidate genes which may influence recombination frequency have already been identified, *inter alia,* genes coding for proteins of the SMC (structural maintenance of chromosomes) class such as MIM from *Arabidopsis thaliana* (Mengiste et al. (1999) EMBO J. 18: 4505-4512), genes which code for components of the DNA repair mechanism such as CENTRIN2 from *Arabidopsis thaliana* (Molinier et al. (2004) Plant Cell 16: 1633-1643) and MLH1 of *Solanum lycopersicum* (WO 2007/030014 A2) and genes which are directly involved in the crossing-over process, such as SPO11 from yeast (Fukuda et al. (2008) Nucleic Acids Res. 36: 984-997) or Rad51 (WO 2007/030014 A2).

Furthermore, proteins such as the ones described above can be fused to a variety of different DNA binding domains, thus resulting in fusion proteins that could recruit the proteins, for example SPO11, to various sites on the DNA (Robine et al. (2007) Mol. Cell. Biol. 27: 1868-1880).

Besides over-expressing the proteins whose effect on recombination frequency is to be tested, the expression of these proteins can also be inhibited by known approaches such as antisense technology, RNAi, shRNA, siRNA and ribozyme technology.

Since it is not known whether the expression of proteins whose effect on meiotic recombination frequency should be investigated also promotes undesired somatic recombination events, it is preferred to use promoters which are exclusively active in gamete development, thereby restricting the activity of the potential recombination modulators spatially and temporally to meiosis. Such promoters which are exclusively active in gamete development include the AtDMCI promoter in pollen mother cells, microspore- or egg cell-specific promoters (Honys et al. (2006) BMC Plant Biol. 6:31; Klimyuk and Jones (1997) Plant J. 11:1-14).

Hence, the present disclosure also relates to a method for identifying proteins which influence meiotic recombination frequencies, comprising the following steps:

(a) obtaining a first plant having in a first locus of a nuclear chromosome a first expression cassette comprising in 5' to 3' direction:

- a promoter functional in plant cells;
- operatively linked thereto a nucleic acid sequence coding for the N-terminal part of a reporter protein;
- a nucleic acid sequence coding for the N-terminal part of a first intein; and
- optionally, operatively linked thereto a termination sequence functional in plant cells;

(b) obtaining a second plant having in a second locus of a nuclear chromosome homologous to said nuclear chromosome of step (a) a second expression cassette comprising in 5' to 3' direction:

- a promoter functional in plant cells;
- operatively linked thereto a nucleic acid sequence coding for the C-terminal part of said first intein;
- a nucleic acid sequence coding for the C-terminal part of said reporter protein; and
- optionally, operatively linked thereto a termination sequence functional in plant cells;
  wherein the first and the second locus are different and wherein both the N-terminal and the C-terminal part of the reporter protein contain a sequence necessary for the function of the reporter protein and wherein the first and/or the second plant comprises a further expression cassette comprising in 5' to 3' direction:
  - a promoter functional in plant cells;
  - operatively linked thereto a nucleic acid sequence coding for a protein the effect of which on recombination frequency is to be tested or a nucleic acid sequence inhibiting the expression of a protein the effect of which on recombination frequency is to be tested; and
  - optionally, operatively linked thereto a termination sequence functional in plant cells;

(c) crossing said first and said second plant to produce F1 progeny; and
(d) analyzing the pollen of the F1 progeny for the expression of the reporter protein.

Instead of, or in addition to introducing a nucleic acid sequence coding for a protein whose effect on recombination frequency should be investigated or a nucleic acid sequence inhibiting expression of said protein into the first and/or the second plant, the effect of treatment with a mutagen or irradiation or biotic or abiotic stimuli on meiotic recombination frequency can be determined. For example, seeds obtained from crossing the first and the second plant may be mutagenized by treating the seeds with known chemical mutagens such as ethylmethane sulfonate (EMS) or chemicals which are known to induce double strand breaks, such as micomycin, or irradiation such as X ray or UV irradiation. Suitable concentrations of the chemical mutagen are known to the person skilled in the art. For example, 2% (w/v) EMS may be used. Further, suitable irradiation doses are known to the skilled person and include a dose of 60 Gray for the X ray irradiation of seed material.

Furthermore, seeds obtained from crossing the first and the second plant or the $F_1$ progeny plants can be exposed to abiotic stress. Abiotic stress is the negative impact of non-living factors such as drought, high temperature, pH of the soil and/or salinity on the plants. Further, the plants may be exposed to pathogens such as fungi to determine whether pathogen infection has an effect on meiotic recombination frequencies.

Hence, the present disclosure also relates to a method of identifying factors which influence meiotic recombination frequencies, comprising the following steps:

(a) obtaining a first plant having in a first locus of a nuclear chromosome a first expression cassette comprising in 5' to 3' direction:

- a promoter functional in plant cells;
- operatively linked thereto a nucleic acid sequence coding for the N-terminal part of a reporter protein;
- a nucleic acid sequence coding for the N-terminal part of a first intein; and
- optionally, operatively linked thereto a termination sequence functional in plant cells;

(b) obtaining a second plant having in a second locus of a nuclear chromosome homologous to said nuclear chromosome of step (a) a second expression cassette comprising in 5' to 3' direction:

- a promoter functional in plant cells;
- operatively linked thereto a nucleic acid sequence coding for the C-terminal part of said first intein;
- a nucleic acid sequence coding for the C-terminal part of said reporter protein; and
- optionally, operatively linked thereto a termination sequence functional in plant cells; wherein the first and the second locus are different;

(c) crossing said first and said second plant;
(d) mutagenizing the seeds obtained from the crossing of step (c) and/or exposing said seeds to pathogens and/or abiotic stress;
(e) growing the seeds to F1 progeny and
(f) analyzing the pollen of the F1 progeny for the expression of the reporter protein.

The effect of the expression of the protein on the recombination frequency can be evaluated by calculating the induced meiotic recombination frequency (ki) by the following formula:

$$ki = ((2\ GUS^{pos})/N)-k$$

wherein k represents the natural recombination rate, i.e. the recombination rate in the absence of the protein the effect of which on recombination frequency is to be investigated and in the absence of a mutagen, irradiation, biotic and abiotic stimuli; GUS $P^{os}$ reprsents the number of plants/pollen showing expression of the reporter protein such as GUS; and N represents the number of F2-individuals or seed/F1 pollen/ analysed.

If the induced meiotic recombination frequency has a positive value, the protein or the conditions used have a positive influence on recombination frequency, and if the value for the meiotic recombination frequency is negative, the experimental conditions used have a negative effect on the meiotic recombination frequency.

A more complete understanding of the present invention can be obtained by reference to the following specific examples which are provided herein for the purposes of illustration only and are not intended to limit the scope of the invention.

**EXAMPLES**

**1) Plant material and growth conditions**

Barley *(Hordeum vulgare* L., cultivar "Golden Promise") is used throughout this study. For standard breeding, plants were grown under greenhouse conditions with 16 h of light at 20° C and 8 h of darkness at 16° C. For DNA isolation, plant tissues were harvested, frozen in liquid nitrogen, and stored at -80°C.

**2) Standard molecular biology techniques**

Standard molecular biology procedures were performed as described in Sambrook et al., 2001 (Molecular cloning: A laboratory manual, 3rd edition, Cold Spring Harbor Laboratory Press).

**3) Construction of the vector plasmids**

All vectors used in this study (see Fig. 2) are pBIN19-based binary vectors and were constructed using standard recombinant DNA methods. Major parts of the T-DNA elements have been produced by chemical synthesis (GENEART, Regensburg, Germany).

Split-GUS-System

All vectors are derivates of the "basal" GUS-vectors pHW121 and pHW161. In terms of the GUS-system, the vectors correspond to plasmids that have been described for being functional in Arabidopsis earlier by Yang et al. (2003) Proc. Natl. Acad. Sci. USA (100): 3513-3518. For ligating the GUS-fragments, intein sequences from the Synechocystes sp gene DnaB were fused to the GUS gene fragments.

Protein splicing is depending on the chemical nature of the splice site junction amino acids. Hence, the insertion of stretches containing exon sequences is supposed to increase the efficiency of protein *trans*-splicing (Sun et al. (2001) Appl. Environ. Microbiol. 67: 1025-1029). Consequently, in some of the constructs used, the amino acid sequence RESG from the DnaB extein was introduced between GUS-N and DnaB-inteinN. Similar, SIEQD from the DnaB extein was introduced between DnaB-inteinC and GUS-C. Furthermore, several vectors contained flexible linkers that are introduced in frame between the dnaB InteinC and the Barnase-C fragment. The linkers should not exhibit a propensity for ordered secondary structure or any tendency to interfere with domain folding. Thus, the sequence Gly-Gly-Gly-Gly-Ser was selected to bridge the 5'- and 3' barnase fragments either in a double [(2x Gly-Gly-Gly-Gly-$Ser_2$); pHW301] or triple [3x $(Gly-Gly-Gly-Gly-Ser)_3$; pHW181, pHW511, pHW141, pHW521] configuration). In order to avoid repetitiveness of the sequences, a different codon usage was used for each of the GGGG motifs.

Barnase system For the analysis of the F2 progeny, the vectors contain split *barnase* genes which are fused to N- and C-terminal fragments of the Ssp DnaE intein. In accordance with the split-GUS system, the amino acid sequence CFNHG of the adjacent extein sequence of the *DnaE* gene was inserted between *DnaE* IntC and *Bar-C*. Similarly, the amino acid sequence DVKFAEY from the DnaE extein was introduced between and *Bar-N* and *DnaE* IntN. Additionally, a methionine starting codon was added at the 5' terminus of the DnaE IntC sequence.

Both barnase-intein fusions were transcribed from a tapetum-specific rice promoter (Tsuchiya et al. (1995) Plant Cell Physiol. 36: 487-494).

The intein-barnase and the intein-GUS fusions were chemically synthesized by GENEART, Regensburg, Germany.

Table 1: Overview of the vectors used in the experiments (see also Figure 2)

| **N-terminal** | **GUS-N** | **Extein-amino acid sequence RESG from the DnaB extein was introduced between GUS-N and DnaB-inteinN** | **Spacer GGGGS was inserted between GUS-N and DnaB-inteinN** | **Barnase-N** |
|---|---|---|---|---|
| pHW161 | + | - | - | - |
| pHW171 | + | + | - | - |
| pHW301 | + | + | 2x | - |
| pHW181 | + | + | 3x | - |
| 511 | + | + | 3x | + |
| **C-terminal** | **GUS-C** | **Extein-amino acid sequence SIEQD from the DnaB extein was introduced between DnaB-inteinC and GUS-C** | **Spacer GGGGS was inserted between DnaC-inteinN and GUS-C** | **Barnase-C** |
| pHW121 | + | - | - | - |
| pHW132 | + | + | - | - |
| pHW141 | + | + | 3x | - |
| pHW521 | + | + | 3x | + |

**5) Genetic transformation of barley plants via Agrobacterium and via biolistic particle bombardment**

a) Callus culture maintenance

Immature seeds of wheat were surface-sterilized by immersing them in 70 % ethanol for 3 min. The procedure was followed by incubation in 2.5 % sodium hypochlorite solution, including 0.01 % SDS, with shaking at 125 rpm for 7 min and subsequently by three washing steps in sterile distilled water. Immature embryos (1.0-2.5 mm in length, semitransparent) were excised aseptically and placed, with scutellum-side up, on MS culture medium (Duchefa, M0222; Murashige and Skoog 1962), containing 30 g/l sucrose, 2 mg/l 2,4-D (2,4-dichlorophenoxyacetic acid) and 0.25 % phytagel for solidification. Embryos that develop compact nodular calli were selected using a stereomicroscope and used for bombardment 14-21 days after isolation. The cultures were kept in the dark at 25° C.

b) Microprojectile bombardment of immature embryos

The gold coating procedure was done according to Sanford et al. (1993) Methods Enzymol. 217: 483-509 and following the original protocol of Bio-Rad (Munich, Germany). In some experiments, the coating procedure was modified according to an advanced protocol of Eliby et al. (2000) Abstracts of the 6th International Wheat Conference, Budapest, Hungary, 313.

For particle coating, 50 μl of gold suspension (0.6 Micron gold in 50% glycerol, 60 mg/ml) was mixed with 10 μl (1 μg/μl) plasmid-DNA, 50 μl 2.5 M $CaCl_2$ and 20 μl 0.1 M spermidine. In case of co-transformation, plasmids were mixed in a 1:1 ratio (5 μg each). The mixture was shaken for 2 min, followed by incubation at room temperature for 30 min, brief centrifugation and washing with 70% and 99.5% ethanol. Finally, the gold particle pellet was resuspended in 60 μl of 99.5% ethanol. For one bombardment procedure, 6 μl of the suspension was used. All manipulations were done at room temperature.

Microprojectile bombardment was performed utilizing the Biolistic PDS-1000/He Particle Delivery System (Bio-Rad, Munich, Germany). Prior to the bombardment, the immature embryos were pre-treated for four hours on MS medium supplemented with 100 g/l sucrose.

Approximately 50 Embryos were placed in the centre of a plate to form a circle with a diameter of about 10 mm. The shooting was carried out using a helium pressure of 900 psi, with 15 mm distance from a macrocarrier launch point to the stopping screen and 60 mm distance from the stopping screen to the target tissue. The distance between rupture disk and launch point of the macrocarrier was 12 mm. Finally, 16 hours after treatment, the calli were transferred to MS medium containing 60 g/l sucrose and grown in dark conditions for one week at 25°C.

c) Agrobacterium-mediated transformation of immature embryos

The transformation of barley plants via Agrobacterium was carried is carried out according a protocol from Wang et al. (1997) J. Genet. & Breed. 51:325-334.

d) Selection and regeneration

Embryogenic calli were transferred to MS medium with 2 mg/l 2,4-D and 150 mg/l hygromycin B for selection seven days after bombardment. The cultures were kept in the dark at 22° C. After 5-6 successive callus selection steps (total time: 4-6 months) callus tissue was subcultured in MS regeneration medium supplemented with 1 mg/l kinetin, 7 mg/l zeatin. Regenerating plantlets were transferred to jars with half strength hormone-free MS medium containing 50 mg/l hygromycin B for plant selection. Fully developed plantlets were acclimated for 7-10 days at room temperature in liquid medium containing four-fold diluted MS salts. Plants with developed roots were transferred into soil and grown under greenhouse conditions to maturity.

**6) Transient assay for vector functionality (see Figure 3).**

Combinations of T-DNAs that carry the N- and C-terminal parts of the *GUS* gene (under the control of constitutively expressed promoters) were introduced into leaves of *N. benthamiana* by syringe infiltration using *Agrobacterium* or by biolistic bombardment. Constructs were introduced into *Agrobacterium* strain GV3101:pMP90 and infiltrated into *Nicotiana benthamiana* leaves as described in Marillonnet et al. (2004) Proc. Natl. Acad. Sci. USA 101(18): 6852-6857. For the biolistic bombardment protocol: see above.

When delivered alone, N- or C-terminal vectors do not exhibit a marker-phenotype (according to Gils et al. 2008) Plant Biotechnol J. 6(3):226-35). However, when vectors containing the N- and C-terminal fragments were delivered together by mixing the *Agrobacterium* suspensions or synchronous bombardment, sectors of marker-gene-phenotypes are visible in the affected tissue.

**7) Arabidopsis thaliana transformation**

Vectors were transformed into *Agrobacterium tumefaciens* strain GV3101:pMP90. *Arabidopsis thaliana* plants were transformed using a floral dipping protocol (Clough and Bent (1998) Plant J. 16(6): 735-743). Selection of transgenic plants was performed on MS plates containing 20 mg/l Hygromycin or 50 mg/l Kanamycin.

**8) Molecular analysis of transformants**

a) PCR

The isolation of total plant DNA and PCR amplifications were carried out according to Rubtsova et al. (2008) Plant Cell Rep. 27: 1821-1831.

Primers used were a) for the detection of the N-terminal GUS-intein fusion GUS-N FW (gtctggatcgcgaaaactg) and GUS-N Rev (ctgccagttcagttcgttg), b) for the detection of the C-terminal GUS-intein fusion GUS-C FW (gtggtggccaatggtgatg) and GUS-C REV (ggagttggccccaatccag), c) for the detection of the N-terminal barnase-Intein fusion Barnase-N FW (caagtgattaacaccttcgac) and DnaEIntein-N Rev (ccagcgtcaagtaatggaaag), d) for the detection of the C-terminal barnase-intein fusion Barnase-C REV (gatcttggtgaaggtctggtag) and DnaEIntein-C FW (gtcgtcgttccctcggagtgc).

b) Southern Blotting

Southern blots were performed according to Southern (1992) Biotechnology 24: 122-139.

Total DNA (10 μg) was digested with suitable endonucleases and the DNA fragments were separated using 0.8% agarose gels and transferred onto a nylon membrane (Pall Biodyne B, USA) according to the standard procedure (Southern, 1992). The membranes were hybridized with [$^{32}$P]-labelled DNA fragments.

**8) Cross pollinations**

Pollination of male-sterile plants was performed by tearing anthers of untransformed Arabidopsis/barley plants with tweezers just before anthesis and placing one anther into the closed flower of the male-sterile crossing partner.

**9) Fertility assays**

To check for the viability of pollen, anthers of control wild-type and male sterile plants were stained with Alexander stain (Alexander (1969) Stain Technol. 44: 117-122). The assays were performed prior to 2-3 days before anthesis.

**11) Colorimetric β-glucuronidase activity assay**

Plant tissue or pollen was analysed for GUS activity using histochemical staining (Jefferson (1987) Plant Mol. Biol. Rep. 5: 387-405).
Material was incubated overnight in 96-microwell plates at 37°C with phosphate buffer (50 mM sodium phosphate pH 7.0, 1 mM EDTA, 0.1% Triton-X-100) containing 1 mM X-Gluc and GUS activity was detected due to the blue staining.

## Claims

1. Method for determining meiotic recombination frequencies in plants, comprising the following steps:

   (a) obtaining a first plant having in a first locus of a nuclear chromosome a first expression cassette comprising in 5' to 3' direction:

   - a promoter functional in plant cells;
   - operatively linked thereto a nucleic acid sequence coding for the N-terminal part of a reporter protein;
   - a nucleic acid sequence coding for the N-terminal part of a first intein; and
   - optionally, operatively linked thereto a termination sequence functional in plant cells;

   (b) obtaining a second plant having in a second locus of a nuclear chromosome homologous to said nuclear chromosome of step (a) a second expression cassette comprising in 5' to 3' direction:

   - a promoter functional in plant cells;
   - operatively linked thereto a nucleic acid sequence coding for the C-terminal part of said first intein;
   - a nucleic acid sequence coding for the C-terminal part of said reporter protein; and
   - optionally, operatively linked thereto a termination sequence functional in plant cells;

   wherein the first and the second locus are different and wherein both the N-terminal and the C-terminal part of the reporter protein contain a sequence portion necessary for the function of the reporter protein;

   (c) crossing said first and said second plant to produce F1 progeny; and
   (d) analyzing the pollen of the F1 progeny for the expression of the reporter protein.

2. Method according to claim 1, additionally comprising step (e) of crossing a plant of the F1 progeny with a wild-type plant to produce F2 progeny and analyzing the F2 progeny for the expression of the reporter protein.

3. Method according to claim 1, wherein step (d) is replaced with step (d') of crossing a plant of the F1 progeny with a wild-type plant to produce F2 progeny and analyzing the F2 progeny for the expression of the reporter protein.

4. Method according to any of the preceding claims, further comprising the step of calculating the meiotic recombination frequency.

5. Method according to any of the preceding claims, wherein the reporter protein is β-glucuronidase.

6. Method according to any of the preceding claims, wherein the first intein is selected from the group consisting of DnaB and DnaE.

7. Method according to any of claims 3 to 6, wherein said first plant further comprises a third expression cassette comprising in 5' to 3' direction:

- a tapetum-specific promoter functional in plant cells;
- operatively linked thereto a nucleic acid sequence coding for the N-terminal part of a protein which provides for male sterility;
- a nucleic acid sequence coding for the N-terminal part of a second intein; and
- optionally, operatively linked thereto a termination sequence functional in plant cells;

and wherein said second plant comprises a fourth expression cassette comprising in 5' to 3' direction:

- a tapetum-specific promoter functional in plant cells;
- operatively linked thereto a nucleic acid sequence coding for the C-terminal part of said second intein;
- a nucleic acid sequence coding for the C-terminal part of said protein which provides for male sterility; and
- optionally, operatively linked thereto a termination sequence functional in plant cells;

wherein the second intein is different from the first intein.

8. Method according to claim 7, wherein the protein which provides for male sterility is an Rnase.

9. Method according to any of claims 7 or 8, wherein said second intein is selected from the group consisting of DnaB and DnaE.

10. Method according to any of the preceding claims, wherein said first and said second plant is a monocotyledonous plant.

11. Method according to any of the preceding claims, wherein said first and/or said second plant further comprises at least one expression cassette comprising:

- a promoter functional in plant cells;
- operatively linked thereto a nucleic acid sequence coding for a protein the effect of which on recombination frequency is to be tested or a nucleic acid sequence inhibiting the expression of a protein the effect of which on recombination frequency is to be tested; and
- optionally, operatively linked thereto a termination sequence functional in plant cells.

12. Method according to claim 11, wherein said promoter is a promoter which is exclusively active during gamete development.

13. Method according to any of the preceding claims, further comprising the step of mutagenizing the seeds obtained from crossing the first and the second plants and/or exposing the seeds obtained from crossing the first and the second plants or the F1 progeny plants to biotic or abiotic stress.

14. Method according to any of claims 11 to 13, further comprising a step of calculating the meiotic recombination frequency induced by the protein the effect of which on recombination frequency is to be tested or by mutagenesis or exposure to biotic or abiotic stress.

## Patentansprüche

1. Verfahren zur Bestimmung von meiotischen Rekombinationshäufigkeiten in Pflanzen, umfassend die folgenden Schritte:

(a) Erhalten einer ersten Pflanze, die in einem ersten Lokus eines nukleären Chromosoms eine erste Expressionskassette aufweist, die in 5' nach 3'-Richtung umfasst:

- einen Promotor, der in Pflanzenzellen funktionell ist;
- operativ daran gebunden eine Nukleinsäuresequenz, die für den N-terminalen Teil eines Reporterproteins kodiert;
- eine Nukleinsäuresequenz, die für den N-terminalen Teil eines ersten Inteins kodiert; und
- ggf. operativ daran gebunden eine Terminationssequenz, die in Pflanzenzellen funktionell ist;

(b) Erhalten einer zweiten Pflanze, die in einem zweiten Lokus eines nukleären Chromosoms, das zum nukleären Chromosom von Schritt (a) homolog ist, eine zweite Expressionskassette aufweist, die in 5' nach 3'-Richtung umfasst:

- einen Promotor, der in Pflanzenzellen funktionell ist;
- operativ daran gebunden eine Nukleinsäuresequenz, die für den C-terminalen Teil des ersten Inteins kodiert;
- eine Nukleinsäuresequenz, die für den C-terminalen Teil des Reporterproteins kodiert; und
- ggf. operativ daran gebunden eine Terminationssequenz, die in Pflanzenzellen funktionell ist;
wobei der erste und der zweite Lokus unterschiedlich sind und wobei sowohl der N-terminale als auch der C-terminale Teil des Reporterproteins einen Sequenzteil enthalten, der für die Funktion des Reporterproteins erforderlich ist;

(c) Kreuzen der ersten und der zweiten Pflanze, um F1-Nachkommen zu erzeugen; und
(d) Analysieren des Pollens der F1-Nachkommen hinsichtlich der Expression des Reporterproteins.

**2.** Verfahren nach Anspruch 1, zusätzlich umfassend Schritt (e) des Kreuzens einer Pflanze der F1-Nachkommen mit einer Wildtyp-Pflanze, um F2-Nachkommen zu erzeugen und Analysieren der F2-Nachkommen hinsichtlich der Expression des Reporterproteins.

**3.** Verfahren nach Anspruch 1, wobei Schritt (d) durch Schritt (d') des Kreuzens einer Pflanze der F1-Nachkommen mit einer Wildtyp-Pflanze, um F2-Nachkommen zu erzeugen, und Analysieren der F2-Nachkommen hinsichtlich der Expression des Reporterproteins ersetzt wird.

**4.** Verfahren nach einem der vorhergehenden Ansprüche, weiter umfassend den Schritt des Berechnens der meiotischen Rekombinationshäufigkeit.

**5.** Verfahren nach einem der vorhergehenden Ansprüche, wobei das Reporterprotein β-Glucuronidase ist.

**6.** Verfahren nach einem der vorhergehenden Ansprüche, wobei das erste Intein ausgewählt ist aus der Gruppe bestehend aus DnaB und DnaE.

**7.** Verfahren nach einem der Ansprüche 3 bis 6, wobei die erste Pflanze weiter umfasst eine dritte Expressionskassette, die in 5' nach 3'-Richtung umfasst:

- einen Tapetum-spezifischen Promotor, der in Pflanzenzellen funktionell ist;
- operativ daran gebunden eine Nukleinsäuresequenz, die für den N-terminalen Teil eines Proteins kodiert, das männliche Sterilität herbeiführt;
- eine Nukleinsäuresequenz, die für den N-terminalen Teil eines zweiten Inteins kodiert; und
- ggf. operativ daran gebunden eine Terminationssequenz, die in Pflanzenzellen funktionell ist;
und wobei die zweite Pflanze eine vierte Expressionskassette umfasst, die in 5' nach 3'-Richtung umfasst:

- einen Tapetum-spezifischen Promotor, der in Pflanzenzellen funktionell ist;
- operativ daran gebunden eine Nukleinsäuresequenz, die für den C-terminalen Teil des zweiten Inteins kodiert;
- eine Nukleinsäuresequenz, die für den C-terminalen Teil des Proteins kodiert, das männliche Sterilität herbeiführt; und
- ggf. operativ daran gebunden eine Terminationssequenz, die in Pflanzenzellen funktionell ist;
wobei sich das zweite Intein vom ersten Intein unterscheidet.

**8.** Verfahren nach Anspruch 7, wobei das Protein, das die männliche Sterilität herbeiführt, eine Rnase ist.

**9.** Verfahren nach einem der Ansprüche 7 oder 8, wobei das zweite Intein ausgewählt ist aus der Gruppe bestehend aus DnaB und DnaE.

**10.** Verfahren nach einem der vorhergehenden Ansprüche, wobei die erste und die zweite Pflanze eine monokotyledone Pflanze ist.

**11.** Verfahren nach einem der vorhergehenden Ansprüche, wobei die erste und/oder die zweite Pflanze weiter umfasst mindestens eine Expressionskassette umfassend:

- einen Promotor, der in Pflanzenzellen funktionell ist;
- operativ daran gebunden eine Nukleinsäuresequenz, die für ein Protein kodiert, dessen Wirkung auf die Rekombinationshäufigkeit untersucht werden soll oder eine Nukleinsäuresequenz, die die Expression eines Proteins hemmt, dessen Wirkung auf die Rekombinationshäufigkeit untersucht werden soll; und
- ggf. operativ daran gebunden eine Terminationssequenz, die in Pflanzenzellen funktionell ist.

**12.** Verfahren nach Anspruch 11, wobei der Promotor ein Promotor ist, der ausschließlich während der Keimzellentwicklung aktiv ist.

**13.** Verfahren nach einem der vorhergehenden Ansprüche, weiter umfassend den Schritt des Mutagenisierens der Samen, die aus der Kreuzung der ersten und der zweiten Pflanzen erhalten wurden und/oder das Aussetzen der Samen, die aus der Kreuzung der ersten und der zweiten Pflanzen erhalten wurden oder der F1-Nachkommenpflanzen gegenüber biotischem oder abiotischem Stress.

**14.** Verfahren nach einem der Ansprüche 11 bis 13, weiter umfassend einen Schritt des Berechnens der meiotischen Rekombinationshäufigkeit, die durch das Protein, dessen Wirkung auf die Rekombinationshäufigkeit getestet werden soll oder durch Mutagenese oder durch Aussetzen gegenüber biotischem oder abiotischem Stress induziert wird.

## Revendications

**1.** Procédé de détermination de fréquences de recombinaison méiotique dans des plantes, comprenant les étapes suivantes :

(a) obtention d'une première plante présentant, dans un premier locus d'un chromosome nucléaire, une première cassette d'expression comprenant en direction 5' à 3' :

- un promoteur fonctionnel dans des cellules végétales ;
- liée de manière opérationnelle à celui-ci, une séquence d'acide nucléique codant la partie N-terminale d'une protéine rapporteuse ;
- une séquence d'acide nucléique codant la partie N-terminale d'une première intéine ;
et
- facultativement, liée de manière opérationnelle à celle-ci, une séquence de terminaison fonctionnelle dans des cellules végétales ;

(b) obtention d'une deuxième plante présentant, dans un deuxième locus d'un chromosome nucléaire homologue audit chromosome nucléaire de l'étape (a), une deuxième cassette d'expression comprenant en direction 5' à 3' :

- un promoteur fonctionnel dans des cellules végétales ;
- liée de manière opérationnelle à celui-ci, une séquence d'acide nucléique codant la partie C-terminale de ladite première intéine ;
- une séquence d'acide nucléique codant la partie C-terminale de ladite protéine rapporteuse ; et
- facultativement, liée de manière opérationnelle à celle-ci, une séquence de terminaison fonctionnelle dans des cellules végétales ;

sachant que le premier et le deuxième locus sont différents et sachant qu'à la fois la partie N-terminale et la partie C-terminale de la protéine rapporteuse contiennent une portion de séquence nécessaire pour la fonction de la protéine rapporteuse ;

(c) croisement de ladite première et de ladite deuxième plante pour produire une progéniture F1 ; et
(d) analyse du pollen de la progéniture F1 pour l'expression de la protéine rapporteuse.

2. Procédé selon la revendication 1, comprenant en outre l'étape (e) consistant à croiser une plante de la progéniture F1 avec une plante de type sauvage pour produire une progéniture F2 et à analyser la progéniture F2 pour l'expression de la protéine rapporteuse.

3. Procédé selon la revendication 1, sachant que l'étape (d) est remplacée par l'étape (d') consistant à croiser une plante de la progéniture F1 avec une plante de type sauvage pour produire une progéniture F2 et à analyser la progéniture F2 pour l'expression de la protéine rapporteuse.

4. Procédé selon l'une quelconque des revendications précédentes, comprenant en outre l'étape consistant à calculer la fréquence de recombinaison méiotique.

5. Procédé selon l'une quelconque des revendications précédentes, sachant que la protéine rapporteuse est de la β-glucuronidase.

6. Procédé selon l'une quelconque des revendications précédentes, sachant que la première intéine est sélectionnée dans le groupe constitué par DnaB et DnaE.

7. Procédé selon l'une quelconque des revendications 3 à 6, sachant que la première plante comprend en outre une troisième cassette d'expression comprenant en direction 5' à 3' :

   - un promoteur spécifique du tapetum, fonctionnel dans des cellules végétales ;
   - liée de manière opérationnelle à celui-ci, une séquence d'acide nucléique codant la partie N-terminale d'une protéine qui confère la stérilité mâle ;
   - une séquence d'acide nucléique codant la partie N-terminale d'une deuxième intéine ; et
   - facultativement, liée de manière opérationnelle à celle-ci, une séquence de terminaison fonctionnelle dans des cellules végétales ;

   et sachant que ladite deuxième plante comprend une quatrième cassette d'expression comprenant en direction 5' à 3' :

     - un promoteur spécifique du tapetum, fonctionnel dans des cellules végétales ;
     - liée de manière opérationnelle à celui-ci, une séquence d'acide nucléique codant la partie C-terminale de ladite deuxième intéine ;
     - une séquence d'acide nucléique codant la partie C-terminale de ladite protéine qui confère la stérilité mâle ; et
     - facultativement, liée de manière opérationnelle à celle-ci, une séquence de terminaison fonctionnelle dans des cellules végétales ;

     sachant que la deuxième intéine est différente de la première intéine.

8. Procédé selon la revendication 7, sachant que la protéine qui confère la stérilité mâle est un Rnase.

9. Procédé selon l'une quelconque des revendications 7 ou 8, sachant que ladite deuxième intéine est sélectionnée dans le groupe constitué par DnaB et DnaE.

10. Procédé selon l'une quelconque des revendications précédentes, sachant que ladite première et ladite deuxième plante est une plante monocotylédone.

11. Procédé selon l'une quelconque des revendications précédentes, sachant que ladite première et/ou ladite deuxième plante comprend en outre au moins une cassette d'expression comprenant :

   - un promoteur fonctionnel dans des cellules végétales ;
   - liée de manière opérationnelle à celui-ci, une séquence d'acide nucléique codant une protéine dont l'effet sur la fréquence de recombinaison doit être testée ou une séquence d'acide nucléique inhibant l'expression d'une protéine dont l'effet sur la fréquence de recombinaison doit être testée ; et
   - facultativement, liée de manière opérationnelle à celle-ci, une séquence de terminaison fonctionnelle dans des cellules végétales.

12. Procédé selon la revendication 11, sachant que ledit promoteur est un promoteur qui est exclusivement actif pendant le développement de gamètes.

**13.** Procédé selon l'une quelconque des revendications précédentes, comprenant en outre l'étape consistant à effectuer la mutagenèse des graines obtenues à partir du croisement de la première et de la deuxième plante et/ou à exposer les graines obtenues à partir du croisement de la première et de la deuxième plante ou des plantes de progéniture F1 à un stress biotique ou abiotique.

**14.** Procédé selon l'une quelconque des revendications 11 à 13, comprenant en outre une étape consistant à calculer la fréquence de recombinaison méiotique induite par la protéine dont l'effet sur la fréquence de recombinaison doit être testée ou par mutagenèse ou exposition à un stress biotique ou abiotique.

Figure 1a

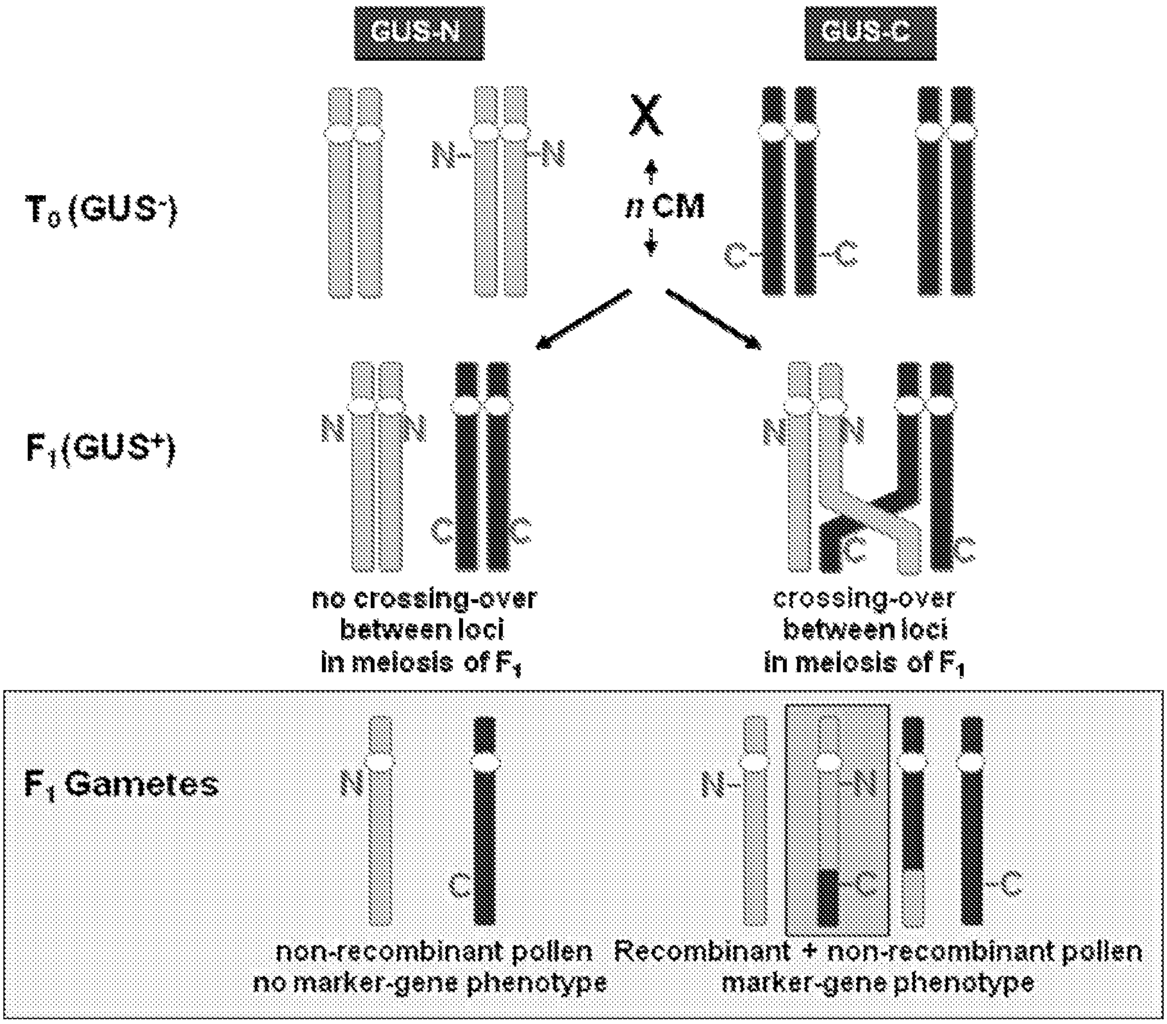
GUS-N
GUS-C
X
$T_0$ (GUS$^-$)
N
N
$n$ CM
C
C
$F_1$(GUS$^+$)
N
N
C
C
no crossing-over between loci in meiosis of $F_1$
crossing-over between loci in meiosis of $F_1$
$F_1$ Gametes
non-recombinant pollen no marker-gene phenotype
Recombinant + non-recombinant pollen marker-gene phenotype

Figure 1b

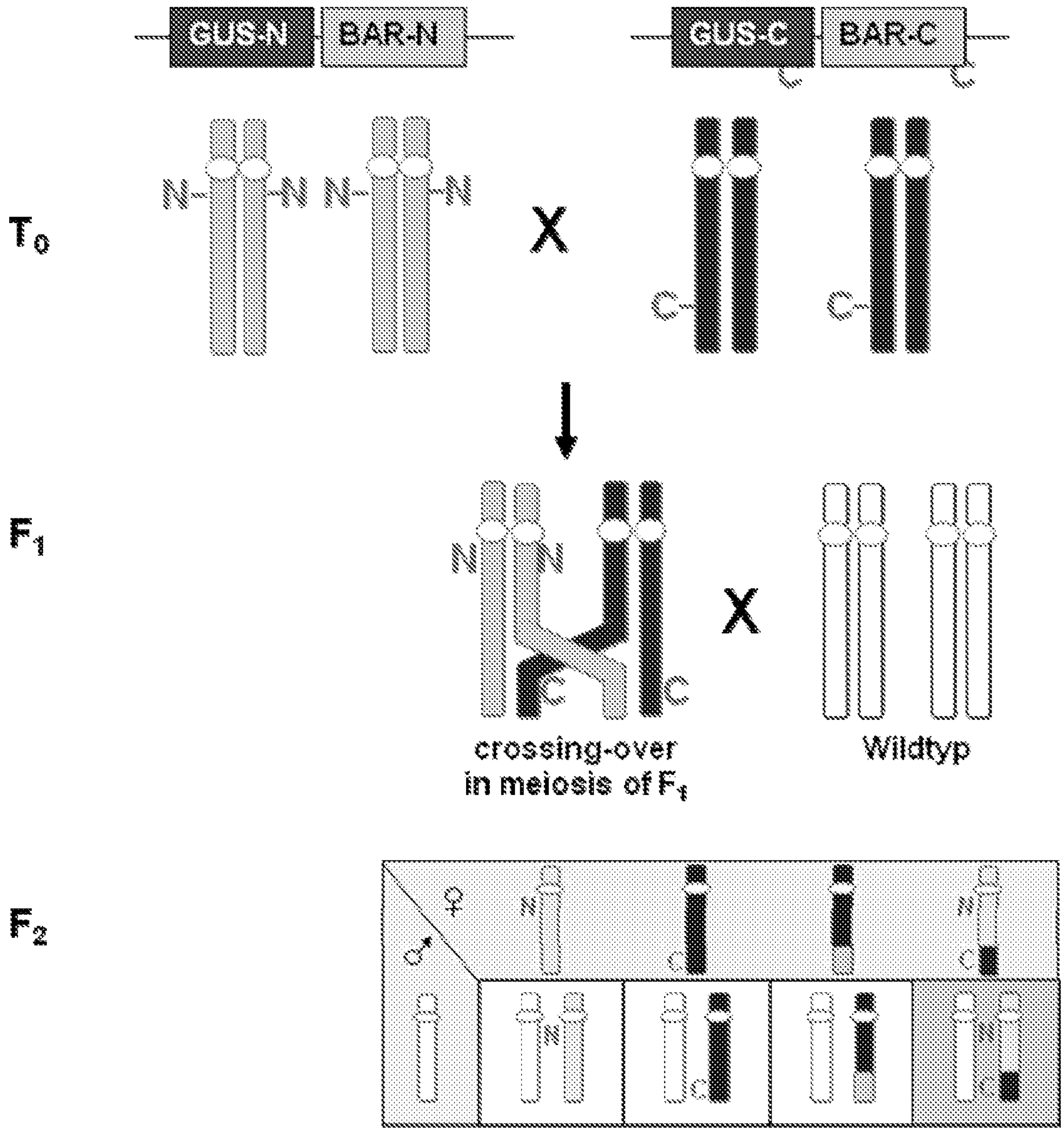
GUS-N
BAR-N
GUS-C
BAR-C
T0
N
C
X
F1
crossing-over
in meiosis of F1
Wildtyp
F2
♀
♂

Figure 1c

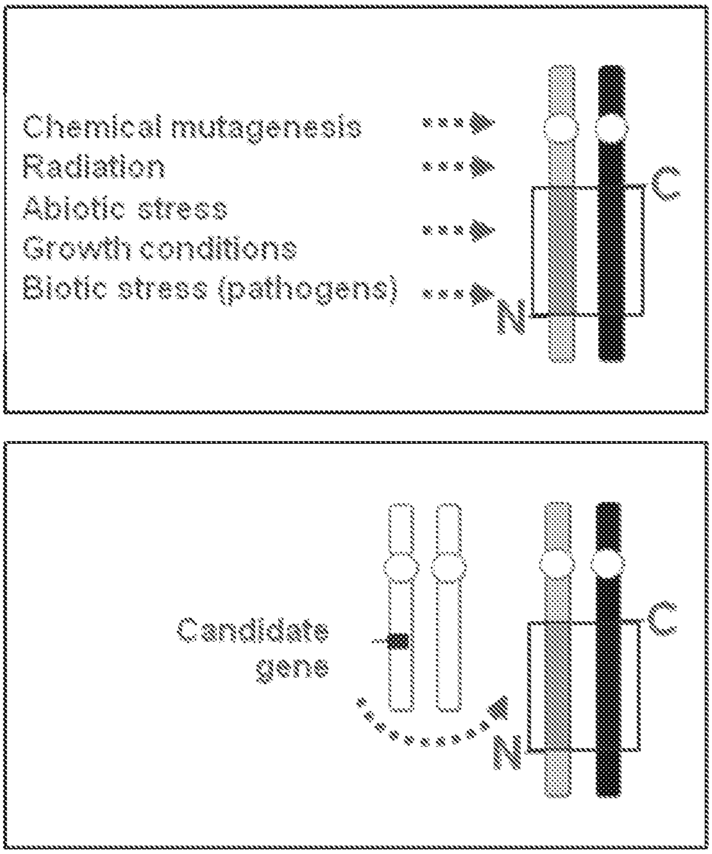
Chemical mutagenesis
Radiation
Abiotic stress
Growth conditions
Biotic stress (pathogens)
C
N
Candidate
gene
C
N

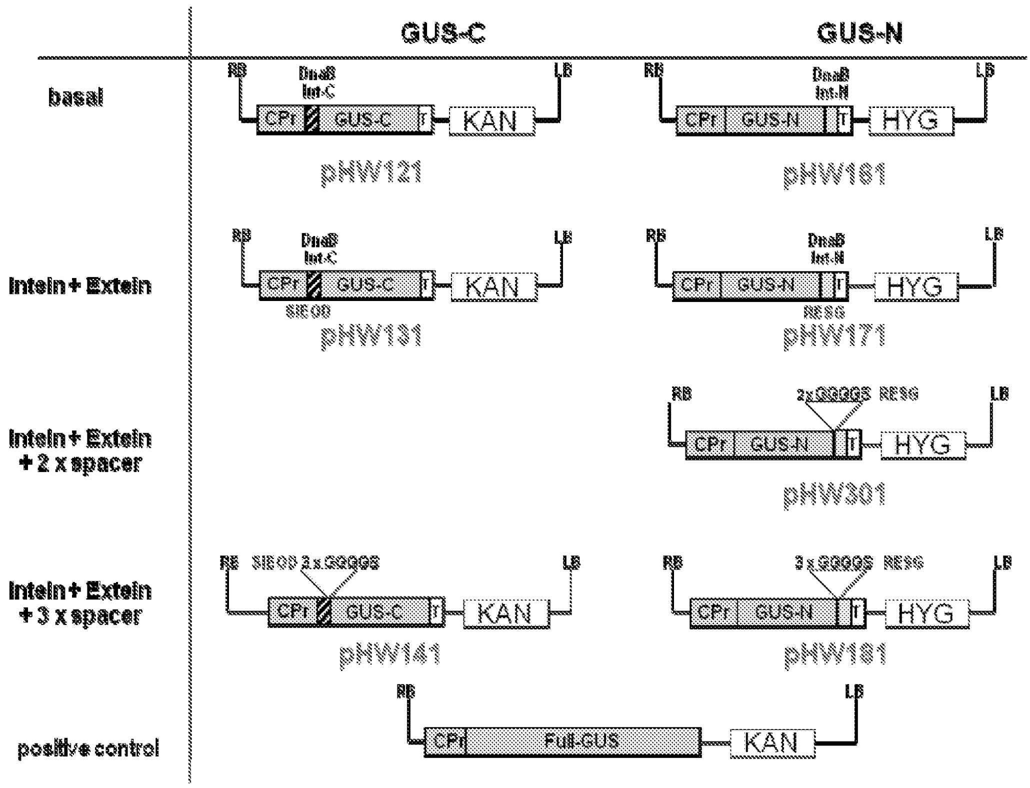
GUS-C
GUS-N
basal
RB
DnaB
Int-C
LB
CPr
GUS-C
T
KAN
pHW121
RB
DnaB
Int-N
LB
CPr
GUS-N
T
HYG
pHW161
Intein + Extein
SIEOD
pHW131
RESG
pHW171
Intein + Extein
+ 2 x spacer
2 x GGGGS
pHW301
Intein + Extein
+ 3 x spacer
3 x GGGGS
pHW141
pHW181
positive control
Full-GUS

Figure 2a

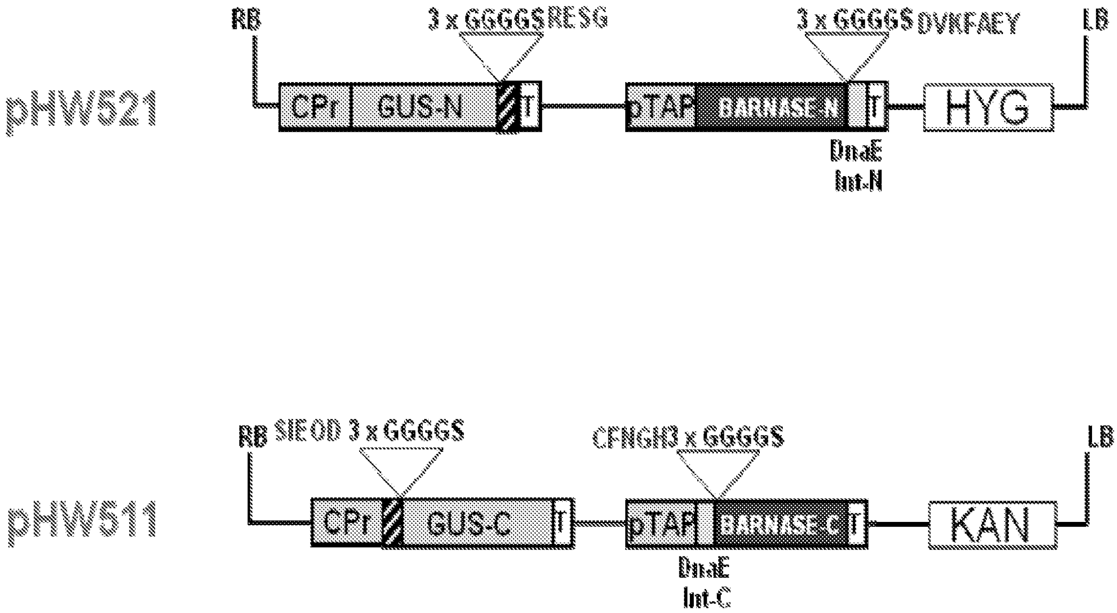
pHW521
RB
3 x GGGGS RESG
3 x GGGGS DVKFAEY
LB
CPr
GUS-N
T
pTAP
BARNASE-N
T
HYG
DnaE
Int-N
pHW511
RB
SIEOD 3 x GGGGS
CFNGH3 x GGGGS
LB
CPr
GUS-C
T
pTAP
BARNASE-C
T
KAN
DnaE
Int-C

Figure 2b

Figure 3

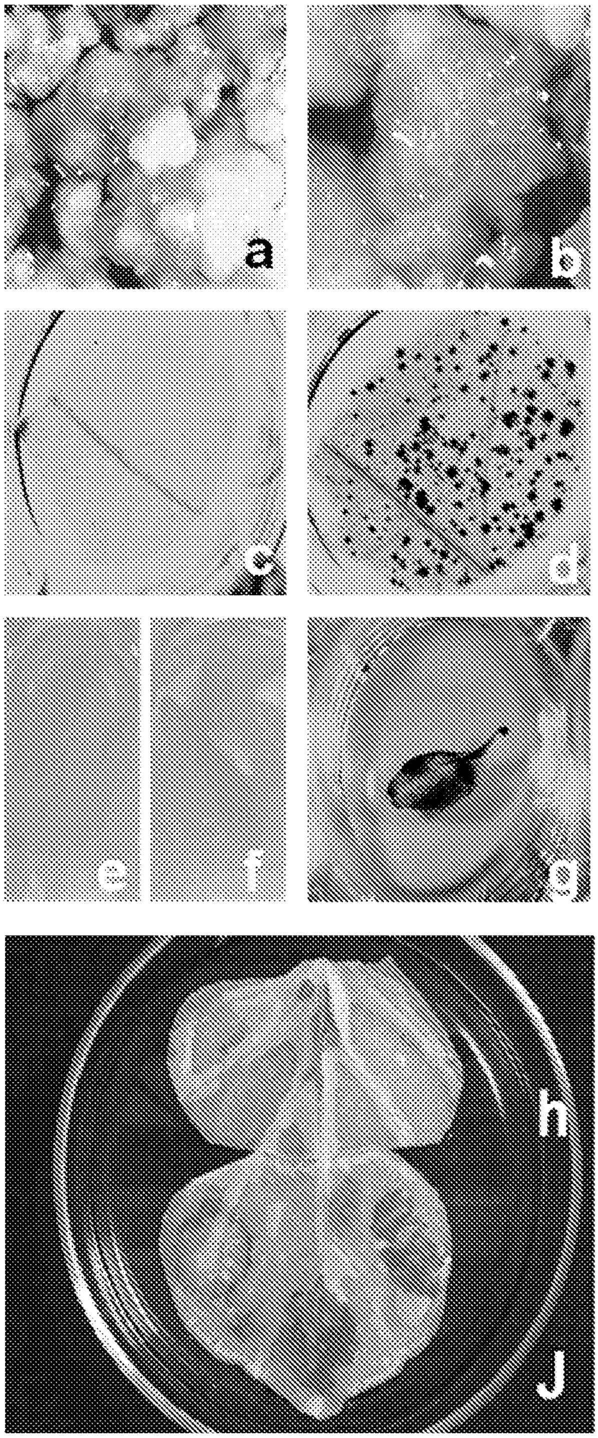

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0120515 A2
- WO 9213956 A
- WO 9213957 A
- WO 0159135 A
- WO 2007030014 A2


### Non-patent literature cited in the description


- **PETRONCZKI et al.** *Cell,* 2003, vol. 112 (4), 423-440
- **DROUAUD et al.** *Genome Res.,* 2006, vol. 16 (1), 106-114
- **MEZARD et al.** *Trends Gent.,* 2007, vol. 23 (2), 91-99
- **T. LENORMAND ; J. DUTHEIL.** *PLoS Biol,* vol. 3 (3), e63
- **E. SANCHEZ-MORAN et al.** *Genetics,* vol. 162 (3), 1415-1422
- **KATHIRIA et al.** *Plant Physiol.,* 2010, vol. 153 (4), 1859-1870
- **SIAUD et al.** *EMBO J.,* 2004, vol. 23, 1392-1401
- **COPENHAVER et al.** *Plant Physiol.,* 2000, vol. 124, 7-16
- **BERCHOWITZ ; COPENHAVER.** *Nat. Protoc.,* 2008, vol. 3 (1), 41-50
- **MOLINIER et al.** *Plant Cell,* 2004, vol. 16, 1633-1643
- **PUCHTA ; HOHN.** *Mol. Gen. Genet.,* 1991, vol. 230, 1-7
- **SWOBODA et al.** *EMBO J.,* 1994, vol. 13, 484-489
- **SAMBROOK et al.** Molecular cloning: A laboratory manual. Cold Spring Harbor Laboratory Press, 2001
- **MCELROY et al.** *Plant Cell,* 1990, vol. 2, 163-171
- **CHRISTENSEN et al.** *Transgenic Res.,* 1996, vol. 5, 213-218
- **JONES et al.** *Transgenic Res.,* 1992, vol. 1, 285-297
- **PAULMURUGAN et al.** *Proc. Natl. Acad. Sci. USA,* 2002, vol. 99 (24), 15608-15613
- **DEMIDOV et al.** *Proc. Natl. Acad. Sci. USA,* 2006, vol. 103 (7), 2052-2056
- **SOUTHWORTH et al.** *EMBO J.,* 1998, vol. 17, 918-926
- **WU et al.** *Proc. Natl. Acad. Sci. USA,* 1998, vol. 95, 9226-9231
- **SALEH ; PERLER.** *Chem. Rec.,* 2006, vol. 6, 183-193
- **PERLER.** *Cell,* 1998, vol. 92, 1-4
- **CHONG ; XU.** *J. Biol. Chem.,* 1997, vol. 272, 15587-15590
- **SUN et al.** *Appl. Environ. Microbiol.,* 2001, vol. 67, 1025-1029
- **CHAN et al.** *Plant Mol. Biol.,* 1993, vol. 22, 491-506
- **WAN ; LEMAUX.** *Plant Physiol.,* 1994, vol. 104, 37-48
- **VASIL et al.** *Bio/Technology,* 1993, vol. 11, 1553-1558
- **RITALA et al.** *Plant Mol. Bio.,* 1994, vol. 24, 317-325
- **SPENCER et al.** *Theor. Appl. Genet.,* 1990, vol. 79, 625-631
- **L. WILLMITZER ; H.J. REHM et al.** Transgenic Plants in: Biotechnology, A Multi-Volume Comprehensive Treatise. VCH Weinheim, 1993, vol. 2, 627-659
- **MCCORMICK et al.** *Plant Cell Reports,* 1986, vol. 5, 81-84
- **TSUCHIYA et al.** *Plant Cell Physiol.,* 1995, vol. 36, 487-494
- **CHO et al.** *Mol. Cells,* 2001, vol. 11, 326-333
- **TSUCHIYA et al.** *Cell Physiol.,* 1995, vol. 36, 487-494
- **MARIANI et al.** *Nature,* 1990, vol. 347, 737-741
- **MARIANI et al.** *Nature,* 1992, vol. 357, 384-387
- **DENIS et al.** *Plant Physiol.,* 1993, vol. 101, 1295-1304
- **BERND-SOUZA et al.** *Genet. Mol. Biol.,* 2000, vol. 23 (2), 435-443
- **MENGISTE et al.** *EMBO J.,* 1999, vol. 18, 4505-4512
- **FUKUDA et al.** *Nucleic Acids Res.,* 2008, vol. 36, 984-997
- **ROBINE et al.** *Mol. Cell. Biol.,* 2007, vol. 27, 1868-1880

- **HONYS et al.** *BMC Plant Biol.,* 2006, vol. 6, 31
- **KLIMYUK ; JONES.** *Plant J.,* 1997, vol. 11, 1-14
- **YANG et al.** *Proc. Natl. Acad. Sci. USA,* 2003, vol. 100, 3513-3518
- **SUN et al.** *Appl. Environ. Microbiol.,* 2001, vol. 67, 1025-1029
- **TSUCHIYA et al.** *Plant Cell Physiol.,* 1995, vol. 36, 487-494
- **SANFORD et al.** *Methods Enzymol.,* 1993, vol. 217, 483-509
- **ELIBY et al.** *Abstracts of the 6th International Wheat Conference, Budapest, Hungary,* 2000, 313
- **WANG et al.** *J. Genet. & Breed.,* 1997, vol. 51, 325-334
- **MARILLONNET et al.** *Proc. Natl. Acad. Sci. USA,* 2004, vol. 101 (18), 6852-6857
- **GILS et al.** *Plant Biotechnol J.,* 2008, vol. 6 (3), 226-35
- **CLOUGH ; BENT.** *Plant J.,* 1998, vol. 16 (6), 735-743
- **RUBTSOVA et al.** *Plant Cell Rep.,* 2008, vol. 27, 1821-1831
- **SOUTHERN.** *Biotechnology,* 1992, vol. 24, 122-139
- **ALEXANDER.** *Stain Technol.,* 1969, vol. 44, 117-122
- **JEFFERSON.** *Plant Mol. Biol. Rep.,* 1987, vol. 5, 387-405

SEQUENCE LISTING

<110> Leibniz-Institut fur Pflanzengenetik und Kulturpflanzenforschung (IPK) Nordsaat Saatzuchtgesellschaft mbH Saatzucht Langenstein

<120> Method of determining meiotic recombination frequencies in plants

<130> I 8252/RN

<160> 42

<170> PatentIn version 3.5

<210> 1
<211> 1824
<212> DNA
<213> E. coli

<400> 1

```
atgggtcagt cccttatgtt acgtcctgta gaaaccccaa cccgtgaaat caaaaaactc      60
gacggcctgt gggcattcag tctggatcgc gaaaactgtg gaattgatca gcgttggtgg     120
gaaagcgcgt tacaagaaag ccgggcaatt gctgtgccag gcagttttaa cgatcagttc     180
gccgatgcag atattcgtaa ttatgcgggc aacgtctggt atcagcgcga agtctttata     240
ccgaaaggtt gggcaggcca gcgtatcgtg ctgcgtttcg atgcggtcac tcattacggc     300
aaagtgtggg tcaataatca ggaagtgatg gagcatcagg gcggctatac gccatttgaa     360
gccgatgtca cgccgtatgt tattgccggg aaaagtgtac gtatcaccgt ttgtgtgaac     420
aacgaactga actggcagac tatcccgccg ggaatggtga ttaccgacga aaacggcaag     480
aaaaagcagt cttacttcca tgatttcttt aactatgccg gaatccatcg cagcgtaatg     540
ctctacacca cgccgaacac ctgggtggac gatatcaccg tggtgacgca tgtcgcgcaa     600
gactgtaacc acgcgtctgt tgactggcag gtggtggcca atggtgatgt cagcgttgaa     660
ctgcgtgatg cggatcaaca ggtggttgca actggacaag gcactagcgg gactttgcaa     720
gtggtgaatc cgcacctctg gcaaccgggt gaaggttatc tctatgaact gtgcgtcaca     780
gccaaaagcc agacagagtg tgatatctac ccgcttcgcg tcggcatccg gtcagtggca     840
gtgaagggcg aacagttcct gattaaccac aaaccgttct actttactgg ctttggtcgt     900
catgaagatg cggacttgcg tggcaaagga ttcgataacg tgctgatggt gcacgaccac     960
gcattaatgg actggattgg ggccaactcc taccgtacct cgcattaccc ttacgctgaa    1020
gagatgctcg actgggcaga tgaacatggc atcgtggtga ttgatgaaac tgctgctgtc    1080
ggctttaacc tctctttagg cattggtttc gaagcgggca acaagccgaa agaactgtac    1140
agcgaagagg cagtcaacgg ggaaactcag caagcgcact tacaggcgat taaagagctg    1200
atagcgcgtg acaaaaacca cccaagcgtg gtgatgtgga gtattgccaa cgaaccggat    1260
acccgtccgc aaggtgcacg ggaatatttc gcgccactgg cggaagcaac gcgtaaactc    1320
gacccgacgc gtccgatcac ctgcgtcaat gtaatgttct gcgacgctca caccgatacc    1380
atcagcgatc tctttgatgt gctgtgcctg aaccgttatt acggatggta tgtccaaagc    1440
ggcgatttgg aaacggcaga gaaggtactg gaaaaagaac ttctggcctg gcaggagaaa    1500

ctgcatcagc cgattatcat caccgaatac ggcgtggata cgttagccgg gctgcactca    1560
atgtacaccg acatgtggag tgaagagtat cagtgtgcat ggctggatat gtatcaccgc    1620
gtctttgatc gcgtcagcgc cgtcgtcggt gaacaggtat ggaatttcgc cgattttgcg    1680
acctcgcaag gcatattgcg cgttggcggt aacaagaaag ggatcttcac tcgcgaccgc    1740
aaaccgaagt cggcggcttt tctgctgcaa aaacgctgga ctggcatgaa cttcggtgaa    1800
aaaccgcagc agggaggcaa acaa                                           1824
```

<210> 2
<211> 608
<212> PRT
<213> E. coli

<400> 2

```
Met Gly Gln Ser Leu Met Leu Arg Pro Val Glu Thr Pro Thr Arg Glu
1               5                   10                  15

Ile Lys Lys Leu Asp Gly Leu Trp Ala Phe Ser Leu Asp Arg Glu Asn
            20                  25                  30

Cys Gly Ile Asp Gln Arg Trp Trp Glu Ser Ala Leu Gln Glu Ser Arg
        35                  40                  45

Ala Ile Ala Val Pro Gly Ser Phe Asn Asp Gln Phe Ala Asp Ala Asp
    50                  55                  60

Ile Arg Asn Tyr Ala Gly Asn Val Trp Tyr Gln Arg Glu Val Phe Ile
65                  70                  75                  80

Pro Lys Gly Trp Ala Gly Gln Arg Ile Val Leu Arg Phe Asp Ala Val
                85                  90                  95

Thr His Tyr Gly Lys Val Trp Val Asn Asn Gln Glu Val Met Glu His
            100                 105                 110

Gln Gly Gly Tyr Thr Pro Phe Glu Ala Asp Val Thr Pro Tyr Val Ile
        115                 120                 125

Ala Gly Lys Ser Val Arg Ile Thr Val Cys Val Asn Asn Glu Leu Asn
    130                 135                 140

Trp Gln Thr Ile Pro Pro Gly Met Val Ile Thr Asp Glu Asn Gly Lys
145                 150                 155                 160

Lys Lys Gln Ser Tyr Phe His Asp Phe Phe Asn Tyr Ala Gly Ile His
                165                 170                 175

Arg Ser Val Met Leu Tyr Thr Thr Pro Asn Thr Trp Val Asp Asp Ile
            180                 185                 190
```

```
Thr Val Val Thr His Val Ala Gln Asp Cys Asn His Ala Ser Val Asp
        195                 200                 205

Trp Gln Val Val Ala Asn Gly Asp Val Ser Val Glu Leu Arg Asp Ala
    210                 215                 220

Asp Gln Gln Val Val Ala Thr Gly Gln Gly Thr Ser Gly Thr Leu Gln
225                 230                 235                 240

Val Val Asn Pro His Leu Trp Gln Pro Gly Glu Gly Tyr Leu Tyr Glu
                245                 250                 255

Leu Cys Val Thr Ala Lys Ser Gln Thr Glu Cys Asp Ile Tyr Pro Leu
            260                 265                 270

Arg Val Gly Ile Arg Ser Val Ala Val Lys Gly Glu Gln Phe Leu Ile
        275                 280                 285

Asn His Lys Pro Phe Tyr Phe Thr Gly Phe Gly Arg His Glu Asp Ala
    290                 295                 300

Asp Leu Arg Gly Lys Gly Phe Asp Asn Val Leu Met Val His Asp His
305                 310                 315                 320

Ala Leu Met Asp Trp Ile Gly Ala Asn Ser Tyr Arg Thr Ser His Tyr
                325                 330                 335

Pro Tyr Ala Glu Glu Met Leu Asp Trp Ala Asp Glu His Gly Ile Val
            340                 345                 350

Val Ile Asp Glu Thr Ala Ala Val Gly Phe Asn Leu Ser Leu Gly Ile
        355                 360                 365

Gly Phe Glu Ala Gly Asn Lys Pro Lys Glu Leu Tyr Ser Glu Glu Ala
    370                 375                 380

Val Asn Gly Glu Thr Gln Gln Ala His Leu Gln Ala Ile Lys Glu Leu
385                 390                 395                 400

Ile Ala Arg Asp Lys Asn His Pro Ser Val Val Met Trp Ser Ile Ala
                405                 410                 415

Asn Glu Pro Asp Thr Arg Pro Gln Gly Ala Arg Glu Tyr Phe Ala Pro
            420                 425                 430

Leu Ala Glu Ala Thr Arg Lys Leu Asp Pro Thr Arg Pro Ile Thr Cys
        435                 440                 445

Val Asn Val Met Phe Cys Asp Ala His Thr Asp Thr Ile Ser Asp Leu
    450                 455                 460

Phe Asp Val Leu Cys Leu Asn Arg Tyr Tyr Gly Trp Tyr Val Gln Ser
```

```
465                 470                 475                 480

Gly Asp Leu Glu Thr Ala Glu Lys Val Leu Glu Lys Glu Leu Leu Ala
                485                 490                 495

Trp Gln Glu Lys Leu His Gln Pro Ile Ile Ile Thr Glu Tyr Gly Val
            500                 505                 510

Asp Thr Leu Ala Gly Leu His Ser Met Tyr Thr Asp Met Trp Ser Glu
        515                 520                 525

Glu Tyr Gln Cys Ala Trp Leu Asp Met Tyr His Arg Val Phe Asp Arg
    530                 535                 540

Val Ser Ala Val Val Gly Glu Gln Val Trp Asn Phe Ala Asp Phe Ala
545                 550                 555                 560

Thr Ser Gln Gly Ile Leu Arg Val Gly Gly Asn Lys Lys Gly Ile Phe
                565                 570                 575

Thr Arg Asp Arg Lys Pro Lys Ser Ala Ala Phe Leu Leu Gln Lys Arg
            580                 585                 590

Trp Thr Gly Met Asn Phe Gly Glu Lys Pro Gln Gln Gly Gly Lys Gln
        595                 600                 605
```

<210> 3
<211> 603
<212> DNA
<213> artificial

<220>

<223> nucleic acid sequence coding for the N-terminal part of GUS

<400> 3

```
atgggtcagt cccttatgtt acgtcctgta gaaaccccaa cccgtgaaat caaaaaactc        60
gacggcctgt gggcattcag tctggatcgc gaaaactgtg gaattgatca gcgttggtgg       120
gaaagcgcgt tacaagaaag ccgggcaatt gctgtgccag gcagttttaa cgatcagttc       180
gccgatgcag atattcgtaa ttatgcgggc aacgtctggt atcagcgcga agtctttata       240
ccgaaaggtt gggcaggcca gcgtatcgtg ctgcgtttcg atgcggtcac tcattacggc       300
aaagtgtggg tcaataatca ggaagtgatg gagcatcagg gcggctatac gccatttgaa       360
gccgatgtca cgccgtatgt tattgccggg aaaagtgtac gtatcaccgt ttgtgtgaac       420
aacgaactga actggcagac tatcccgccg ggaatggtga ttaccgacga aaacggcaag       480
aaaaagcagt cttacttcca tgatttcttt aactatgccg gaatccatcg cagcgtaatg       540
ctctacacca cgccgaacac ctgggtggac gatatcaccg tggtgacgca tgtcgcgcaa       600
gac                                                                     603
```

<210> 4
<211> 201
<212> PRT
<213> artificial

<220>
<223> amino acid sequence of the N-terminal part of GUS

<400> 4

```
Met Gly Gln Ser Leu Met Leu Arg Pro Val Glu Thr Pro Thr Arg Glu
1               5                   10                  15

Ile Lys Lys Leu Asp Gly Leu Trp Ala Phe Ser Leu Asp Arg Glu Asn
            20                  25                  30

Cys Gly Ile Asp Gln Arg Trp Trp Glu Ser Ala Leu Gln Glu Ser Arg
        35                  40                  45

Ala Ile Ala Val Pro Gly Ser Phe Asn Asp Gln Phe Ala Asp Ala Asp
    50                  55                  60

Ile Arg Asn Tyr Ala Gly Asn Val Trp Tyr Gln Arg Glu Val Phe Ile
65                  70                  75                  80

Pro Lys Gly Trp Ala Gly Gln Arg Ile Val Leu Arg Phe Asp Ala Val
                85                  90                  95

Thr His Tyr Gly Lys Val Trp Val Asn Asn Gln Glu Val Met Glu His
            100                 105                 110

Gln Gly Gly Tyr Thr Pro Phe Glu Ala Asp Val Thr Pro Tyr Val Ile
        115                 120                 125

Ala Gly Lys Ser Val Arg Ile Thr Val Cys Val Asn Asn Glu Leu Asn
    130                 135                 140

Trp Gln Thr Ile Pro Pro Gly Met Val Ile Thr Asp Glu Asn Gly Lys
145                 150                 155                 160

Lys Lys Gln Ser Tyr Phe His Asp Phe Phe Asn Tyr Ala Gly Ile His
                165                 170                 175

Arg Ser Val Met Leu Tyr Thr Thr Pro Asn Thr Trp Val Asp Asp Ile
            180                 185                 190

Thr Val Val Thr His Val Ala Gln Asp
        195                 200
```

<210> 5
<211> 1221
<212> DNA
<213> artificial

<220>
<223> nucleic acid sequence coding for the C-terminal part of GUS

<400> 5

```
tgtaaccacg cgtctgttga ctggcaggtg gtggccaatg gtgatgtcag cgttgaactg      60
cgtgatgcgg atcaacaggt ggttgcaact ggacaaggca ctagcgggac tttgcaagtg     120
gtgaatccgc acctctggca accgggtgaa ggttatctct atgaactgtg cgtcacagcc     180
aaaagccaga cagagtgtga tatctacccg cttcgcgtcg gcatccggtc agtggcagtg     240
aagggcgaac agttcctgat taaccacaaa ccgttctact ttactggctt tggtcgtcat     300
gaagatgcgg acttgcgtgg caaaggattc gataacgtgc tgatggtgca cgaccacgca     360
ttaatggact ggattggggc caactcctac cgtacctcgc attaccctta cgctgaagag     420
atgctcgact gggcagatga acatggcatc gtggtgattg atgaaactgc tgctgtcggc     480
tttaacctct ctttaggcat tggtttcgaa gcgggcaaca agccgaaaga actgtacagc     540
gaagaggcag tcaacgggga aactcagcaa gcgcacttac aggcgattaa agagctgata     600
gcgcgtgaca aaaaccaccc aagcgtggtg atgtggagta ttgccaacga accggatacc     660
cgtccgcaag gtgcacggga atatttcgcg ccactggcgg aagcaacgcg taaactcgac     720
ccgacgcgtc cgatcacctg cgtcaatgta atgttctgcg acgctcacac cgataccatc     780
agcgatctct ttgatgtgct gtgcctgaac cgttattacg gatggtatgt ccaaagcggc     840
gatttggaaa cggcagagaa ggtactggaa aaagaacttc tggcctggca ggagaaactg     900
catcagccga ttatcatcac cgaatacggc gtggatacgt tagccgggct gcactcaatg     960
tacaccgaca tgtggagtga agagtatcag tgtgcatggc tggatatgta tcaccgcgtc    1020
tttgatcgcg tcagcgccgt cgtcggtgaa caggtatgga atttcgccga ttttgcgacc    1080
tcgcaaggca tattgcgcgt tggcggtaac aagaaaggga tcttcactcg cgaccgcaaa    1140
ccgaagtcgg cggcttttct gctgcaaaaa cgctggactg gcatgaactt cggtgaaaaa    1200
ccgcagcagg gaggcaaaca a                                              1221
```

<210> 6
<211> 407
<212> PRT
<213> artificial

<220>

<223> amino acid sequence of the C-terminal part of GUS

<400> 6

```
Cys Asn His Ala Ser Val Asp Trp Gln Val Val Ala Asn Gly Asp Val
1               5                   10                  15

Ser Val Glu Leu Arg Asp Ala Asp Gln Gln Val Val Ala Thr Gly Gln
            20                  25                  30

Gly Thr Ser Gly Thr Leu Gln Val Val Asn Pro His Leu Trp Gln Pro
        35                  40                  45

Gly Glu Gly Tyr Leu Tyr Glu Leu Cys Val Thr Ala Lys Ser Gln Thr
    50                  55                  60
```

```
Glu Cys Asp Ile Tyr Pro Leu Arg Val Gly Ile Arg Ser Val Ala Val
65                  70                  75                  80

Lys Gly Glu Gln Phe Leu Ile Asn His Lys Pro Phe Tyr Phe Thr Gly
                85                  90                  95

Phe Gly Arg His Glu Asp Ala Asp Leu Arg Gly Lys Gly Phe Asp Asn
            100                 105                 110

Val Leu Met Val His Asp His Ala Leu Met Asp Trp Ile Gly Ala Asn
        115                 120                 125

Ser Tyr Arg Thr Ser His Tyr Pro Tyr Ala Glu Glu Met Leu Asp Trp
    130                 135                 140

Ala Asp Glu His Gly Ile Val Val Ile Asp Glu Thr Ala Ala Val Gly
145                 150                 155                 160

Phe Asn Leu Ser Leu Gly Ile Gly Phe Glu Ala Gly Asn Lys Pro Lys
                165                 170                 175

Glu Leu Tyr Ser Glu Glu Ala Val Asn Gly Glu Thr Gln Gln Ala His
            180                 185                 190

Leu Gln Ala Ile Lys Glu Leu Ile Ala Arg Asp Lys Asn His Pro Ser
        195                 200                 205

Val Val Met Trp Ser Ile Ala Asn Glu Pro Asp Thr Arg Pro Gln Gly
    210                 215                 220

Ala Arg Glu Tyr Phe Ala Pro Leu Ala Glu Ala Thr Arg Lys Leu Asp
225                 230                 235                 240

Pro Thr Arg Pro Ile Thr Cys Val Asn Val Met Phe Cys Asp Ala His
                245                 250                 255

Thr Asp Thr Ile Ser Asp Leu Phe Asp Val Leu Cys Leu Asn Arg Tyr
            260                 265                 270

Tyr Gly Trp Tyr Val Gln Ser Gly Asp Leu Glu Thr Ala Glu Lys Val
        275                 280                 285

Leu Glu Lys Glu Leu Leu Ala Trp Gln Glu Lys Leu His Gln Pro Ile
    290                 295                 300

Ile Ile Thr Glu Tyr Gly Val Asp Thr Leu Ala Gly Leu His Ser Met
305                 310                 315                 320

Tyr Thr Asp Met Trp Ser Glu Glu Tyr Gln Cys Ala Trp Leu Asp Met
                325                 330                 335

Tyr His Arg Val Phe Asp Arg Val Ser Ala Val Val Gly Glu Gln Val
```

```
                340                 345                 350

Trp Asn Phe Ala Asp Phe Ala Thr Ser Gln Gly Ile Leu Arg Val Gly
        355                 360                 365

Gly Asn Lys Lys Gly Ile Phe Thr Arg Asp Arg Lys Pro Lys Ser Ala
    370                 375                 380

Ala Phe Leu Leu Gln Lys Arg Trp Thr Gly Met Asn Phe Gly Glu Lys
385                 390                 395                 400

Pro Gln Gln Gly Gly Lys Gln
                405
```

<210> 7
<211> 318
<212> DNA
<213> artificial

<220>
<223> nucleic acid sequence coding for the N-terminal part of the DnaB intein

<400> 7

```
tgcatctccg gcgactccct catctccctc gcctccaccg gcaagcgcgt gtccatcaag      60
gacctcctcg acgagaagga cttcgagatt tgggccatca acgagcagac catgaagctg     120
gagtccgcca aggtgtcccg cgtgttctgc accggcaaga agctcgtcta tatcctcaag     180
acccgcctcg gcaggaccat caaggccacc gccaaccacc gcttcctcac catcgacggc     240
tggaagcgcc tcgacgagct gtccctcaag gagcacatcg ccctcccgcg caagctcgaa     300
tcctcctccc tccagctc                                                   318
```

<210> 8
<211> 106
<212> PRT
<213> artificial

<220>
<223> amino acid sequence of the N-terminal part of the DnaB intein

<400> 8

Cys Ile Ser Gly Asp Ser Leu Ile Ser Leu Ala Ser Thr Gly Lys Arg
1 5 10 15

Val Ser Ile Lys Asp Leu Leu Asp Glu Lys Asp Phe Glu Ile Trp Ala
20 25 30

Ile Asn Glu Gln Thr Met Lys Leu Glu Ser Ala Lys Val Ser Arg Val
35 40 45

Phe Cys Thr Gly Lys Lys Leu Val Tyr Ile Leu Lys Thr Arg Leu Gly
50 55 60

Arg Thr Ile Lys Ala Thr Ala Asn His Arg Phe Leu Thr Ile Asp Gly

65 70 75 80

Trp Lys Arg Leu Asp Glu Leu Ser Leu Lys Glu His Ile Ala Leu Pro
85 90 95

Arg Lys Leu Glu Ser Ser Ser Leu Gln Leu
100 105

<210> 9
<211> 147
<212> DNA
<213> artificial

<220>
<223> nucleic acid sequence coding for the C-terminal part of the DnaB intein

<400> 9

atgagcccgg agatcgagaa gctctcccag tccgacatct actgggactc catcgtgtcc 60
atcaccgaaa cgggcgtgga ggaggtgttc gacctcaccg tgccaggccc gcacaacttc 120
gtggccaacg acatcatcgt gcacaac 147

<210> 10
<211> 49
<212> PRT
<213> artificial

<220>

<223> amino acid sequence of the C-terminal part of the DnaB intein

<400> 10

```
Met Ser Pro Glu Ile Glu Lys Leu Ser Gln Ser Asp Ile Tyr Trp Asp
1               5                   10                  15

Ser Ile Val Ser Ile Thr Glu Thr Gly Val Glu Glu Val Phe Asp Leu
            20                  25                  30

Thr Val Pro Gly Pro His Asn Phe Val Ala Asn Asp Ile Ile Val His
        35                  40                  45

Asn
```

<210> 11
<211> 333
<212> DNA
<213> Bacillus amyloliquefaciens

<400> 11

```
atggcccaag tgattaacac cttcgacggc gtggccgact acctccagac ctaccacaag       60
ctcccggaca actacatcac caagtccgag gcccaggccc tcggctgggt ggcctccaag      120
ggcaacctcg ccgacgtggc cccagggaag tccatcggcg gcgacatctt ctcaaaccgc      180
gagggcaagc tcccaggcaa gtcgggcagg acctggaggg aggccgacat caactacacc      240
tccggcttcc gcaactccga ccgcatcctc tactcctccg actggctcat ctacaagacc      300

accgaccact accagacctt caccaagatc cgc                                   333
```

<210> 12
<211> 111
<212> PRT
<213> Bacillus amyloliquefaciens

<400> 12

```
Met Ala Gln Val Ile Asn Thr Phe Asp Gly Val Ala Asp Tyr Leu Gln
1               5                   10                  15

Thr Tyr His Lys Leu Pro Asp Asn Tyr Ile Thr Lys Ser Glu Ala Gln
            20                  25                  30

Ala Leu Gly Trp Val Ala Ser Lys Gly Asn Leu Ala Asp Val Ala Pro
        35                  40                  45

Gly Lys Ser Ile Gly Gly Asp Ile Phe Ser Asn Arg Glu Gly Lys Leu
    50                  55                  60

Pro Gly Lys Ser Gly Arg Thr Trp Arg Glu Ala Asp Ile Asn Tyr Thr
65                  70                  75                  80

Ser Gly Phe Arg Asn Ser Asp Arg Ile Leu Tyr Ser Ser Asp Trp Leu
                85                  90                  95

Ile Tyr Lys Thr Thr Asp His Tyr Gln Thr Phe Thr Lys Ile Arg
            100                 105                 110
```

<210> 13
<211> 108
<212> DNA
<213> artificial

<220>
<223> nucleic acid sequence coding for the N-terminal part of barnase

<400> 13

```
atggcccaag tgattaacac cttcgacggc gtggccgact acctccagac ctaccacaag       60
ctcccggaca actacatcac caagtccgag gcccaggccc tcggctgg                   108
```

<210> 14
<211> 36
<212> PRT
<213> artificial

<220>
<223> amino acid sequence of the N-terminal part of barnase

<400> 14

```
Met Ala Gln Val Ile Asn Thr Phe Asp Gly Val Ala Asp Tyr Leu Gln
1               5                   10                  15

Thr Tyr His Lys Leu Pro Asp Asn Tyr Ile Thr Lys Ser Glu Ala Gln
            20                  25                  30

Ala Leu Gly Trp
        35
```

<210> 15
<211> 225
<212> DNA
<213> artificial

<220>
<223> nucleic acid sequence coding for the C-terminal part of barnase

<400> 15

```
gtggcctcca agggcaacct cgccgacgtg gccccaggga agtccatcgg cggcgacatc       60
ttctcaaacc gcgagggcaa gctcccaggc aagtcgggca ggacctggag ggaggccgac      120
atcaactaca cctccggctt ccgcaactcc gaccgcatcc tctactcctc cgactggctc      180
atctacaaga ccaccgacca ctaccagacc ttcaccaaga tccgc                      225
```

<210> 16
<211> 75
<212> PRT
<213> artificial

<220>
<223> amino acid sequence of the C-terminal part of barnase

<400> 16

```
Val Ala Ser Lys Gly Asn Leu Ala Asp Val Ala Pro Gly Lys Ser Ile
1               5                   10                  15

Gly Gly Asp Ile Phe Ser Asn Arg Glu Gly Lys Leu Pro Gly Lys Ser
            20                  25                  30

Gly Arg Thr Trp Arg Glu Ala Asp Ile Asn Tyr Thr Ser Gly Phe Arg
        35                  40                  45

Asn Ser Asp Arg Ile Leu Tyr Ser Ser Asp Trp Leu Ile Tyr Lys Thr
    50                  55                  60

Thr Asp His Tyr Gln Thr Phe Thr Lys Ile Arg
65                  70                  75
```

<210> 17
<211> 369
<212> DNA
<213> artificial

<220>
<223> nucleic acid sequence coding for the N-terminal part of the DnaE intein

<400> 17

tgcctcagtt ttggcaccga aattttaacc gttgagtacg gcccattgcc cattggcaaa 60

attgtgagtg aagaaattaa ttgttctgtg tacagtgttg atccagaagg gagagtttac 120

acccaggcga tcgcccaatg gcatgaccgg ggagagcagg aagtattgga atatgaattg 180

gaagatggtt cagtaatccg agctacctct gaccaccgct ttttaaccac cgattatcaa 240

ctgttggcga tcgaagaaat ttttgctagg caactggact tgttgacttt agaaaatatt 300

aagcaaactg aagaagctct tgacaaccat cgtcttccct ttccattact tgacgctggg 360

acaattaaa 369

<210> 18
<211> 123
<212> PRT
<213> artificial

<220>
<223> amino acid sequence of the N-terminal part of the DnaE intein

<400> 18

```
Cys Leu Ser Phe Gly Thr Glu Ile Leu Thr Val Glu Tyr Gly Pro Leu
1               5                   10                  15

Pro Ile Gly Lys Ile Val Ser Glu Glu Ile Asn Cys Ser Val Tyr Ser
            20                  25                  30

Val Asp Pro Glu Gly Arg Val Tyr Thr Gln Ala Ile Ala Gln Trp His
        35                  40                  45

Asp Arg Gly Glu Gln Glu Val Leu Glu Tyr Glu Leu Glu Asp Gly Ser
    50                  55                  60

Val Ile Arg Ala Thr Ser Asp His Arg Phe Leu Thr Thr Asp Tyr Gln
65                  70                  75                  80

Leu Leu Ala Ile Glu Glu Ile Phe Ala Arg Gln Leu Asp Leu Leu Thr
                85                  90                  95

Leu Glu Asn Ile Lys Gln Thr Glu Glu Ala Leu Asp Asn His Arg Leu
            100                 105                 110

Pro Phe Pro Leu Leu Asp Ala Gly Thr Ile Lys
        115                 120
```

<210> 19
<211> 108
<212> DNA
<213> artificial

<220>
<223> nucleic acid sequence coding for the C-terminal part of the DnaE intein

<400> 19

atggttaaag ttatcggtcg tcgttccctc ggagtgcaaa gaatatttga tattggtctt 60

ccccaagacc ataattttct gctagccaat ggggcgatcg ccgccaat 108

<210> 20
<211> 36
<212> PRT
<213> artificial

<220>
<223> amino acid sequence of the C-terminal part of the DnaE intein

<400> 20

Met Val Lys Val Ile Gly Arg Arg Ser Leu Gly Val Gln Arg Ile Phe
1 5 10 15

Asp Ile Gly Leu Pro Gln Asp His Asn Phe Leu Leu Ala Asn Gly Ala
20 25 30

Ile Ala Ala Asn
35

<210> 21
<211> 45
<212> DNA
<213> artificial

<220>
<223> nucleic acid sequence coding for the linker sequence

<400> 21
ggaggcggtg gaagtggagg cggtggatca ggaggcggtg gctca 45

<210> 22
<211> 15
<212> PRT
<213> artificial

<220>
<223> amino acid sequence of the linker sequence

<400> 22

Gly Gly Gly Gly Ser Gly Gly Gly Gly Ser Gly Gly Gly Gly Ser
1 5 10 15

<210> 23
<211> 15
<212> DNA
<213> artificial

<220>
<223> nucleic acid sequence coding for the DnaB extein sequence SIEQD

<400> 23
tccatcgagc aggac 15

<210> 24
<211> 5
<212> PRT
<213> artificial

<220>
<223> amino acid sequence of the DnaB extein sequence SIEQD

<400> 24

Ser Ile Glu Gln Asp
1 5

<210> 25
<211> 12
<212> DNA
<213> artificial

<220>
<223> nucleic acid sequence coding for the DnaB extein sequence RESG

<400> 25
agggagtccg gc 12

<210> 26
<211> 4
<212> PRT
<213> artificial

<220>
<223> amino acid sequence of the DnaB extein sequence RESG

<400> 26

Arg Glu Ser Gly
1

<210> 27
<211> 15
<212> DNA
<213> artificial

<220>
<223> nucleic acid sequence coding for the DnaE extein sequence CFNHG

<400> 27
tgttttaacc atggg 15

<210> 28
<211> 5
<212> PRT
<213> artificial

<220>
<223> amino acid sequence of the DnaE extein sequence CFNHG

<400> 28

Cys Phe Asn His Gly
1               5

<210> 29
<211> 21
<212> DNA
<213> artificial

<220>
<223> nucleic acid sequence coding for the DnaE extein sequence DVKFAEY

<400> 29
gacgtcaagt ttgcggaata t 21

<210> 30
<211> 7
<212> PRT
<213> artificial

<220>
<223> amino acid sequence of the DnaE extein sequence DVKFAEY

<400> 30

Asp Val Lys Phe Ala Glu Tyr
1               5

<210> 31
<211> 9060
<212> DNA
<213> artificial

<220>
<223> complete sequence of vector pHW161

<400> 31

```
catgggtcag tcccttatgt tacgtcctgt agaaacccca acccgtgaaa tcaaaaaact      60
cgacggcctg tgggcattca gtctggatcg cgaaaactgt ggaattgatc agcgttggtg     120
ggaaagcgcg ttacaagaaa gccgggcaat tgctgtgcca ggcagtttta acgatcagtt     180
cgccgatgca gatattcgta attatgcggg caacgtctgg tatcagcgcg aagtctttat     240
accgaaaggt tgggcaggcc agcgtatcgt gctgcgtttc gatgcggtca ctcattacgg     300
caaagtgtgg gtcaataatc aggaagtgat ggagcatcag ggcggctata cgccatttga     360
agccgatgtc acgccgtatg ttattgccgg gaaaagtgta cgtatcaccg tttgtgtgaa     420
caacgaactg aactggcaga ctatcccgcc gggaatggtg attaccgacg aaaacggcaa     480
gaaaaagcag tcttacttcc atgatttctt taactatgcc ggaatccatc gcagcgtaat     540
gctctacacc acgccgaaca cctgggtgga cgatatcacc gtggtgacgc atgtcgcgca     600
agacagggag tccggctgca tctccggcga ctccctcatc tccctcgcct ccaccggcaa     660
gcgcgtgtcc atcaaggacc tcctcgacga gaaggacttc gagatttggg ccatcaacga     720
gcagaccatg aagctggagt ccgccaaggt gtcccgcgtg ttctgcaccg gcaagaagct     780
cgtctatatc ctcaagaccc gcctcggcag gaccatcaag gccaccgcca accaccgctt     840
cctcaccatc gacggctgga agcgcctcga cgagctgtcc ctcaaggagc acatcgccct     900
cccgcgcaag ctcgaatcct cctccctcca gctctgagga tcctctagag tcctgcttta     960
atgagatatg cgagacgcct atgatcgcat gatatttgct ttcaattctg ttgtgcacgt    1020
tgtaaaaaac ctgagcatgt gtagctcaga tccttaccgc cggtttcggt tcattctaat    1080
gaatatatca cccgttacta tcgtattttt atgaataata ttctccgttc aatttactga    1140
ttgtacccta ctacttatat gtacaatatt aaaatgaaaa caatatattg tgctgaatag    1200
gtttatagcg acatctatga tagagcgcca caataacaaa caattgcgtt ttattattac    1260
aaatccaatt ttaaaaaaag cggcagaacc ggtcaaacct aaaagactga ttacataaat    1320
cttattcaaa tttcaaaagt gccccagggg ctagtatcta cgacacaccg agcggcgaac    1380
taataacgct cactgaaggg aactccggtt ccccgccggc gcgcatgggt gagattcctt    1440
```

gaagttgagt attggccgtc cgctctaccg aaagttacgg gcaccattca acccggtcca 1500
gcacggcggc cgggtaaccg acttgctgcc ccgagaatta tgcagcattt ttttggtgta 1560
tgtgggcccc aaatgaagtg caggtcaaac cttgacagtg acgacaaatc gttgggcggg 1620
tccagggcga attttgcgac aacatgtcga ggctcagcag gacctgcagg catgcaagct 1680
tagatcagat tgtcgtttcc cgccttcagt ttaaactatc agtgtttgac aggatatatt 1740
ggcgggtaaa cctaagagaa aagagcgttt attagaataa tcggatattt aaaagggcgt 1800
gaaaaggttt atccgttcgt ccatttgtat gtgcatgcca accacagggt tccccagatc 1860
aggatctcct ttgcgacgct caccgggctg gttgccctcg ccgctgggct ggcggccgtc 1920
tatggccctg caaacgcgcc agaaacgccg tcgaagccgt gtgcgagaca ccgcggccgc 1980
cggcgttgtg gatacctcgc ggaaaacttg gccctcactg acagatgagg ggcggacgtt 2040
gacacttgag gggccgactc acccggcgcg gcgttgacag atgaggggca ggctcgattt 2100
cggccggcga cgtggagctg gccagcctcg caaatcggcg aaaacgcctg attttacgcg 2160
agtttcccac agatgatgtg gacaagcctg gggataagtg ccctgcggta ttgacacttg 2220
aggggcgcga ctactgacag atgaggggcg cgatccttga cacttgaggg gcagagtgct 2280
gacagatgag gggcgcacct attgacattt gaggggctgt ccacaggcag aaaatccagc 2340
atttgcaagg gtttccgccc gtttttcggc caccgctaac ctgtctttta acctgctttt 2400
aaaccaatat ttataaacct tgtttttaac cagggctgcg ccctgtgcgc gtgaccgcgc 2460
acgccgaagg ggggtgcccc cccttctcga accctcccgg cccgctaacg cgggcctccc 2520
atccccccag gggctgcgcc cctcggccgc gaacggcctc accccaaaaa tggcagcgct 2580
ggccaattcg tgcgcggaac ccctatttgt ttatttttct aaatacattc aaatatgtat 2640
ccgctcatga gacaataacc ctgataaatg cttcaataat attgaaaaag gaagagtatg 2700
agtattcaac atttccgtgt cgcccttatt cccttttttg cggcattttg ccttcctgtt 2760
tttgctcacc cagaaacgct ggtgaaagta aaagatgctg aagatcagtt gggtgcacga 2820
gtgggttaca tcgaactgga tctcaacagc ggtaagatcc ttgagagttt tcgccccgaa 2880
gaacgttttc caatgatgag cacttttaaa gttctgctat gtggcgcggt attatcccgt 2940
attgacgccg ggcaagagca actcggtcgc cgcatacact attctcagaa tgacttggtt 3000
gagtactcac cagtcacaga aaagcatctt acggatggca tgacagtaag agaattatgc 3060
agtgctgcca taaccatgag tgataacact gcggccaact tacttctgac aacgatcgga 3120
ggaccgaagg agctaaccgc ttttttgcac aacatggggg atcatgtaac tcgccttgat 3180
cgttgggaac cggagctgaa tgaagccata ccaaacgacg agcgtgacac cacgatgcct 3240
gtagcaatgg caacaacgtt gcgcaaacta ttaactggcg aactacttac tctagcttcc 3300
cggcaacaat taatagactg gatggaggcg gataaagttg caggaccact tctgcgctcg 3360
gcccttccgg ctggctggtt tattgctgat aaatctggag ccggtgagcg tgggtctcgc 3420
ggtatcattg cagcactggg gccagatggt aagccctccc gtatcgtagt tatctacacg 3480
acggggagtc aggcaactat ggatgaacga aatagacaga tcgctgagat aggtgcctca 3540

ctgattaagc attggtaact gtcagaccaa gtttactcat atatacttta gattgattta 3600
aaacttcatt tttaatttaa aaggatctag gtgaagatcc tttttgataa tctcatgacc 3660
aaaatccctt aacgtgagtt ttcgttccac tgagcgtcag accccgtaga aaagatcaaa 3720
ggatcttctt gagatccttt ttttctgcgc gtaatctgct gcttgcaaac aaaaaaacca 3780
ccgctaccag cggtggtttg tttgccggat caagagctac caactctttt tccgaaggta 3840
actggcttca gcagagcgca gataccaaat actgtccttc tagtgtagcc gtagttaggc 3900
caccacttca agaactctgt agcaccgcct acatacctcg ctctgctaat cctgttacca 3960
gtggctgctg ccagtggcga taagtcgtgt cttaccgggt tggactcaag acgatagtta 4020
ccggataagg cgcagcggtc gggctgaacg gggggttcgt gcacacagcc cagcttggag 4080
cgaacgacct acaccgaact gagataccta cagcgtgagc tatgagaaag cgccacgctt 4140
cccgaaggga gaaaggcgga caggtatccg gtaagcggca gggtcggaac aggagagcgc 4200
acgagggagc ttccaggggg aaacgcctgg tatctttata gtcctgtcgg gtttcgccac 4260
ctctgacttg agcgtcgatt tttgtgatgc tcgtcagggg ggcggagcct atggaaaaac 4320
gccagcaacg cggccttttt acggttcctg gcagatccta gatgtggcgc aacgatgccg 4380
gcgacaagca ggagcgcacc gacttcttcc gcatcaagtg ttttggctct caggccgagg 4440
cccacggcaa gtatttgggc aaggggtcgc tggtattcgt gcagggcaag attcggaata 4500
ccaagtacga gaaggacggc cagacggtct acgggaccga cttcattgcc gataaggtgg 4560
attatctgga caccaaggca ccaggcgggt caaatcagga ataagggcac attgccccgg 4620
cgtgagtcgg ggcaatcccg caaggagggt gaatgaatcg gacgtttgac cggaaggcat 4680
acaggcaaga actgatcgac gcggggtttt ccgccgagga tgccgaaacc atcgcaagcc 4740
gcaccgtcat gcgtgcgccc cgcgaaacct tccagtccgt cggctcgatg gtccagcaag 4800
ctacggccaa gatcgagcgc gacagcgtgc aactggctcc ccctgccctg cccgcgccat 4860
cggccgccgt ggagcgttcg cgtcgtctcg aacaggaggc ggcaggtttg gcgaagtcga 4920
tgaccatcga cacgcgagga actatgacga ccaagaagcg aaaaaccgcc ggcgaggacc 4980
tggcaaaaca ggtcagcgag gccaagcagg ccgcgttgct gaaacacacg aagcagcaga 5040
tcaaggaaat gcagctttcc ttgttcgata ttgcgccgtg gccggacacg atgcgagcga 5100
tgccaaacga cacggcccgc tctgccctgt tcaccacgcg caacaagaaa atcccgcgcg 5160
aggcgctgca aaacaaggtc attttccacg tcaacaagga cgtgaagatc acctacaccg 5220
gcgtcgagct gcgggccgac gatgacgaac tggtgtggca gcaggtgttg gagtacgcga 5280
agcgcacccc tatcggcgag ccgatcacct tcacgttcta cgagctttgc caggacctgg 5340
gctggtcgat caatggccgg tattacacga aggccgagga atgcctgtcg cgcctacagg 5400
cgacggcgat gggcttcacg tccgaccgcg ttgggcacct ggaatcggtg tcgctgctgc 5460
accgcttccg cgtcctggac cgtggcaaga aaacgtcccg ttgccaggtc ctgatcgacg 5520
aggaaatcgt cgtgctgttt gctggcgacc actacacgaa attcatatgg gagaagtacc 5580

gcaagctgtc gccgacggcc cgacggatgt tcgactattt cagctcgcac cgggagccgt 5640
acccgctcaa gctggaaacc ttccgcctca tgtgcggatc ggattccacc cgcgtgaaga 5700
agtggcgcga gcaggtcggc gaagcctgcg aagagttgcg aggcagcggc ctggtggaac 5760
acgcctgggt caatgatgac ctggtgcatt gcaaacgcta gggccttgtg gggtcagttc 5820
cggctggggg ttcagcagcc agcgcctgat ctgactgatg ggctgcctgt atcgagtggt 5880
gattttgtgc cgagctgccg gtcggggagc tgttggctgg ctggtggcag gatatattgt 5940
ggtgtaaaca aattgacgct tagacaactt aataacacat tgcggacgtt tttaatgtac 6000
tggggtggat gcaggtcctg ctgagcctcg acatgttgtc gcaaaattcg ccctggaccc 6060
gcccaacgat ttgtcgtcac tgtcaaggtt tgacctgcac ttcatttggg gcccacatac 6120
accaaaaaaa tgctgcataa ttctcggggc agcaagtcgg ttacccggcc gccgtgctgg 6180
accgggttga atggtgcccg taactttcgg tagagcggac ggccaatact caacttcaag 6240
gaatctcacc catgcgcgcc ggcggggaac cggagttccc ttcagtgagc gttattagtt 6300
cgccgctcgg tgtgtcgtag atactagccc ctggggcact tttgaaattt gaataagatt 6360
tatgtaatca gtcttttagg tttgaccggt tctgccgctt tttttaaaat tggatttgta 6420
ataataaaac gcaattgttt gttattgtgg cgctctatca tagatgtcgc tataaaccta 6480
ttcagcacaa tatattgttt tcattttaat attgtacata taagtagtag ggtacaatca 6540
gtaaattgaa cggagaatat tattcataaa aatacgatag taacgggtga tatattcatt 6600
agaatgaacc gaaaccggcg gtaaggatct gagctacaca tgctcaggtt ttttacaacg 6660
tgcacaacag aattgaaagc aaatatcatg cgatcatagg cgtctcgcat atctcattaa 6720
agcaggactc taggtcatca aatctcggtg acgggcagga ccggacgggg cggtaccggc 6780
aggctgaagt ccagctgcca gaaacccacg tcatgccagt tcccgtgctt gaagccggcc 6840
gcccgcagca tgccgcgggg ggcatatccg agcgcctcgt gcatgcgcac gctcgggtcg 6900
ttgggcagcc cgatgacagc gaccacgctc ttgaagccct gtgcctccag ggacttcagc 6960
aggtgggtgt agagcgtgga gcccagtccc gtccgctggt ggcgggggga gacgtacacg 7020
gttgactcgg ccgtccagtc gtaggcgttg cgtgccttcc aggggcccgc gtaggcgatg 7080
ccggcgacct cgccgtccac ctcggcgacg agccagggat agcgctcccg cagacggacg 7140
aggtcgtccg tccactcctg cggttcctgc ggctcggtac ggaagttgac cgtgcttgtc 7200
tcgatgtagt ggttgacgat ggtgcagacc gccggcatgt ccgcctcggt ggcacggcgg 7260
atgtcggccg ggcgtcgttc tgggctcatg gtaattgtaa atagtaattg taatgttgtt 7320
tgttgtttgt tgttgttggt aattgttgta aaaatagtcg agttgagagt gaatatgaga 7380
ctctaattgg ataccgaggg gaatttatgg aacgtcagtg gagcattttt gacaagaaat 7440
atttgctagc tgatagtgac cttaggcgac ttttgaacgc gcaataatgg tttctgacgt 7500
atgtgcttag ctcattaaac tccagaaacc cgcggctgag tggctccttc aacgttgcgg 7560
ttctgtcagt tccaaacgta aaacggcttg tcccgcgtca tcggcggggg tcataacgtg 7620
actcccttaa ttctccgctc agaattccaa tcccacaaaa atctgagctt aacagcacag 7680

```
ttgctcctct cagagcagaa tcgggtattc aacaccctca tatcaactac tacgttgtgt      7740
ataacggtcc acatgccggt atatacgatg actggggttg tacaaaggcg gcaacaaacg      7800
gcgttcccgg agttgcacac aagaaatttg ccactattac agaggcaaga gcagcagctg      7860
acgcgtacac aacaagtcag caaacagaca ggttgaactt catccccaaa ggagaagctc      7920
aactcaagcc caagagcttt gctaaggccc taacaagccc accaaagcaa aaagcccact      7980
ggctcacgct aggaaccaaa aggcccagca gtgatccagc cccaaaagag atctcctttg      8040
ccccggagat tacaatggac gatttcctct atctttacga tctaggaagg aagttcgaag      8100
gtgaaggtga cgacactatg ttcaccactg ataatgagaa ggttagcctc ttcaatttca      8160
gaaagaatgc tgacccacag atggttagag aggcctacgc agcaggtctc atcaagacga      8220
tctacccgag taacaatctc caggagatca aataccttcc caagaaggtt aaagatgcag      8280
tcaaaagatt caggactaat tgcatcaaga acacagagaa agacatattt ctcaagatca      8340
gaagtactat tccagtatgg acgattcaag gcttgcttca taaaccaagg caagtaatag      8400
agattggagt ctctaaaaag gtagttccta ctgaatctaa ggccatgcat ggagtctaag      8460
attcaaatcg aggatctaac agaactcgcc gtgaagactg gcgaacagtt catacagagt      8520
cttttacgac tcaatgacaa gaagaaaatc ttcgtcaaca tggtggagca cgacactctg      8580
gtctactcca aaaatgtcaa agatacagtc tcagaagacc aaagggctat tgagactttt      8640
caacaaagga taatttcggg aaacctcctc ggattccatt gcccagctat ctgtcacttc      8700
atcgaaagga cagtagaaaa ggaaggtggc tcctacaaat gccatcattg cgataaagga      8760
aaggctatca ttcaagatct ctctgccgac agtggtccca aagatggacc cccacccacg      8820
aggagcatcg tggaaaaaga agacgttcca accacgtctt caaagcaagt ggattgatgt      8880
gacatctcca ctgacgtaag ggatgacgca caatcccact atccttcgca agacccttcc      8940
tctatataag gaagttcatt tcatttggag aggacacgct cgagtataag agctctattt      9000
ttacaacaat taccaacaac aacaaacaac aaacaacatt acaattacat ttacaattac      9060
```

<210> 32
<211> 9105
<212> DNA
<213> artificial

<220>
<223> complete sequence of vector pHW181

<400> 32

```
catgggtcag tcccttatgt tacgtcctgt agaaacccca acccgtgaaa tcaaaaaact      60
cgacggcctg tgggcattca gtctggatcg cgaaaactgt ggaattgatc agcgttggtg     120
ggaaagcgcg ttacaagaaa gccgggcaat tgctgtgcca ggcagtttta acgatcagtt     180
cgccgatgca gatattcgta attatgcggg caacgtctgg tatcagcgcg aagtctttat     240
accgaaaggt tgggcaggcc agcgtatcgt gctgcgtttc gatgcggtca ctcattacgg     300
caaagtgtgg gtcaataatc aggaagtgat ggagcatcag ggcggctata cgccatttga     360
```

agccgatgtc acgccgtatg ttattgccgg gaaaagtgta cgtatcaccg tttgtgtgaa 420
caacgaactg aactggcaga ctatcccgcc gggaatggtg attaccgacg aaaacggcaa 480
gaaaaagcag tcttacttcc atgatttctt taactatgcc ggaatccatc gcagcgtaat 540
gctctacacc acgccgaaca cctgggtgga cgatatcacc gtggtgacgc atgtcgcgca 600
agacggaggc ggtggaagtg gaggcggtgg atcaggaggc ggtggctcaa gggagtccgg 660
ctgcatctcc ggcgactccc tcatctccct cgcctccacc ggcaagcgcg tgtccatcaa 720
ggacctcctc gacgagaagg acttcgagat ttgggccatc aacgagcaga ccatgaagct 780
ggagtccgcc aaggtgtccc gcgtgttctg caccggcaag aagctcgtct atatcctcaa 840
gacccgcctc ggcaggacca tcaaggccac cgccaaccac cgcttcctca ccatcgacgg 900
ctggaagcgc ctcgacgagc tgtccctcaa ggagcacatc gccctcccgc gcaagctcga 960
atcctcctcc ctccagctct gaggatcctc tagagtcctg ctttaatgag atatgcgaga 1020
cgcctatgat cgcatgatat ttgctttcaa ttctgttgtg cacgttgtaa aaaacctgag 1080
catgtgtagc tcagatcctt accgccggtt tcggttcatt ctaatgaata tatcacccgt 1140
tactatcgta tttttatgaa taatattctc cgttcaattt actgattgta ccctactact 1200
tatatgtaca atattaaaat gaaaacaata tattgtgctg aataggttta tagcgacatc 1260
tatgatagag cgccacaata acaaacaatt gcgttttatt attacaaatc caattttaaa 1320
aaaagcggca gaaccggtca aacctaaaag actgattaca taaatcttat tcaaatttca 1380
aaagtgcccc aggggctagt atctacgaca caccgagcgg cgaactaata acgctcactg 1440
aagggaactc cggttccccg ccggcgcgca tgggtgagat tccttgaagt tgagtattgg 1500
ccgtccgctc taccgaaagt tacgggcacc attcaacccg gtccagcacg gcggccgggt 1560
aaccgacttg ctgccccgag aattatgcag catttttttg gtgtatgtgg gccccaaatg 1620
aagtgcaggt caaaccttga cagtgacgac aaatcgttgg gcgggtccag ggcgaatttt 1680
gcgacaacat gtcgaggctc agcaggacct gcaggcatgc aagcttagat cagattgtcg 1740
tttcccgcct tcagtttaaa ctatcagtgt ttgacaggat atattggcgg gtaaacctaa 1800
gagaaaagag cgtttattag aataatcgga tatttaaaag ggcgtgaaaa ggtttatccg 1860
ttcgtccatt tgtatgtgca tgccaaccac agggttcccc agatcaggat ctcctttgcg 1920
acgctcaccg ggctggttgc cctcgccgct gggctggcgg ccgtctatgg ccctgcaaac 1980
gcgccagaaa cgccgtcgaa gccgtgtgcg agacaccgcg gccgccggcg ttgtggatac 2040
ctcgcggaaa acttggccct cactgacaga tgaggggcgg acgttgacac ttgaggggcc 2100
gactcacccg gcgcggcgtt gacagatgag gggcaggctc gatttcggcc ggcgacgtgg 2160
agctggccag cctcgcaaat cggcgaaaac gcctgatttt acgcgagttt cccacagatg 2220
atgtggacaa gcctggggat aagtgccctg cggtattgac acttgagggg cgcgactact 2280
gacagatgag ggcgcgatcc ttgacacttg aggggcaga gtgctgacag atgaggggcg 2340
cacctattga catttgaggg gctgtccaca ggcagaaaat ccagcatttg caagggtttc 2400
cgcccgtttt tcggccaccg ctaacctgtc ttttaacctg cttttaaacc aatatttata 2460

aaccttgttt ttaaccaggg ctgcgccctg tgcgcgtgac cgcgcacgcc gaaggggggt 2520
gccccccctt ctcgaaccct cccggcccgc taacgcgggc ctcccatccc cccaggggct 2580
gcgcccctcg gccgcgaacg gcctcacccc aaaaatggca gcgctggcca attcgtgcgc 2640
ggaaccccta tttgtttatt tttctaaata cattcaaata tgtatccgct catgagacaa 2700
taaccctgat aaatgcttca ataatattga aaaaggaaga gtatgagtat tcaacatttc 2760
cgtgtcgccc ttattccctt ttttgcggca ttttgccttc ctgtttttgc tcacccagaa 2820
acgctggtga aagtaaaaga tgctgaagat cagttgggtg cacgagtggg ttacatcgaa 2880
ctggatctca acagcggtaa gatccttgag agttttcgcc ccgaagaacg ttttccaatg 2940
atgagcactt ttaaagttct gctatgtggc gcggtattat cccgtattga cgccgggcaa 3000
gagcaactcg gtcgccgcat acactattct cagaatgact tggttgagta ctcaccagtc 3060
acagaaaagc atcttacgga tggcatgaca gtaagagaat tatgcagtgc tgccataacc 3120
atgagtgata acactgcggc caacttactt ctgacaacga tcggaggacc gaaggagcta 3180
accgcttttt tgcacaacat gggggatcat gtaactcgcc ttgatcgttg ggaaccggag 3240
ctgaatgaag ccataccaaa cgacgagcgt gacaccacga tgcctgtagc aatggcaaca 3300
acgttgcgca aactattaac tggcgaacta cttactctag cttcccggca acaattaata 3360
gactggatgg aggcggataa agttgcagga ccacttctgc gctcggccct tccggctggc 3420
tggtttattg ctgataaatc tggagccggt gagcgtgggt ctcgcggtat cattgcagca 3480
ctggggccag atggtaagcc ctcccgtatc gtagttatct acacgacggg gagtcaggca 3540
actatggatg aacgaaatag acagatcgct gagataggtg cctcactgat taagcattgg 3600
taactgtcag accaagttta ctcatatata ctttagattg atttaaaact tcatttttaa 3660
tttaaaagga tctaggtgaa gatccttttt gataatctca tgaccaaaat cccttaacgt 3720
gagttttcgt tccactgagc gtcagacccc gtagaaaaga tcaaaggatc ttcttgagat 3780
cctttttttc tgcgcgtaat ctgctgcttg caaacaaaaa aaccaccgct accagcggtg 3840
gtttgtttgc cggatcaaga gctaccaact ctttttccga aggtaactgg cttcagcaga 3900
gcgcagatac caaatactgt ccttctagtg tagccgtagt taggccacca cttcaagaac 3960
tctgtagcac cgcctacata cctcgctctg ctaatcctgt taccagtggc tgctgccagt 4020
ggcgataagt cgtgtcttac cgggttggac tcaagacgat agttaccgga taaggcgcag 4080
cggtcgggct gaacgggggg ttcgtgcaca cagcccagct tggagcgaac gacctacacc 4140
gaactgagat acctacagcg tgagctatga gaaagcgcca cgcttcccga agggagaaag 4200
gcggacaggt atccggtaag cggcagggtc ggaacaggag agcgcacgag ggagcttcca 4260
gggggaaacg cctggtatct ttatagtcct gtcgggtttc gccacctctg acttgagcgt 4320
cgatttttgt gatgctcgtc aggggggcgg agcctatgga aaaacgccag caacgcggcc 4380
tttttacggt tcctggcaga tcctagatgt ggcgcaacga tgccggcgac aagcaggagc 4440
gcaccgactt cttccgcatc aagtgttttg gctctcaggc cgaggcccac ggcaagtatt 4500

tgggcaaggg gtcgctggta ttcgtgcagg gcaagattcg gaataccaag tacgagaagg 4560
acggccagac ggtctacggg accgacttca ttgccgataa ggtggattat ctggacacca 4620
aggcaccagg cgggtcaaat caggaataag ggcacattgc cccggcgtga gtcggggcaa 4680
tcccgcaagg agggtgaatg aatcggacgt ttgaccggaa ggcatacagg caagaactga 4740
tcgacgcggg gttttccgcc gaggatgccg aaaccatcgc aagccgcacc gtcatgcgtg 4800
cgccccgcga aaccttccag tccgtcggct cgatggtcca gcaagctacg gccaagatcg 4860
agcgcgacag cgtgcaactg gctccccctg ccctgcccgc gccatcggcc gccgtggagc 4920
gttcgcgtcg tctcgaacag gaggcggcag gtttggcgaa gtcgatgacc atcgacacgc 4980
gaggaactat gacgaccaag aagcgaaaaa ccgccggcga ggacctggca aaacaggtca 5040
gcgaggccaa gcaggccgcg ttgctgaaac acacgaagca gcagatcaag gaaatgcagc 5100
tttccttgtt cgatattgcg ccgtggccgg acacgatgcg agcgatgcca aacgacacgg 5160
cccgctctgc cctgttcacc acgcgcaaca agaaaatccc gcgcgaggcg ctgcaaaaca 5220
aggtcatttt ccacgtcaac aaggacgtga agatcaccta caccggcgtc gagctgcggg 5280
ccgacgatga cgaactggtg tggcagcagg tgttggagta cgcgaagcgc acccctatcg 5340
gcgagccgat caccttcacg ttctacgagc tttgccagga cctgggctgg tcgatcaatg 5400
gccggtatta cacgaaggcc gaggaatgcc tgtcgcgcct acaggcgacg gcgatgggct 5460
tcacgtccga ccgcgttggg cacctggaat cggtgtcgct gctgcaccgc ttccgcgtcc 5520
tggaccgtgg caagaaaacg tcccgttgcc aggtcctgat cgacgaggaa atcgtcgtgc 5580
tgtttgctgg cgaccactac acgaaattca tatgggagaa gtaccgcaag ctgtcgccga 5640
cggcccgacg gatgttcgac tatttcagct cgcaccggga gccgtacccg ctcaagctgg 5700
aaaccttccg cctcatgtgc ggatcggatt ccacccgcgt gaagaagtgg cgcgagcagg 5760
tcggcgaagc ctgcgaagag ttgcgaggca gcggcctggt ggaacacgcc tgggtcaatg 5820
atgacctggt gcattgcaaa cgctagggcc ttgtggggtc agttccggct gggggttcag 5880
cagccagcgc ctgatctgac tgatgggctg cctgtatcga gtggtgattt tgtgccgagc 5940
tgccggtcgg ggagctgttg gctggctggt ggcaggatat attgtggtgt aaacaaattg 6000
acgcttagac aacttaataa cacattgcgg acgtttttaa tgtactgggg tggatgcagg 6060
tcctgctgag cctcgacatg ttgtcgcaaa attcgccctg gacccgccca acgatttgtc 6120
gtcactgtca aggtttgacc tgcacttcat ttggggccca catacaccaa aaaaatgctg 6180
cataattctc ggggcagcaa gtcggttacc cggccgccgt gctggaccgg gttgaatggt 6240
gcccgtaact ttcggtagag cggacggcca atactcaact tcaaggaatc tcacccatgc 6300
gcgccggcgg ggaaccggag ttcccttcag tgagcgttat tagttcgccg ctcggtgtgt 6360
cgtagatact agcccctggg gcacttttga aatttgaata agatttatgt aatcagtctt 6420
ttaggtttga ccggttctgc cgcttttttt aaaattggat ttgtaataat aaaacgcaat 6480
tgtttgttat tgtggcgctc tatcatagat gtcgctataa acctattcag cacaatatat 6540
tgttttcatt ttaatattgt acatataagt agtagggtac aatcagtaaa ttgaacggag 6600

aatattattc ataaaaatac gatagtaacg ggtgatatat tcattagaat gaaccgaaac 6660
cggcggtaag gatctgagct acacatgctc aggtttttta caacgtgcac aacagaattg 6720
aaagcaaata tcatgcgatc ataggcgtct cgcatatctc attaaagcag gactctaggt 6780
catcaaatct cggtgacggg caggaccgga cggggcggta ccggcaggct gaagtccagc 6840
tgccagaaac ccacgtcatg ccagttcccg tgcttgaagc cggccgcccg cagcatgccg 6900
cggggggcat atccgagcgc ctcgtgcatg cgcacgctcg ggtcgttggg cagcccgatg 6960
acagcgacca cgctcttgaa gccctgtgcc tccagggact tcagcaggtg ggtgtagagc 7020
gtggagccca gtcccgtccg ctggtggcgg ggggagacgt acacggttga ctcggccgtc 7080
cagtcgtagg cgttgcgtgc cttccagggg cccgcgtagg cgatgccggc gacctcgccg 7140
tccacctcgg cgacgagcca gggatagcgc tcccgcagac ggacgaggtc gtccgtccac 7200
tcctgcggtt cctgcggctc ggtacggaag ttgaccgtgc ttgtctcgat gtagtggttg 7260
acgatggtgc agaccgccgg catgtccgcc tcggtggcac ggcggatgtc ggccgggcgt 7320
cgttctgggc tcatggtaat tgtaaatagt aattgtaatg ttgtttgttg tttgttgttg 7380
ttggtaattg ttgtaaaaat agtcgagttg agagtgaata tgagactcta attggatacc 7440
gaggggaatt tatggaacgt cagtggagca tttttgacaa gaaatatttg ctagctgata 7500
gtgaccttag gcgacttttg aacgcgcaat aatggtttct gacgtatgtg cttagctcat 7560
taaactccag aaacccgcgg ctgagtggct ccttcaacgt tgcggttctg tcagttccaa 7620
acgtaaaacg gcttgtcccg cgtcatcggc gggggtcata acgtgactcc cttaattctc 7680
cgctcagaat tccaatccca caaaaatctg agcttaacag cacagttgct cctctcagag 7740
cagaatcggg tattcaacac cctcatatca actactacgt tgtgtataac ggtccacatg 7800
ccggtatata cgatgactgg ggttgtacaa aggcggcaac aaacggcgtt cccggagttg 7860
cacacaagaa atttgccact attacagagg caagagcagc agctgacgcg tacacaacaa 7920
gtcagcaaac agacaggttg aacttcatcc ccaaaggaga agctcaactc aagcccaaga 7980
gctttgctaa ggccctaaca agcccaccaa agcaaaaagc ccactggctc acgctaggaa 8040
ccaaaaggcc cagcagtgat ccagccccaa aagagatctc ctttgccccg gagattacaa 8100
tggacgattt cctctatctt tacgatctag gaaggaagtt cgaaggtgaa ggtgacgaca 8160
ctatgttcac cactgataat gagaaggtta gcctcttcaa tttcagaaag aatgctgacc 8220
cacagatggt tagagaggcc tacgcagcag gtctcatcaa gacgatctac ccgagtaaca 8280
atctccagga gatcaaatac cttcccaaga aggttaaaga tgcagtcaaa agattcagga 8340
ctaattgcat caagaacaca gagaaagaca tatttctcaa gatcagaagt actattccag 8400
tatggacgat tcaaggcttg cttcataaac caaggcaagt aatagagatt ggagtctcta 8460
aaaaggtagt tcctactgaa tctaaggcca tgcatggagt ctaagattca aatcgaggat 8520
ctaacagaac tcgccgtgaa gactggcgaa cagttcatac agagtctttt acgactcaat 8580
gacaagaaga aaatcttcgt caacatggtg gagcacgaca ctctggtcta ctccaaaaat 8640

gtcaaagata cagtctcaga agaccaaagg gctattgaga cttttcaaca aaggataatt 8700

tcgggaaacc tcctcggatt ccattgccca gctatctgtc acttcatcga aaggacagta 8760

gaaaaggaag gtggctccta caaatgccat cattgcgata aaggaaaggc tatcattcaa 8820

gatctctctg ccgacagtgg tcccaaagat ggacccccac ccacgaggag catcgtggaa 8880

aaagaagacg ttccaaccac gtcttcaaag caagtggatt gatgtgacat ctccactgac 8940

gtaagggatg acgcacaatc ccactatcct tcgcaagacc cttcctctat ataaggaagt 9000

tcatttcatt tggagaggac acgctcgagt ataagagctc tatttttaca acaattacca 9060

acaacaacaa acaacaaaca acattacaat tacatttaca attac 9105

<210> 33
<211> 11916
<212> DNA
<213> artificial

<220>
<223> complete sequence of vector pHW511

<400> 33

```
cgattcatga gcccggagat cgagaagctc tcccagtccg acatctactg ggactccatc      60
gtgtccatca ccgaaacggg cgtggaggag gtgttcgacc tcaccgtgcc aggcccgcac     120
aacttcgtgg ccaacgacat catcgtgcac aactccatcg agcaggacgg aggcggtgga     180
agtggaggcg gtggatcagg aggcggtggc tcatgtaacc acgcgtctgt tgactggcag     240
gtggtggcca atggtgatgt cagcgttgaa ctgcgtgatg cggatcaaca ggtggttgca     300
actggacaag gcactagcgg gactttgcaa gtggtgaatc cgcacctctg gcaaccgggt     360
gaaggttatc tctatgaact gtgcgtcaca gccaaaagcc agacagagtg tgatatctac     420
ccgcttcgcg tcggcatccg gtcagtggca gtgaagggcg aacagttcct gattaaccac     480
aaaccgttct actttactgg ctttggtcgt catgaagatg cggacttgcg tggcaaagga     540
ttcgataacg tgctgatggt gcacgaccac gcattaatgg actggattgg ggccaactcc     600
taccgtacct cgcattaccc ttacgctgaa gagatgctcg actgggcaga tgaacatggc     660
atcgtggtga ttgatgaaac tgctgctgtc ggctttaacc tctctttagg cattggtttc     720
gaagcgggca acaagccgaa agaactgtac agcgaagagg cagtcaacgg ggaaactcag     780
caagcgcact tacaggcgat taaagagctg atagcgcgtg acaaaaacca cccaagcgtg     840
gtgatgtgga gtattgccaa cgaaccggat acccgtccgc aaggtgcacg ggaatatttc     900
gcgccactgg cggaagcaac gcgtaaactc gacccgacgc gtccgatcac ctgcgtcaat     960
gtaatgttct gcgacgctca caccgatacc atcagcgatc tctttgatgt gctgtgcctg    1020
aaccgttatt acggatggta tgtccaaagc ggcgatttgg aaacggcaga gaaggtactg    1080
gaaaaagaac ttctggcctg gcaggagaaa ctgcatcagc cgattatcat caccgaatac    1140
ggcgtggata cgttagccgg gctgcactca atgtacaccg acatgtggag tgaagagtat    1200
cagtgtgcat ggctggatat gtatcaccgc gtctttgatc gcgtcagcgc cgtcgtcggt    1260
gaacaggtat ggaatttcgc cgattttgcg acctcgcaag gcatattgcg cgttggcggt    1320
```

aacaagaaag ggatcttcac tcgcgaccgc aaaccgaagt cggcggcttt tctgctgcaa 1380
aaacgctgga ctggcatgaa cttcggtgaa aaaccgcagc agggaggcaa acaatgagtc 1440
ctgctttaat gagatatgcg agacgcctat gatcgcatga tatttgcttt caattctgtt 1500
gtgcacgttg taaaaaacct gagcatgtgt agctcagatc cttaccgccg gtttcggttc 1560
attctaatga atatatcacc cgttactatc gtatttttat gaataatatt ctccgttcaa 1620
tttactgatt gtaccctact acttatatgt acaatattaa aatgaaaaca atatattgtg 1680
ctgaataggt ttatagcgac atctatgata gagcgccaca ataacaaaca attgcgtttt 1740
attattacaa atccaatttt aaaaaaagcg gcagaaccgg tcaaacctaa aagactgatt 1800
acataaatct tattcaaatt tcaaaagtgc cccaggggct agtatctacg acacaccgag 1860
cggcgaacta ataacgctca ctgaagggaa ctccggttcc ccgccggcgc gcatgggtga 1920
gattccttga agttgagtat tggccgtccg ctctaccgaa agttacgggc accattcaac 1980
ccggtccagc acggcggccg ggtaaccgac ttgctgcccc gagaattatg cagcattttt 2040
ttggtgtatg tgccaaatga agtgcaggtc aaaccttgac agtgacgaca aatcgttggg 2100
cgggtccagg gcgaattttg cgacaacatg tcgaggctca gcaggacttt tttttacaca 2160
gttcaaagtg aattttggtt aaaaccctca ggttgtattt ggataatggg gaataatgtg 2220
ggtgggaatt gggattggga aatgaacgaa gggttaggat taaatggaag aaggagaata 2280
aatggttaaa atttaaagat gtcttttagt gggtgggaaa tgatttccct ttcccattag 2340
ccaaacgggg cctcagtata ttttcaatta acagaagttt aatacttaat aatttaaatg 2400
acagttcaat attttagcca tgacacatgg catccaatga aagggtcgtc cactagaaat 2460
aaaggtgaca gacggtcact gaataggtac acccatacca gccacctttc tattgtcttt 2520
gcacttggga ttgaaaaggt ggtcaccaag gggttaaaac cctgtattca ttcgggaaat 2580
gtttttgcca cacaaatgag ttccaaatac actgagtgac actacgggtc agtccctaaa 2640
atttctgaaa tgttgttacc tacccgtctc tttgtccaaa aataaaccaa acccgtacgg 2700
tgtgaatata ccttcaatgt tacctacact gtacaaggtt agcttatttt gtaacggagg 2760
gaacatacat ctttccgata cccagcatta ataattgttg tgccgccttg gttcagttca 2820
aatttctttt tacaaaattc cgcttgcatc tttgtcccgg cgcggcaaaa aaaaaatcca 2880
caataaaggt atcatataaa aacaatgatg gcagttaatc agtttaggtt ggtcactatc 2940
ttaatagatg caaattaagt tgggttgtag cgaaaccaac gaacggcatt tgttttgtgc 3000
tcccacgata caatccctta aatcagcaca cacgcacaat gcatgcacca cattttagat 3060
cgatttcgta gagaatattt cgatcacata gccacaatta atctacattc tagaagctcc 3120
aacaaactta tttaattagt tcctgcaaat taacatttac aaatatctca aactgaagaa 3180
ataactttaa ttgcaatgcc ggcagccaac ccggcgtgta tgcttccatt tgttcggatg 3240
taaaaggtgc tgtttatcca taggaagagg tgtaatatta ataactactc catccgtttc 3300
taaatatttg acgtcattga ctatttgttt aaatatgttt gaatgttcgt cttattttaa 3360

aaaaatttaa gtaattatta attattttct atcattttga tttattggtt aaatatacct 3420
tatatgtata catatagttt tacatatttc acaaaagttt ttgaatatga cgaaaggtta 3480
aacatgtgct aaaaagtcaa tggtatcaaa tatttagaaa cggagggagt atgtttgttg 3540
aaaatgtttt accttctctc aatcttaata aatttggtca gggcaggcac cggaaaaaaa 3600
aaacaggaag gcataacagc aaaacaaaaa cagtggaaat taagcattgc taattacaaa 3660
cttttctgat cattcacacc attttcatgt ttgatcccgc tcaaacttca cttcaacagc 3720
ttagacactc ttagctagca aaagtcctaa tcacaggcat tataaatggc acaggcaatt 3780
agcctcatct acacacactg ccatcactcc aattaaccaa agctaattaa gcatggttaa 3840
agttatcggt cgtcgttccc tcggagtgca aagaatattt gatattggtc ttccccaaga 3900
ccataatttt ctgctagcca atggggcgat cgccgccaat tgttttaacc atgggggagg 3960
cggtggaagt ggaggcggtg gatcaggagg cggtggctca gtggcctcca agggcaacct 4020
cgccgacgtg gccccaggga agtccatcgg cggcgacatc ttctcaaacc gcgagggcaa 4080
gctcccaggc aagtcgggca ggacctggag ggaggccgac atcaactaca cctccggctt 4140
ccgcaactcc gaccgcatcc tctactcctc cgactggctc atctacaaga ccaccgacca 4200
ctaccagacc ttcaccaaga tccgctgagt cctgctttaa tgagatatgc gagacgccta 4260
tgatcgcatg atatttgctt tcaattctgt tgtgcacgtt gtaaaaaacc tgagcatgtg 4320
tagctcagat ccttaccgcc ggtttcggtt cattctaatg aatatatcac ccgttactat 4380
cgtattttta tgaataatat tctccgttca atttactgat tgtaccctac tacttatatg 4440
tacaatatta aaatgaaaac aatatattgt gctgaatagg tttatagcga catctatgat 4500
agagcgccac aataacaaac aattgcgttt tattattaca aatccaattt taaaaaaagc 4560
ggcagaaccg gtcaaaccta aaagactgat tacataaatc ttattcaaat ttcaaaagtg 4620
ccccaggggc tagtatctac gacacaccga gcggcgaact aataacgctc actgaaggga 4680
actccggttc cccgccggcg cgcatgggtg agattccttg aagttgagta ttggccgtcc 4740
gctctaccga aagttacggg caccattcaa cccggtccag cacggcggcc gggtaaccga 4800
cttgctgccc cgagaattat gcagcatttt tttggtgtat gtgccaaatg aagtgcaggt 4860
caaaccttga cagtgacgac aaatcgttgg gcgggtccag ggcgaatttt gcgacaacat 4920
gtcgaggctc agcaggacgg atcctctaga ctgcaggcat gcaagcttag atcagattgt 4980
cgtttcccgc cttcagttta aactatcagt gtttgacagg atatattggc gggtaaacct 5040
aagagaaaag agcgtttatt agaataatcg gatatttaaa agggcgtgaa aaggtttatc 5100
cgttcgtcca tttgtatgtg catgccaacc acagggttcc ccagatcagg cgctggctgc 5160
tgaaccccca gccggaactg accccacaag gccctagcgt ttgcaatgca ccaggtcatc 5220
attgacccag gcgtgttcca ccaggccgct gcctcgcaac tcttcgcagg cttcgccgac 5280
ctgctcgcgc cacttcttca cgcgggtgga atccgatccg cacatgaggc ggaaggtttc 5340
cagcttgagc gggtacggct cccggtgcga gctgaaatag tcgaacatcc gtcgggccgt 5400
cggcgacagc ttgcggtact tctcccatat gaatttcgcc caacgcggtc ggacgtgaag 5460

cccatcgccg tcgcctgtag gcgcgacagg cattcctcgg ccttcgtgta ataccggcca 5520
ttgatcgacc agcccaggtc ctggcaaagc tcgtagaacg tgaaggtgat cggctcgccg 5580
ataggggtgc gcttcgcgta ctccaacacc tgctgccaca ccagttcgtc atcgtcggcc 5640
cgcagctcga cgccggtgta ggtgatcttc acgtccttgt tgacgtggaa aatgaccttg 5700
ttttgcagcg cctcgcgcgg gattttcttg ttgcgcgtgg tgaacagggc agagcgggcc 5760
gtgtcgtttg gcatcgctcg catcgtgtcc ggccacggcg caatatcgaa caaggaaagc 5820
tgcatttcct tgatctgctg cttcgtgtgt ttcagcaacg cggcctgctt ggcctcgctg 5880
acctgttttg ccaggtcctc gccggcggtt tttcgcttct tggtcgtcat agttcctcgc 5940
gtgtcgatgg tcatcgactt cgccaaacct gccgcctcct gttcgagacg acgcgaacgc 6000
tccacggcgg ccgatggcgc gggcagggca gggggagcca gttgcacgct gtcgcgctcg 6060
atcttggccg tagcttgctg gaccatcgag ccgacggact ggaaggtttc gcggggcgca 6120
cgcatgacgg tgcggcttgc gatggtttcg gcatcctcgg cggaaaaccc cgcgtcgatc 6180
agttcttgcc tgtatgcctt ccggtcaaac gtccgattca ttcaccctcc ttgcgggatt 6240
gccccgactc acgccggggc aatgtgccct tattcctgat ttgacccgcc tggtgccttg 6300
gtgtccagat aatccacctt atcggcaatg aagtcggtcc cgtagaccgt ctggccgtcc 6360
ttctcgtact tggtattccg aatcttgccc tgcacgaata ccagcgaccc cttgcccaaa 6420
tacttgccgt gggcctcggc ctgagagcca aaacacttga tgcggaagaa gtcggtgcgc 6480
tcctgcttgt cgccggcatc gttgcgccac atctaggatc tgccaggaac cgtaaaaagg 6540
ccgcgttgct ggcgtttttc cataggctcc gcccccctga cgagcatcac aaaaatcgac 6600
gctcaagtca gaggtggcga aacccgacag gactataaag ataccaggcg tttccccctg 6660
gaagctccct cgtgcgctct cctgttccga ccctgccgct taccggatac ctgtccgcct 6720
ttctcccttc gggaagcgtg gcgctttctc atagctcacg ctgtaggtat ctcagttcgg 6780
tgtaggtcgt tcgctccaag ctgggctgtg tgcacgaacc ccccgttcag cccgaccgct 6840
gcgccttatc cggtaactat cgtcttgagt ccaacccggt aagacacgac ttatcgccac 6900
tggcagcagc cactggtaac aggattagca gagcgaggta tgtaggcggt gctacagagt 6960
tcttgaagtg gtggcctaac tacggctaca ctagaaggac agtatttggt atctgcgctc 7020
tgctgaagcc agttaccttc ggaaaaagag ttggtagctc ttgatccggc aaacaaacca 7080
ccgctggtag cggtggtttt tttgtttgca agcagcagat tacgcgcaga aaaaaaggat 7140
ctcaagaaga tcctttgatc ttttctacgg ggtctgacgc tcagtggaac gaaaactcac 7200
gttaagggat tttggtcatg agattatcaa aaaggatctt cacctagatc cttttaaatt 7260
aaaaatgaag ttttaaatca atctaaagta tatatgagta aacttggtct gacagttacc 7320
aatgcttaat cagtgaggca cctatctcag cgatctgtct atttcgttca tccatagttg 7380
cctgactccc cgtcgtgtag ataactacga tacgggaggg cttaccatct ggccccagtg 7440
ctgcaatgat accgcgagac ccacgctcac cggctccaga tttatcagca ataaaccagc 7500

cagccggaag ggccgagcgc agaagtggtc ctgcaacttt atccgcctcc atccagtcta 7560
ttaattgttg ccgggaagct agagtaagta gttcgccagt taatagtttg cgcaacgttg 7620
ttgccattgc tacaggcatc gtggtgtcac gctcgtcgtt tggtatggct tcattcagct 7680
ccggttccca acgatcaagg cgagttacat gatcccccat gttgtgcaaa aaagcggtta 7740
gctccttcgg tcctccgatc gttgtcagaa gtaagttggc cgcagtgtta tcactcatgg 7800
ttatggcagc actgcataat tctcttactg tcatgccatc cgtaagatgc ttttctgtga 7860
ctggtgagta ctcaaccaag tcattctgag aatagtgtat gcggcgaccg agttgctctt 7920
gcccggcgtc aatacgggat aataccgcgc cacatagcag aactttaaaa gtgctcatca 7980
ttggaaaacg ttcttcgggg cgaaaactct caaggatctt accgctgttg agatccagtt 8040
cgatgtaacc cactcgtgca cccaactgat cttcagcatc ttttactttc accagcgttt 8100
ctgggtgagc aaaaacagga aggcaaaatg ccgcaaaaaa gggaataagg gcgacacgga 8160
aatgttgaat actcatactc ttcctttttc aatattattg aagcatttat cagggttatt 8220
gtctcatgag cggatacata tttgaatgta tttagaaaaa taaacaaata ggggttccgc 8280
gcacgaattg gccagcgctg ccatttttgg ggtgaggccg ttcgcggccg aggggcgcag 8340
cccctggggg gatgggaggc ccgcgttagc gggccgggag ggttcgagaa ggggggggcac 8400
ccccccttcgg cgtgcgcggt cacgcgcaca gggcgcagcc ctggttaaaa acaaggttta 8460
taaatattgg tttaaaagca ggttaaaaga caggttagcg gtggccgaaa aacgggcgga 8520
aacccttgca aatgctggat tttctgcctg tggacagccc ctcaaatgtc aataggtgcg 8580
cccctcatct gtcagcactc tgcccctcaa gtgtcaagga tcgcgcccct catctgtcag 8640
tagtcgcgcc cctcaagtgt caataccgca gggcacttat ccccaggctt gtccacatca 8700
tctgtgggaa actcgcgtaa aatcaggcgt tttcgccgat ttgcgaggct ggccagctcc 8760
acgtcgccgg ccgaaatcga gcctgcccct catctgtcaa cgccgcgccg ggtgagtcgg 8820
cccctcaagt gtcaacgtcc gcccctcatc tgtcagtgag ggccaagttt tccgcgaggt 8880
atccacaacg ccggcggccg cggtgtctcg cacacggctt cgacggcgtt tctggcgcgt 8940
ttgcagggcc atagacggcc gccagcccag cggcgagggc aaccagcccg gtgagcgtcg 9000
caaaggagat cctgatctga ctgatgggct gcctgtatcg agtggtgatt ttgtgccgag 9060
ctgccggtcg ggagctgttg gctggctgg tggcaggata tattgtggtg taaacaaatt 9120
gacgcttaga caacttaata acacattgcg gacgttttta atgtactggg gtggatgcag 9180
gtcgatctag taacatagat gacaccgcgc gcgataattt atcctagttt gcgcgctata 9240
ttttgttttc tatcgcgtat taaatgtata attgcgggac tctaatcata aaaacccatc 9300
tcataaataa cgtcatgcat tacatgttaa ttattacatg cttaacgtaa ttcaacagaa 9360
attatatgat aatcatcgca agaccggcaa caggattcaa tcttaagaaa ctttattgcc 9420
aaatgtttga acgatctgct tgactctaga tccagagtcc cgctcagaag aactcgtcaa 9480
gaaggcgata gaaggcgatg cgctgcgaat cgggagcggc gataccgtaa agcacgagga 9540
agcggtcagc ccattcgccg ccaagctctt cagcaatatc acgggtagcc aacgctatgt 9600

cctgatagcg gtccgccaca cccagccggc cacagtcgat gaatccagaa aagcggccat 9660
tttccaccat gatattcggc aagcaggcat cgccatgagt cacgacgaga tcctcgccgt 9720
cgggcatacg cgccttgagc ctggcgaaca gttcggctgg cgcgagcccc tgatgctctt 9780
cgtccagatc atcctgatcg acaagaccgg cttccatccg agtacgtgct cgctcgatgc 9840
gatgtttcgc ttggtggtcg aatgggcagg tagccggatc aagcgtatgc agccgccgca 9900
ttgcatcagc catgatggat actttctcgg caggagcaag gtgagatgac aggagatcct 9960
gccccggcac ttcgcccaat agcagccagt cccttcccgc ttcagtgaca acgtcgagca 10020
cagctgcgca aggaacgccc gtcgtggcca gccacgatag ccgcgctgcc tcgtcctgga 10080
gttcattcag ggcaccggac aggtcggtct tgacaaaaag aaccgggcgc ccctgcgctg 10140
acagccggaa cacggcggca tcagagcagc cgattgtctg ttgtgcccag tcatagccga 10200
atagcctctc cacccaagcg gccggagaac ctgcgtgcaa tccatcttgt tcaatcatgc 10260
gaaacgatcc agatccggtg cagattattt ggattgagag tgaatatgag actctaattg 10320
gataccgagg ggaatttatg gaacgtcagt ggagcatttt tgacaagaaa tatttgctag 10380
ctgatagtga ccttaggcga cttttgaacg cgcaataatg gtttctgacg tatgtgctta 10440
gctcattaaa ctccagaaac ccgcggctga gtggctcctt caacgttgcg gttctgtcag 10500
ttccaaacgt aaaacggctt gtcccgcgtc atcggcgggg gtcataacgt gactccctta 10560
attctccgct cagaattcca atcccacaaa aatctgagct taacagcaca gttgctcctc 10620
tcagagcaga atcgggtatt caacaccctc atatcaacta ctacgttgtg tataacggtc 10680
cacatgccgg tatatacgat gactggggtt gtacaaaggc ggcaacaaac ggcgttcccg 10740
gagttgcaca caagaaattt gccactatta cagaggcaag agcagcagct gacgcgtaca 10800
caacaagtca gcaaacagac aggttgaact tcatccccaa aggagaagct caactcaagc 10860
ccaagagctt tgctaaggcc ctaacaagcc caccaaagca aaaagcccac tggctcacgc 10920
taggaaccaa aaggcccagc agtgatccag ccccaaaaga gatctccttt gccccggaga 10980
ttacaatgga cgatttcctc tatctttacg atctaggaag gaagttcgaa ggtgaaggtg 11040
acgacactat gttcaccact gataatgaga aggttagcct cttcaatttc agaaagaatg 11100
ctgacccaca gatggttaga gaggcctacg cagcaggtct catcaagacg atctacccga 11160
gtaacaatct ccaggagatc aaataccttc ccaagaaggt taaagatgca gtcaaaagat 11220
tcaggactaa ttgcatcaag aacacagaga aagacatatt tctcaagatc agaagtacta 11280
ttccagtatg gacgattcaa ggcttgcttc ataaaccaag gcaagtaata gagattggag 11340
tctctaaaaa ggtagttcct actgaatcta aggccatgca tggagtctaa gattcaaatc 11400
gaggatctaa cagaactcgc cgtgaagact ggcgaacagt tcatacagag tcttttacga 11460
ctcaatgaca agaagaaaat cttcgtcaac atggtggagc acgacactct ggtctactcc 11520
aaaaatgtca aagatacagt ctcagaagac caaagggcta ttgagacttt tcaacaaagg 11580
ataatttcgg gaaacctcct cggattccat tgcccagcta tctgtcactt catcgaaagg 11640

acagtagaaa aggaaggtgg ctcctacaaa tgccatcatt gcgataaagg aaaggctatc 11700
attcaagatc tctctgccga cagtggtccc aaagatggac ccccacccac gaggagcatc 11760
gtggaaaaag aagacgttcc aaccacgtct tcaaagcaag tggattgatg tgacatctcc 11820
actgacgtaa gggatgacgc acaatcccac tatccttcgc aagacccttc ctctatataa 11880
ggaagttcat ttcatttgga gaggacacgc agctat 11916

<210> 34
<211> 11558
<212> DNA
<213> artificial

<220>
<223> complete sequence of vector pHW521

<400> 34

```
catgggtcag tcccttatgt tacgtcctgt agaaacccca acccgtgaaa tcaaaaaact      60
cgacggcctg tgggcattca gtctggatcg cgaaaactgt ggaattgatc agcgttggtg     120
ggaaagcgcg ttacaagaaa gccgggcaat tgctgtgcca ggcagtttta acgatcagtt     180
cgccgatgca gatattcgta attatgcggg caacgtctgg tatcagcgcg aagtctttat     240
accgaaaggt tgggcaggcc agcgtatcgt gctgcgtttc gatgcggtca ctcattacgg     300
caaagtgtgg gtcaataatc aggaagtgat ggagcatcag ggcggctata cgccatttga     360
agccgatgtc acgccgtatg ttattgccgg gaaaagtgta cgtatcaccg tttgtgtgaa     420
caacgaactg aactggcaga ctatcccgcc gggaatggtg attaccgacg aaaacggcaa     480
gaaaaagcag tcttacttcc atgatttctt taactatgcc ggaatccatc gcagcgtaat     540
gctctacacc acgccgaaca cctgggtgga cgatatcacc gtggtgacgc atgtcgcgca     600
agacggaggc ggtggaagtg gaggcggtgg atcaggaggc ggtggctcaa gggagtccgg     660
ctgcatctcc ggcgactccc tcatctccct cgcctccacc ggcaagcgcg tgtccatcaa     720
ggacctcctc gacgagaagg acttcgagat ttgggccatc aacgagcaga ccatgaagct     780
ggagtccgcc aaggtgtccc gcgtgttctg caccggcaag aagctcgtct atatcctcaa     840
gacccgcctc ggcaggacca tcaaggccac cgccaaccac cgcttcctca ccatcgacgg     900
ctggaagcgc ctcgacgagc tgtccctcaa ggagcacatc gccctcccgc gcaagctcga     960
atcctcctcc ctccagctct gaggatcctc tagagtcctg ctttaatgag atatgcgaga    1020
cgcctatgat cgcatgatat ttgctttcaa ttctgttgtg cacgttgtaa aaaacctgag    1080
catgtgtagc tcagatcctt accgccggtt tcggttcatt ctaatgaata tatcacccgt    1140
tactatcgta tttttatgaa taatattctc cgttcaattt actgattgta ccctactact    1200
tatatgtaca atattaaaat gaaaacaata tattgtgctg aataggttta tagcgacatc    1260
tatgatagag cgccacaata acaaacaatt gcgttttatt attacaaatc caattttaaa    1320
aaaagcggca gaaccggtca aacctaaaag actgattaca taaatcttat tcaaatttca    1380
aaagtgcccc aggggctagt atctacgaca caccgagcgg cgaactaata acgctcactg    1440
aagggaactc cggttccccg ccggcgcgca tgggtgagat tccttgaagt tgagtattgg    1500
```

```
ccgtccgctc taccgaaagt tacgggcacc attcaacccg gtccagcacg gcggccgggt     1560
aaccgacttg ctgccccgag aattatgcag catttttttg gtgtatgtgg gccccaaatg     1620
aagtgcaggt caaaccttga cagtgacgac aaatcgttgg gcgggtccag ggcgaatttt     1680
gcgacaacat gtcgaggctc agcaggacct gcaggcatgc aagcttagat cagattgtcg     1740
tttcccgcct tcagtttaaa ctatcagtgt ttgacaggat atattggcgg gtaaacctaa     1800
gagaaaagag cgtttattag aataatcgga tatttaaaag ggcgtgaaaa ggtttatccg     1860
ttcgtccatt tgtatgtgca tgccaaccac aggaattcgg cgcctttttt ttacacagtt     1920
caaagtgaat tttggttaaa accctcaggt tgtatttgga caatgggaaa tatgtggtgg     1980
aattgggatt gggaaataaa tgaagggtta ggattaaatg gaagaaggag aataaaaggt     2040
taagatttaa agatgtcttt tagtgggtgg gaaatgattt tcctttccca ttagccaaac     2100
ggggcctcag tatatttcca attaacagaa gtttaatact taataattta aatgacagtt     2160
caatatttta gccatgacac atggcatcca atgaaagggt cgtccactag aaataaaggt     2220
gacagacgat cactgaatag gtacacccat accagccacc tttctattgt ctttgcactt     2280
ggattgaaga gtggtcacaa gggttaaaac cctgaatcat tcggaaatgt ttttgccaca     2340
caaatgagct ccaaatacac tgagtgacac tacgggtcag tcactaaaat ttctgaaatg     2400
ttgttaccta ctcgtctctt tgtccaaaaa taaaccaaac tcgtacggtg tgaatacatc     2460
ttcaatgtta cctacactgt acaagattag cttattttgt aacggaggga acatacatct     2520
ttcgataaca ctataattgt tgtgccgcct tggttcagtt caaatttctt tttacaaaat     2580
tccgcttgca tctttgtccc ggcgcggcaa aaaaaaaaat ccacaataaa ggtatcatat     2640
aaaaacaatg atggcagtta atcagtttag gttggtcact atcttaatag atgcaaatta     2700
agttgggttg tagcgaaacc aacgaacggc atttgttttg tgctcccacg atacaatccc     2760
ttaaatcagc acacacgcac aatgcatgca ccacatttta gatcgatttc gtagagaata     2820
tttcgatcac atagccacaa ttaatctaca ttctagaagc tccaacaaac ttatttaatt     2880
agttcctgca aattaacatt tacaaatatc tcaaactgaa gaaataactt taattgcaat     2940
gccggcagcc aacccggcgt gtatgcttcc atttgttcga tgtaaagtgc tgtttatcca     3000
taggaagaga tgtaatatta ataactactc catcagtttc taaatatttg acgccattga     3060
ttttttttta aatatgtttg aatgttcatc ttatttaaaa aaatttaagt aattattaat     3120
tatttttcta tcatttgatt tattgttaaa tatacttata tgtatacata tagttttaca     3180
tatttcacaa aagtttttga atatgacgaa cggttaaata tgtgctaaaa agtcaatggt     3240
atcaaatatt tagaaacgga gggagtatat ttgttgaaaa tgttttacct tctctcaatc     3300
ttaataaatt tggtcagggc aggcacagaa aaaaaaaaca ggaagacata acagcaaaac     3360
aaaaacagtg gaaattaagc attgctaatt acaaactttt ctgatcattc acaccatttt     3420
catgtttgat ccagctcaaa cttcactaca acagcttaga acactcttag ctagcaaaag     3480
tcctaatcac agccattata aatagacaca agcaattagc ctcatctaca cacactccca     3540
```

tcactccaat taaccaaagc taattaagca tatggcccaa gtgattaaca ccttcgacgg 3600
cgtggccgac tacctccaga cctaccacaa gctcccggac aactacatca ccaagtccga 3660
ggcccaggcc ctcggctggg acgtcaagtt tgcggaatat tgcctcagtt ttggcaccga 3720
aattttaacc gttgagtacg gcccattgcc cattggcaaa attgtgagtg aagaaattaa 3780
ttgttctgtg tacagtgttg atccagaagg gagagtttac acccaggcga tcgcccaatg 3840
gcatgaccgg ggagagcagg aagtattgga atatgaattg gaagatggtt cagtaatccg 3900
agctacctct gaccaccgct tttaaccac cgattatcaa ctgttggcga tcgaagaaat 3960
ttttgctagg caactggact tgttgacttt agaaaatatt aagcaaactg aagaagctct 4020
tgacaaccat cgtcttccct ttccattact tgacgctggg acaattaaat aactagagtc 4080
aagcagatcg ttcaaacatt tggcaataaa gtttcttaag attgaatcct gttgccggtc 4140
ttgcgatgat tatcatataa tttctgttga attacgttaa gcatgtaata attaacatgt 4200
aatgcatgac gttatttatg agatgggttt ttatgattag agtcccgcaa ttatacattt 4260
aatacgcgat agaaaacaaa atatagcgcg caaactagga taaattatcg cgcgcggtgt 4320
catctatgtt actagatcga cctgcagttc cccagatcag gatctccttt gcgacgctca 4380
ccgggctggt tgccctcgcc gctgggctgg cggccgtcta tggccctgca aacgcgccag 4440
aaacgccgtc gaagccgtgt gcgagacacc gcggccgccg gcgttgtgga tacctcgcgg 4500
aaaacttggc cctcactgac agatgagggg cggacgttga cacttgaggg gccgactcac 4560
ccggcgcggc gttgacagat gaggggcagg ctcgatttcg gccggcgacg tggagctggc 4620
cagcctcgca aatcggcgaa aacgcctgat tttacgcgag tttcccacag atgatgtgga 4680
caagcctggg gataagtgcc ctgcggtatt gacacttgag ggggcgcgact actgacagat 4740
gaggggcgcg atccttgaca cttgaggggc agagtgctga cagatgaggg gcgcacctat 4800
tgacatttga ggggctgtcc acaggcagaa aatccagcat ttgcaagggt ttccgcccgt 4860
ttttcggcca ccgctaacct gtcttttaac ctgcttttaa accaatattt ataaaccttg 4920
tttttaacca gggctgcgcc ctgtgcgcgt gaccgcgcac gccgaagggg ggtgcccccc 4980
cttctcgaac cctcccggcc cgctaacgcg ggcctcccat ccccccaggg gctgcgcccc 5040
tcggccgcga acggcctcac cccaaaaatg gcagcgctgg ccaattcgtg cgcggaaccc 5100
ctatttgttt atttttctaa atacattcaa atatgtatcc gctcatgaga caataaccct 5160
gataaatgct tcaataatat tgaaaaagga agagtatgag tattcaacat ttccgtgtcg 5220
cccttattcc cttttttgcg gcattttgcc ttcctgtttt tgctcaccca gaaacgctgg 5280
tgaaagtaaa agatgctgaa gatcagttgg gtgcacgagt gggttacatc gaactggatc 5340
tcaacagcgg taagatcctt gagagttttc gccccgaaga acgttttcca atgatgagca 5400
cttttaaagt tctgctatgt ggcgcggtat tatcccgtat tgacgccggg caagagcaac 5460
tcggtcgccg catacactat tctcagaatg acttggttga gtactcacca gtcacagaaa 5520
agcatcttac ggatggcatg acagtaagag aattatgcag tgctgccata accatgagtg 5580
ataacactgc ggccaactta cttctgacaa cgatcggagg accgaaggag ctaaccgctt 5640

ttttgcacaa catgggggat catgtaactc gccttgatcg ttgggaaccg gagctgaatg 5700
aagccatacc aaacgacgag cgtgacacca cgatgcctgt agcaatggca acaacgttgc 5760
gcaaactatt aactggcgaa ctacttactc tagcttcccg gcaacaatta atagactgga 5820
tggaggcgga taaagttgca ggaccacttc tgcgctcggc ccttccggct ggctggttta 5880
ttgctgataa atctggagcc ggtgagcgtg ggtctcgcgg tatcattgca gcactggggc 5940
cagatggtaa gccctcccgt atcgtagtta tctacacgac ggggagtcag gcaactatgg 6000
atgaacgaaa tagacagatc gctgagatag gtgcctcact gattaagcat tggtaactgt 6060
cagaccaagt ttactcatat atactttaga ttgatttaaa acttcatttt taatttaaaa 6120
ggatctaggt gaagatcctt tttgataatc tcatgaccaa aatcccttaa cgtgagtttt 6180
cgttccactg agcgtcagac cccgtagaaa agatcaaagg atcttcttga gatccttttt 6240
ttctgcgcgt aatctgctgc ttgcaaacaa aaaaaccacc gctaccagcg gtggtttgtt 6300
tgccggatca agagctacca actctttttc cgaaggtaac tggcttcagc agagcgcaga 6360
taccaaatac tgtccttcta gtgtagccgt agttaggcca ccacttcaag aactctgtag 6420
caccgcctac atacctcgct ctgctaatcc tgttaccagt ggctgctgcc agtggcgata 6480
agtcgtgtct taccgggttg gactcaagac gatagttacc ggataaggcg cagcggtcgg 6540
gctgaacggg gggttcgtgc acacagccca gcttggagcg aacgacctac accgaactga 6600
gatacctaca gcgtgagcta tgagaaagcg ccacgcttcc cgaagggaga aaggcggaca 6660
ggtatccggt aagcggcagg gtcggaacag gagagcgcac gagggagctt ccagggggaa 6720
acgcctggta tctttatagt cctgtcgggt ttcgccacct ctgacttgag cgtcgatttt 6780
tgtgatgctc gtcagggggg cggagcctat ggaaaaacgc cagcaacgcg gcctttttac 6840
ggttcctggc agatcctaga tgtggcgcaa cgatgccggc gacaagcagg agcgcaccga 6900
cttcttccgc atcaagtgtt ttggctctca ggccgaggcc cacggcaagt atttgggcaa 6960
ggggtcgctg gtattcgtgc agggcaagat tcggaatacc aagtacgaga aggacggcca 7020
gacggtctac gggaccgact tcattgccga taaggtggat tatctggaca ccaaggcacc 7080
aggcgggtca aatcaggaat aagggcacat tgccccggcg tgagtcgggg caatcccgca 7140
aggagggtga atgaatcgga cgtttgaccg gaaggcatac aggcaagaac tgatcgacgc 7200
ggggttttcc gccgaggatg ccgaaaccat cgcaagccgc accgtcatgc gtgcgccccg 7260
cgaaaccttc cagtccgtcg gctcgatggt ccagcaagct acggccaaga tcgagcgcga 7320
cagcgtgcaa ctggctcccc ctgccctgcc cgcgccatcg gccgccgtgg agcgttcgcg 7380
tcgtctcgaa caggaggcgg caggtttggc gaagtcgatg accatcgaca cgcgaggaac 7440
tatgacgacc aagaagcgaa aaaccgccgg cgaggacctg gcaaaacagg tcagcgaggc 7500
caagcaggcc gcgttgctga aacacacgaa gcagcagatc aaggaaatgc agctttcctt 7560
gttcgatatt gcgccgtggc cggacacgat gcgagcgatg ccaaacgaca cggcccgctc 7620
tgccctgttc accacgcgca acaagaaaat cccgcgcgag gcgctgcaaa acaaggtcat 7680

tttccacgtc aacaaggacg tgaagatcac ctacaccggc gtcgagctgc gggccgacga 7740
tgacgaactg gtgtggcagc aggtgttgga gtacgcgaag cgcaccccta tcggcgagcc 7800
gatcaccttc acgttctacg agctttgcca ggacctgggc tggtcgatca atggccggta 7860
ttacacgaag gccgaggaat gcctgtcgcg cctacaggcg acggcgatgg gcttcacgtc 7920
cgaccgcgtt gggcacctgg aatcggtgtc gctgctgcac cgcttccgcg tcctggaccg 7980
tggcaagaaa acgtcccgtt gccaggtcct gatcgacgag gaaatcgtcg tgctgtttgc 8040
tggcgaccac tacacgaaat tcatatggga gaagtaccgc aagctgtcgc cgacggcccg 8100
acggatgttc gactatttca gctcgcaccg ggagccgtac ccgctcaagc tggaaacctt 8160
ccgcctcatg tgcggatcgg attccacccg cgtgaagaag tggcgcgagc aggtcggcga 8220
agcctgcgaa gagttgcgag gcagcggcct ggtggaacac gcctgggtca atgatgacct 8280
ggtgcattgc aaacgctagg gccttgtggg gtcagttccg gctgggggtt cagcagccag 8340
cgcctgatct gactgatggg ctgcctgtat cgagtggtga ttttgtgccg agctgccggt 8400
cggggagctg ttggctggct ggtggcagga tatattgtgg tgtaaacaaa ttgacgctta 8460
gacaacttaa taacacattg cggacgtttt taatgtactg gggtggatgc aggtcctgct 8520
gagcctcgac atgttgtcgc aaaattcgcc ctggacccgc ccaacgattt gtcgtcactg 8580
tcaaggtttg acctgcactt catttggggc ccacatacac caaaaaaatg ctgcataatt 8640
ctcggggcag caagtcggtt acccggccgc cgtgctggac cgggttgaat ggtgcccgta 8700
actttcggta gagcggacgg ccaatactca acttcaagga atctcaccca tgcgcgccgg 8760
cggggaaccg gagttccctt cagtgagcgt tattagttcg ccgctcggtg tgtcgtagat 8820
actagcccct ggggcacttt tgaaatttga ataagattta tgtaatcagt cttttaggtt 8880
tgaccggttc tgccgctttt tttaaaattg gatttgtaat aataaaacgc aattgtttgt 8940
tattgtggcg ctctatcata gatgtcgcta taaacctatt cagcacaata tattgttttc 9000
attttaatat tgtacatata agtagtaggg tacaatcagt aaattgaacg gagaatatta 9060
ttcataaaaa tacgatagta acgggtgata tattcattag aatgaaccga aaccggcggt 9120
aaggatctga gctacacatg ctcaggtttt ttacaacgtg cacaacagaa ttgaaagcaa 9180
atatcatgcg atcataggcg tctcgcatat ctcattaaag caggactcta ggtcatcaaa 9240
tctcggtgac gggcaggacc ggacggggcg gtaccggcag gctgaagtcc agctgccaga 9300
aacccacgtc atgccagttc ccgtgcttga agccggccgc ccgcagcatg ccgcgggggg 9360
catatccgag cgcctcgtgc atgcgcacgc tcgggtcgtt gggcagcccg atgacagcga 9420
ccacgctctt gaagccctgt gcctccaggg acttcagcag gtgggtgtag agcgtggagc 9480
ccagtcccgt ccgctggtgg cggggggaga cgtacacggt tgactcggcc gtccagtcgt 9540
aggcgttgcg tgccttccag ggcccgcgt aggcgatgcc ggcgacctcg ccgtccacct 9600
cggcgacgag ccagggatag cgctcccgca gacggacgag gtcgtccgtc cactcctgcg 9660
gttcctgcgg ctcggtacgg aagttgaccg tgcttgtctc gatgtagtgg ttgacgatgg 9720
tgcagaccgc cggcatgtcc gcctcggtgg cacggcggat gtcggccggg cgtcgttctg 9780

```
ggctcatggt aattgtaaat agtaattgta atgttgtttg ttgtttgttg ttgttggtaa      9840
ttgttgtaaa aatagtcgag ttgagagtga atatgagact ctaattggat accgagggga      9900
atttatggaa cgtcagtgga gcatttttga caagaaatat ttgctagctg atagtgacct      9960
taggcgactt ttgaacgcgc aataatggtt tctgacgtat gtgcttagct cattaaactc     10020
cagaaacccg cggctgagtg gctccttcaa cgttgcggtt ctgtcagttc caaacgtaaa     10080
acggcttgtc ccgcgtcatc ggcgggggtc ataacgtgac tcccttaatt ctccgctcag     10140
aattccaatc ccacaaaaat ctgagcttaa cagcacagtt gctcctctca gagcagaatc     10200
gggtattcaa caccctcata tcaactacta cgttgtgtat aacggtccac atgccggtat     10260
atacgatgac tggggttgta caaaggcggc aacaaacggc gttcccggag ttgcacacaa     10320
gaaatttgcc actattacag aggcaagagc agcagctgac gcgtacacaa caagtcagca     10380
aacagacagg ttgaacttca tccccaaagg agaagctcaa ctcaagccca agagctttgc     10440
taaggcccta acaagcccac caaagcaaaa agcccactgg ctcacgctag gaaccaaaag     10500
gcccagcagt gatccagccc caaaagagat ctcctttgcc ccggagatta caatggacga     10560
tttcctctat ctttacgatc taggaaggaa gttcgaaggt gaaggtgacg acactatgtt     10620
caccactgat aatgagaagg ttagcctctt caatttcaga aagaatgctg acccacagat     10680
ggttagagag gcctacgcag caggtctcat caagacgatc tacccgagta acaatctcca     10740
ggagatcaaa taccttccca agaaggttaa agatgcagtc aaaagattca ggactaattg     10800
catcaagaac acagagaaag acatatttct caagatcaga agtactattc cagtatggac     10860
gattcaaggc ttgcttcata aaccaaggca agtaatagag attggagtct ctaaaaaggt     10920
agttcctact gaatctaagg ccatgcatgg agtctaagat tcaaatcgag gatctaacag     10980
aactcgccgt gaagactggc gaacagttca tacagagtct tttacgactc aatgacaaga     11040
agaaaatctt cgtcaacatg gtggagcacg acactctggt ctactccaaa aatgtcaaag     11100
atacagtctc agaagaccaa agggctattg agacttttca acaaaggata atttcgggaa     11160
acctcctcgg attccattgc ccagctatct gtcacttcat cgaaaggaca gtagaaaagg     11220
aaggtggctc ctacaaatgc catcattgcg ataaaggaaa ggctatcatt caagatctct     11280
ctgccgacag tggtcccaaa gatggacccc cacccacgag gagcatcgtg gaaaaagaag     11340
acgttccaac cacgtcttca aagcaagtgg attgatgtga catctccact gacgtaaggg     11400
atgacgcaca atcccactat ccttcgcaag acccttcctc tatataagga agttcatttc     11460
atttggagag gacacgctcg agtataagag ctctattttt acaacaatta ccaacaacaa     11520
caaacaacaa acaacattac aattacattt acaattac                             11558
```

<210> 35
<211> 19
<212> DNA
<213> artificial

<220>
<223> DNA primer

<400> 35
gtctggatcg cgaaaactg 19

<210> 36
<211> 19
<212> DNA
<213> artificial

<220>
<223> DNA primer

<400> 36
ctgccagttc agttcgttg 19

<210> 37
<211> 19
<212> DNA
<213> artificial

<220>
<223> DNA primer

<400> 37
gtggtggcca atggtgatg 19

<210> 38
<211> 19
<212> DNA
<213> artificial

<220>
<223> DNA primer

<400> 38
ggagttggcc ccaatccag 19

<210> 39
<211> 21
<212> DNA
<213> artificial

<220>
<223> DNA primer

<400> 39
caagtgatta acaccttcga c 21

<210> 40
<211> 21
<212> DNA
<213> artificial

<220>
<223> DNA primer

<400> 40
ccagcgtcaa gtaatggaaa g 21

<210> 41
<211> 22
<212> DNA
<213> artificial

<220>
<223> DNA primer

<400> 41
gatcttggtg aaggtctggt ag 22

<210> 42
<211> 21
<212> DNA
<213> artificial

<220>
<223> DNA primer

<400> 42
gtcgtcgttc cctcggagtg c 21